(19) Europäisches Patentamt
European Patent Office
Office européen des brevets

(11) EP 4 745 239 A1

(12) EUROPEAN PATENT APPLICATION
published in accordance with Art. 153(4) EPC

(43) Date of publication:
**20.05.2026 Bulletin 2026/21**

(21) Application number: **24848185.5**

(22) Date of filing: **26.07.2024**

(51) International Patent Classification (IPC):
***C12N 15/113*** *(2010.01)* ***A61K 31/713*** *(2006.01)*
***A61K 31/712*** *(2006.01)* ***A61K 31/7088*** *(2006.01)*

(52) Cooperative Patent Classification (CPC):
**A61K 31/7088; A61K 31/712; A61K 31/713; C12N 15/113**

(86) International application number:
**PCT/CN2024/107887**

(87) International publication number:
**WO 2025/026228 (06.02.2025 Gazette 2025/06)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **28.07.2023 CN 202310938654**

(71) Applicant: **Rigerna Therapeutics Co., Ltd.**
**Suzhou, Jiangsu 215128 (CN)**

(72) Inventors:
- **HUANG, Yuanyu**
  **Suzhou, Jiangsu 215128 (CN)**
- **LI, Haitao**
  **Suzhou, Jiangsu 215128 (CN)**
- **KONG, Lina**
  **Suzhou, Jiangsu 215128 (CN)**
- **FAN, Zhibin**
  **Suzhou, Jiangsu 215128 (CN)**

(74) Representative: **Vogelbruch, Keang**
**VOGELBRUCH Patentanwaltsgesellschaft mbH**
**Paul-Gerhardt-Straße 16**
**40593 Düsseldorf (DE)**

Remarks:
The complete document including Reference Table(s) and the Sequence Listing(s) can be downloaded from the EPO website

# (54) CFB INHIBITOR COMPOSITION AND USE THEREOF

(57) The present invention relates to the technical field of nucleic acid drugs, and particularly provides a double-stranded oligonucleotide for inhibiting complement factor B (CFB) gene expression. The double-stranded oligonucleotide comprises a sense strand and an antisense strand; the sense strand contains at least fifteen contiguous nucleotides in any one sequence shown as SEQ ID NO: 1-SEQ ID NO: 255 in Table 1 or a nucleotide sequence which differs from the at least fifteen contiguous nucleotides in respect of not more than three nucleotides; and/or, the antisense strand contains at least fifteen contiguous nucleotides in any one sequence shown as SEQ ID NO: 256-SEQ ID NO: 510 in Table 1 or a nucleotide sequence which differs from the at least fifteen contiguous nucleotides in respect of not more than three nucleotides. The double-stranded oligonucleotide can inhibit CFB gene expression in cells, thereby achieving the effects of relieving, treating and/or preventing diseases or disorders mediated by abnormal CFB gene expression.

EP 4 745 239 A1

## Description

## CROSS-REFERENCE TO RELATED APPLICATION

The present disclosure claims priority to Chinese Patent Application No. 202310938654.8, filed with the China National Intellectual Property Administration on July 28, 2023, and entitled "CFB INHIBITOR COMPOSITION AND USE THEREOF," the contents of which are incorporated herein by reference in entirety.

## TECHNICAL FIELD

The present disclosure relates to the technical field of nucleic acid medicaments, and particularly to an RNAi composition for inhibiting complement factor B (CFB) gene expression, such as a double-stranded ribonucleic acid (dsRNA) composition and use thereof.

## BACKGROUND ART

Complement system is part of the host innate immune system, and consists of a variety of proteins that are either present as soluble proteins in the blood or are present as membrane-associated proteins. Inappropriate activation of the complement system is responsible for propagating and/or initiating pathology in many diseases. Complement factor B is an essential component of an alternative pathway of complement activation, and circulates as single-chain polypeptide in the blood. Upon activation of the alternative pathway, the factor B is cleaved by complement factor D, producing non-catalytic chain Ba and catalytic subunit Bb. The active subunit Bb is a serine protease that binds to C3b to form a bypass pathway C3 convertase. The C3 convertase catalyzes the formation of C3 to form C3a and C3b, C3b binds to the C3 convertase again mediated by CFB and results in a sequential cascade of enzymatic reactions, including production of C5 convertase. The C5 convertase activates a membrane attack pathway, resulting in the formation of a series of membrane attack complexes (MACs), causing a variety of physiological responses including inflammatory responses and even apoptosis.

The complement system regulates immunity and plays an important role in normal inflammatory responses, but dysregulation of the complement system may lead to diseases associated with complement dysregulation. Inhibition of excess or deregulated complement activation can treat or relieve associated diseases or disorders.

Therefore, there is a need in the art to treat diseases or disorders associated with complement activation by modulating complement factor B activity.

## SUMMARY

The present disclosure provides a complement factor B (CFB) inhibitor, comprising a double-stranded oligonucleotide, a double-stranded RNAi agent, and a pharmaceutical composition containing the same. The present disclosure provides a method for treating, preventing, or alleviating a disease associated with dysregulation of a complement alternative pathway in a subject by administering the complement factor B (CFB) inhibitor. The present disclosure further provides a method for inhibiting CFB expression by administering a CFB specific inhibitor (double-stranded RNAi agent) to a subject.

In order to solve the above technical problems, the present disclosure specifically adopts technical solutions as follows.

In the first aspect, the present disclosure provides a double-stranded oligonucleotide (dsRNA) for inhibiting complement factor B (CFB) gene expression, wherein the double-stranded oligonucleotide is capable of inhibiting the CFB gene expression in mammals, including humans, monkeys, rats, and mice. The double-stranded oligonucleotide is capable of inhibiting the CFB gene expression in a cell *in vitro,* and is also capable of inhibiting the CFB gene expression in a body *in vivo.*

The double-stranded oligonucleotide comprises a sense strand and an antisense strand, wherein the sense strand comprises at least 15 contiguous nucleotides in any one of sequences as set forth in SEQ ID NOs: 1-255 in Table 1 or a nucleotide sequence which differs from the at least 15 contiguous nucleotides by no more than 3 nucleotides; and/or, the antisense strand comprises at least 15 contiguous nucleotides in any one of sequences as set forth in SEQ ID NOs: 256-510 in Table 1 or a nucleotide sequence which differs from the at least 15 contiguous nucleotides by no more than 3 nucleotides.

Preferably, the sense strand comprises a nucleotide sequence which differs from any one of sequences as set forth in SEQ ID NOs: 1-255 in Table 1 by no more than 3 nucleotides, and/or the antisense strand comprises a nucleotide sequence which differs from any one of sequences as set forth in SEQ ID NOs: 256-510 in Table 1 by no more than 3 nucleotides.

The sense strand and the antisense strand are complementary or substantially complementary to form a double-stranded region (duplex region), wherein substantially complementary means that there are no more than three-nucleotide mismatches between the sense strand and the antisense strand within the double-stranded region.

In the second aspect, the present disclosure provides a double-stranded RNAi agent for inhibiting complement factor B (CFB) gene expression, wherein the double-stranded RNAi agent comprises a sense strand and an antisense strand, wherein the sense strand and the antisense strand are complementary or substantially complementary to form a double-stranded region, wherein substantially complementary means that there are no more than three-nucleotide mismatches between the sense strand and the antisense strand within the double-stranded region, and the double-stranded region is represented by Formula (I) as follows:

$$SS: 5'-(N)_{a'}-(X)_{p'}-(N)_{b'}-(X)_{q'}-(N)_{c'}-(X)_{r'}-(N)_{d'}-3'\ AS: 3'-(N)_a-(X)_p-(N)_b-(X)_q-(N)_c-5' \quad (I).$$

Herein, SS represents the sense strand, and AS represents the antisense strand.

All nucleotides of the sense strand and the antisense strand are modified nucleotides.

Each N independently represents a modified nucleotide selected from the group consisting of a 2'-O-methyl-modified nucleotide, a 2'-fluoro-modified nucleotide, and a 2'-deoxy-modified nucleotide.

Each X independently represents a 2'-O-methoxyethyl-modified nucleotide, a 2'-O-methyl-modified nucleotide, or a 2'-$O(CH_2)_nOR$ substituent-modified nucleotide, wherein n is 1 or 2, and R is selected from substituted or unsubstituted $C_1$-$C_6$ alkyl and substituted or unsubstituted $C_1$-$C_6$ alkoxy, wherein the substituent is selected from the group consisting of halogen, $C_1$-$C_6$ alkyl, $C_1$-$C_6$ alkoxy, hydroxyl, and amino.

The a, a', p, p', b, b', q, q', c, c', r', and d' each independently represent the number of nucleotides, wherein a' is an integer selected from 3 to 8; p' is an integer selected from 0 to 3; b' is an integer selected from 4 to 13; q' is an integer selected from 0 to 4; c' is an integer selected from 3 to 9; r' is an integer selected from 0 to 3; d' is an integer selected from 0 to 9; a is an integer selected from 4 to 7; p is 0 or 1; b is an integer selected from 4 to 8; q is an integer selected from 0 to 4; and c is an integer selected from 6 to 10.

Moreover, p', q', r', p, and q are not simultaneously 0, and $0 \leq q' + r' \leq 4$.

In the third aspect, the present disclosure provides a double-stranded RNAi agent for inhibiting complement factor B (CFB) expression, wherein the double-stranded RNAi agent comprises a sense strand and an antisense strand that form a double-stranded region, wherein the sense strand comprises a nucleotide sequence as set forth in SEQ ID NOs: 1-255 in Table 1, and the antisense strand comprises a nucleotide sequence as set forth in SEQ ID NOs: 256-510 in Table 1. Herein, each strand is no more than 25 nucleotides in length, and the sense strand and the antisense strand are both modified nucleotides. The modified nucleotides are selected from the group consisting of 2'-O-methyl-modified nucleotides and 2'-fluoro-modified nucleotides. The sense strand comprises 1-3 phosphorothioate internucleotide linkages. Herein, the modified nucleotides in the antisense strand are selected from the group consisting of 2'-O-methyl-modified nucleotides, 2'-O-methoxyethyl-modified nucleotides, and 2'-fluoro-modified nucleotides, wherein the antisense strand comprises 2-5 phosphorothioate internucleotide linkages. Moreover, the sense strand is conjugated to a GalNAc ligand at 3'-end.

In the fourth aspect, the present disclosure provides use of the double-stranded RNAi agent in the manufacture of a medicament for relieving, preventing and/or treating a disease or disorder mediated by a complement factor B gene.

In the fifth aspect, the present disclosure provides a pharmaceutical composition, comprising the double-stranded RNAi agent of the present disclosure, and further comprising a pharmaceutically optional auxiliary material.

In the sixth aspect, the present disclosure provides a method for inhibiting complement factor B (CFB) expression in a cell *in vitro,* including:

(a) contacting the cell with the double-stranded RNAi agent of the present disclosure or the pharmaceutical composition thereof; and
(b) maintaining the cell produced in step (a) for a period sufficient to obtain degradation of an mRNA transcript of a complement factor B (CFB) gene, thereby inhibiting the complement factor B (CFB) gene expression in the cell, wherein the complement factor B (CFB) expression is inhibited by at least 70%.

In the seventh aspect, the present disclosure provides use of the double-stranded RNAi agent or the pharmaceutical composition thereof for the manufacture of a medicament for treating a subject suffering from dysregulation associated with complement factor B (CFB).

In the eighth aspect, the present disclosure provides use of the double-stranded RNAi agent for the manufacture of a medicament for treating a subject suffering from a disorder associated with deregulation of complement factor B (CFB), wherein the medicament is subcutaneously administered to the subject, and optionally, the subject is a human or other mammal.

In the ninth aspect, the present disclosure provides a kit, wherein the kit includes the double-stranded RNAi agent

of the present disclosure or the pharmaceutical composition thereof.

In the tenth aspect, the present disclosure provides a method for inhibiting complement factor B gene expression, wherein the method comprises administering the double-stranded RNAi agent of the present disclosure or the pharmaceutical composition thereof to a subject. In some embodiments, the complement factor B expression is inhibited by at least 50%, 60%, 70%, 80%, 90%, or 95%. In some embodiments, inhibition of the complement factor B expression reduces a complement factor B protein level in serum of the subject by at least 50%, 60%, 70%, 80%, 90%, or 95%.

In the eleventh aspect, the present disclosure provides a method for relieving, treating and/or preventing a disease or disorder mediated by complement factor B, comprising administering the double-stranded RNAi agent of the present disclosure or the pharmaceutical composition thereof to a subject.

Herein, the disease or disorder mediated by the complement factor B includes a disease associated with mRNA level of complement factor B gene expression.

Herein, the disease associated with the complement factor B is selected from the group consisting of paroxysmal nocturnal hemoglobinuria (PNH), asthma, rheumatoid arthritis, systemic lupus erythmatosis, glomerulonephritis, psoriasis, dermatomyositis bullous pemphigoid, atypical hemolytic uremic syndrome, Shiga toxin *E. coli*-related hemolytic uremic syndrome, myasthenia gravis, neuromyelistis optica, dense deposit disease, C3 neuropathy, age-related macular degeneration, cold agglutinin disease, anti-neutrophil cytoplasmic antibody-associated vasculitis, myocardial infarction, and sepsis.

Optionally, the disease or disorder includes, but is not limited to, kidney disease, systemic lupus erythematosus and related diseases, macular degeneration, atypical hemolytic uremic syndrome, thrombotic microangiopathy, myasthenia gravis, ischemia-reperfusion injury, paroxysmal nocturnal hemoglobinuria, and rheumatoid arthritis.

Optionally, the kidney disease includes C3 glomerulopathy, lupus nephritis, IgA nephropathy, diabetic nephropathy, membranous nephropathy, and polycystic kidney disease.

Additional aspects and advantages of the present disclosure will be partially given in the following description, and partially become apparent from the following description, or be comprehended by practicing the present disclosure.

## BRIEF DESCRIPTION OF THE DRAWING

The above and/or additional aspects and advantages of the present disclosure will become apparent and readily understood from the description of embodiments in conjunction with the drawings attached below. In the drawings,

FIG. 1 shows inhibitory activity against target gene in primary mouse hepatocytes after administration of a double-stranded RNAi agent in Experiment 3.

FIG. 2 shows inhibitory activity against target gene in C57BL/6J mice after administration of the double-stranded RNAi agent in Experiment 4.

FIG. 3 shows inhibitory activity against target gene in C57BL/6J mice after administration of double-stranded RNAi agents with different ligands and modifications in Experiment 5.

## DETAILED DESCRIPTION OF EMBODIMENTS

Embodiments of the present disclosure will be described in detail below. The embodiments described below are illustrative, merely for explaining the present disclosure, but should not be construed as limitation to the present disclosure. Technical solutions in the embodiments of the present disclosure will be described clearly and completely below, and apparently, only some but not all embodiments of the present disclosure are described. Based on the embodiments in the present disclosure, all of other embodiments obtained by those ordinarily skilled in the art without using any inventive efforts shall fall within the scope of protection of the present disclosure.

Compounds in the present disclosure can be prepared through multiple synthesis methods which are well-known to those skilled in the art, including specific embodiments listed below, embodiments formed through combination of them with other methods, and equivalent alternatives which are well-known to those skilled in the art. Preferred embodiments include but are not limited to embodiments of the present disclosure.

In the first aspect, the present disclosure provides a double-stranded oligonucleotide (dsRNA) for inhibiting complement factor B (CFB) gene expression, wherein the double-stranded oligonucleotide is capable of inhibiting the CFB gene expression in mammals, including humans, monkeys, rats, and mice. The double-stranded oligonucleotide is capable of inhibiting the CFB gene expression in a cell *in vitro,* and is also capable of inhibiting the CFB gene expression in a body *in vivo.*

The double-stranded oligonucleotide comprises a sense strand and an antisense strand, wherein the sense strand comprises at least 15 contiguous nucleotides in any one of sequences as set forth in SEQ ID NOs: 1-255 in Table 1 or a nucleotide sequence which differs from the at least 15 contiguous nucleotides by no more than 3 nucleotides; and/or, the antisense strand comprises at least 15 contiguous nucleotides in any one of sequences as set forth in SEQ ID NOs:

256-510 in Table 1 or a nucleotide sequence which differs from the at least 15 contiguous nucleotides by no more than 3 nucleotides; and the sense strand and the antisense strand are complementary or substantially complementary to form a double-stranded region, wherein substantially complementary means that there are no more than three-nucleotide mismatches between the sense strand and the antisense strand within the double-stranded region.

In some optional embodiments, the sense strand comprises a nucleotide sequence which differs from any one of sequences as set forth in SEQ ID NOs: 1-255 in Table 1 by no more than 3 nucleotides, and/or the antisense strand comprises a nucleotide sequence which differs from any one of sequences as set forth in SEQ ID NOs: 256-510 in Table 1 by no more than 3 nucleotides.

In some optional embodiments, the sense strand comprises a nucleotide sequence which differs from any one of sequences as set forth in SEQ ID NOs: 1-255 in Table 1 by no more than 1 or 2 nucleotides, and/or the antisense strand comprises a nucleotide sequence which differs from any one of sequences as set forth in SEQ ID NOs: 256-510 in Table 1 by no more than 1 or 2 nucleotides.

In some optional embodiments, the sense strand comprises a nucleotide sequence which differs from any one of sequences as set forth in SEQ ID NOs: 1-255 in Table 1 by no more than 1 nucleotide, and/or the antisense strand comprises a nucleotide sequence which differs from any one of sequences as set forth in SEQ ID NOs: 256-510 in Table 1 by no more than 1 nucleotide.

In some specific embodiments of the present disclosure, the sense strand comprises any one of nucleotide sequences as set forth in SEQ ID NOs: 1-255 in Table 1, and/or the antisense strand comprises any one of nucleotide sequences as set forth in SEQ ID NOs: 256-510 in Table 1.

In some optional embodiments, the sense strand or the antisense strand comprises a nucleotide sequence of any sense strand or antisense strand selected from the group consisting of duplexes represented by the following in Table 1: RN011001, RN011005, RN011006, RN011007, RN011018, RN011021, RN011022, RN011033, RN011034, RN011035, RN011038, RN011052, RN011076, RN011097, RN011098, RN011101, RN011102, RN011103, RN011104, RN011106, RN011107, RN011109, RN011113, RN011114, RN011117, RN011123, RN011124, RN011125, RN011140, RN01115, RN011151, RN011152, RN011156, RN011160, RN011171, RN011183, RN011193, RN011194, RN011199, RN011201, RN011202, RN011233, RN011238, RN011241, RN011242, RN011243, RN011254, and RN011255.

In some specific embodiments, the sense strand or the antisense strand comprises a nucleotide sequence of the sense strand or antisense strand of the duplex represented by RN011255.

In some optional embodiments, the double-stranded oligonucleotide comprises one or more selected from the group consisting of duplexes represented by following numbers in Table 1: RN011001, RN011005, RN011006, RN011007, RN011018, RN011021, RN011022, RN011033, RN011034, RN011035, RN011038, RN011052, RN011076, RN011097, RN011098, RN011101, RN011102, RN011103, RN011104, RN011106, RN011107, RN011109, RN011113, RN011114, RN011117, RN011123, RN011124, RN011125, RN011140, RN01115, RN011151, RN011152, RN011156, RN011160, RN011171, RN011183, RN011193, RN011194, RN011199, RN011201, RN011202, RN011233, RN011238, RN011241, RN011242, RN011243, RN011254, and RN011255.

In some specific embodiments, the double-stranded oligonucleotide comprises nucleotide sequences of the duplex represented by RN011255.

In some optional embodiments, each nucleotide in the double-stranded oligonucleotide is unmodified or modified nucleotide.

In some optional embodiments, at least one nucleotide in the double-stranded oligonucleotide is modified.

In some optional embodiments, all nucleotides of the sense strand and all nucleotides of the antisense strand of the double-stranded oligonucleotide are modified nucleotides.

In some optional embodiments, the modified nucleotides are selected from one or more of 3'-terminal deoxythymine (dT) nucleotides, 2'-O-methyl-modified nucleotides, 2'-fluoro-modified nucleotides, 2'-deoxy-modified nucleotides, 2'-O-methoxyethyl-modified nucleotides, and 2'-O$(CH_2)_n$OR substituted nucleotides, wherein n is 1 or 2, and R is selected from substituted or unsubstituted $C_1$-$C_6$ alkyl and substituted or unsubstituted $C_1$-$C_6$ alkoxy, wherein the substituent is selected from the group consisting of halogen, $C_1$-$C_6$ alkyl, $C_1$-$C_6$ alkoxy, hydroxyl, and amino.

In some optional embodiments, the modified nucleotides are selected from one or more of 2'-O-methyl-modified nucleotides, 2'-fluoro-modified nucleotides, 2'-O-methoxyethyl-modified nucleotides, and 2'-O$(CH_2)_n$OR substituted nucleotides.

In some specific embodiments of the present disclosure, the sense strand of the double-stranded oligonucleotide comprises any modified nucleotide sequence in the sense strand as listed in Table 3, and/or the antisense strand comprises any modified nucleotide sequence in the antisense strand as listed in Table 3.

In some optional embodiments, the antisense strand and the sense strand are 17-25 nucleotides in length.

In some optional embodiments, at least one of the antisense strand and the sense strand comprises a 3'-overhang of 1-2 nucleotides.

In the second aspect, the present disclosure provides a double-stranded RNAi agent for inhibiting complement factor B (CFB) gene expression, wherein the double-stranded RNAi agent comprises a sense strand and an antisense

strand, wherein the sense strand and the antisense strand are complementary or substantially complementary to form a double-stranded region, wherein substantially complementary means that there are no more than three-nucleotide mismatches between the sense strand and the antisense strand within the double-stranded region, and the double-stranded region is represented by Formula (I) as follows:

$$SS: 5'-(N)_{a'}-(X)_{p'}-(N)_{b'}-(X)_{q'}-(N)_{c'}-(X)_{r'}-(N)_{d'}-3' \quad AS: 3'-(N)_{a}-(X)_{p}-(N)_{b}-(X)_{q}-(N)_{c}-5' \qquad (I).$$

Herein, SS represents the sense strand, and AS represents the antisense strand.

All nucleotides of the sense strand and the antisense strand are modified nucleotides.

Each N independently represents a modified nucleotide selected from the group consisting of a 2'-O-methyl-modified nucleotide, a 2'-fluoro-modified nucleotide, and a 2'-deoxy-modified nucleotide.

Each X independently represents a 2'-O-methoxyethyl-modified nucleotide, a 2'-O-methyl-modified nucleotide, or a 2'-$O(CH_2)_nOR$ substituent-modified nucleotide, wherein n is 1 or 2, and R is selected from substituted or unsubstituted $C_1$-$C_6$ alkyl or substituted or unsubstituted $C_1$-$C_6$ alkoxy, wherein the substituent is selected from the group consisting of halogen, $C_1$-$C_6$ alkyl, $C_1$-$C_6$ alkoxy, hydroxyl, and amino.

The a, a', p, p', b, b', q, q', c, c', r', and d' each independently represent the number of nucleotides, wherein a' is an integer selected from 3 to 8; p' is an integer selected from 0 to 3; b' is an integer selected from 4 to 13; q' is an integer selected from 0 to 4; c' is an integer selected from 3 to 9; r' is an integer selected from 0 to 3; d' is an integer selected from 0 to 9; a is an integer selected from 4 to 7; p is 0 or 1; b is an integer selected from 4 to 8; q is an integer selected from 0 to 4; and c is an integer selected from 6 to 10.

Moreover, p', q', r', p, and q are not simultaneously 0, and $0 \leq q' + r' \leq 4$.

In some optional embodiments, N is selected from a 2'-O-methyl-modified nucleotide or a 2'-fluoro-modified nucleotide.

In some optional embodiments, the nucleotide X is a 2'-O-methyl-modified nucleotide or a 2'-O-methoxyethyl-modified nucleotide.

In some optional embodiments, the duplex comprises at least one 2'-O-methoxyethyl-modified nucleotide or 2'-$O(CH_2)_nOR$-modified nucleotide.

In some optional embodiments, the double-stranded RNAi agent comprises a duplex consisting of following modified sense strands and antisense strands, and in a direction from 5'-end to 3'-end:

The sense strand is: GmsGmsAmUmUmUmGfGfGfUfUmUmUmCmUmAmUmAmAm, or
GmsGmsAmUmUmUmGfGfGfUfUmUmUmCmUmAmUmAmsAm;
The antisense strand is: UmsUfsAmUmAmGfAmAmAfAmCmCmCmAfAmAfUmCmCmsUmsCm, or
UmsUfsAmUmAmGfAmAmAmAmCmCfCmAfAmAfUmCmCmsUmsCm, or
UmsUfsAmUmAmGfAmAmAfAmCmCmCmAfA(moe)AfUmCmCmsUmsCm, or
UmsUfsAmUmAmGfAmAmAmAmCmCfCmAfA(moe)AfUmCmCmsUmsCm, or
UmsUfsAmUmAmGfAmAmAfAmsCmCmCmAfA(moe)AfUmCmCmsUmsCm.

In some optional embodiments, the nucleotides of the sense strand and/or antisense strand of the double-stranded RNAi are modified nucleotides, and in the direction from the 5'-end to the 3'-end, at least three of the nucleotides at positions 7-10 of the nucleotide sequence in the sense strand are fluoro-modified nucleotides, and the nucleotides at the remaining positions are 2'-O-methyl-modified nucleotides or 2'-O-methoxyethyl-modified nucleotides; and optionally, in the direction from the 5'-end to the 3'-end, at least four of the nucleotides at positions 2, 6, 9-12, 14, and 16 of the nucleotide sequence in the antisense strand are 2'-fluoro-modified nucleotides, and the nucleotides at the remaining positions are 2'-O-methyl-modified or 2'-O-methoxyethyl-modified nucleotides.

In some optional embodiments, the sense strand and/or the antisense strand comprises at least one 2'-O-methoxyethyl-modified nucleotide.

In some optional embodiments, in the double-stranded RNAi agent, in the direction from the 5'-end to the 3'-end, at least three of the nucleotides at positions 7-10 of the nucleotide sequence in the sense strand are fluoro-modified nucleotides, and the nucleotides at the remaining positions are 2'-O-methyl-modified nucleotides or 2'-O-methoxyethyl-modified nucleotides.

In some optional embodiments, in the direction from the 5'-end to the 3'-end, at least four of the nucleotides at positions 2, 6, 9-12, 14, and 16 of the nucleotide sequence in the antisense strand are 2'-fluoro-modified nucleotides, and the nucleotides at the remaining positions are 2'-O-methyl-modified nucleotides or 2'-O-methoxyethyl-modified nucleotides.

In some optional embodiments, in the double-stranded RNAi agent, in the direction from the 5'-end to the 3'-end, the nucleotides at positions 7-10 of the nucleotide sequence in the sense strand are fluoro-modified nucleotides, and the nucleotides at the remaining positions are 2'-O-methyl-modified nucleotides or 2'-O-methoxyethyl-modified nucleotides;

and positions 2, 6, 14, and 16 of the nucleotide sequence in the antisense strand are 2'-fluoro-modified nucleotides, a nucleotide at any position selected from position 9, 10, 11 or 12 is a 2'-fluoro-modified nucleotide, and the nucleotides at the remaining positions are 2'-O-methyl-modified nucleotides or 2'-O-methoxyethyl-modified nucleotides.

In some optional embodiments, in the double-stranded RNAi agent, in the direction from the 5'-end to the 3'-end, the nucleotides at positions 7-10 of the nucleotide sequence in the sense strand are fluoro-modified nucleotides, and the nucleotides at the remaining positions are 2'-O-methyl-modified nucleotides.

Furthermore, positions 2, 6, 14, and 16 of the nucleotide sequence in the antisense strand are 2'-fluoro-modified nucleotides, a nucleotide at any position selected from position 9, 10, 11 or 12 is a 2'-fluoro-modified nucleotide, the nucleotide at position 15 is a 2'-O-methoxyethyl-modified nucleotide, and the remaining positions are 2'-O-methyl-modified nucleotides.

In some optional embodiments, all the nucleotides of the sense strand and/or the antisense strand are modified nucleotides; and the modification of the sense strand and the antisense strand is selected from any one of following (1)-(4).

(1) In the direction from the 5'-end to the 3'-end, in the sense strand, the nucleotides at positions 7-10 of the nucleotide sequence are fluoro-modified nucleotides, and the nucleotides at the remaining positions are 2'-O-methyl-modified nucleotides; and in the antisense strand, positions 2, 6, 9, 14, and 16 of the nucleotide sequence are 2'-fluoro-modified nucleotides, and the remaining positions are 2'-O-methyl-modified nucleotides.

(2) In the direction from the 5'-end to the 3'-end, in the sense strand, the nucleotides at positions 7-10 of the nucleotide sequence are fluoro-modified nucleotides, and the nucleotides at the remaining positions are 2'-O-methyl-modified nucleotides; and in the antisense strand, positions 2, 6, 12, 14, and 16 of the nucleotide sequence are 2'-fluoro-modified nucleotides, and the remaining positions are 2'-O-methyl-modified nucleotides.

(3) In the direction from the 5'-end to the 3'-end, in the sense strand, the nucleotides at positions 7-10 of the nucleotide sequence are fluoro-modified nucleotides, and the nucleotides at the remaining positions are 2'-O-methyl-modified nucleotides; and in the antisense strand, positions 2, 6, 9, 14, and 16 of the nucleotide sequence are 2'-fluoro-modified nucleotides, the nucleotide at position 15 is a 2'-O-methoxyethyl-modified nucleotide, and the remaining positions are 2'-O-methyl-modified nucleotides.

(4) In the direction from the 5'-end to the 3'-end, in the sense strand, the nucleotides at positions 7-10 of the nucleotide sequence are fluoro-modified nucleotides, and the nucleotides at the remaining positions are 2'-O-methyl-modified nucleotides; and in the antisense strand, positions 2, 6, 12, 14, and 16 of the nucleotide sequence are 2'-fluoro-modified nucleotides, the nucleotide at position 15 is a 2'-O-methoxyethyl-modified nucleotide, and the remaining positions are 2'-O-methyl-modified nucleotides.

In some optional embodiments, the double-stranded RNAi agent further comprises a ligand.

In some optional embodiments, the ligand is conjugated to the 3'-end of the sense strand of the double-stranded RNAi agent.

In some optional embodiments, the ligand is one or more N-acetylgalactosamine (GalNAc) derivatives attached through a monovalent, bivalent, or trivalent linker.

In some optional embodiments, the structure of the ligand is as follows:

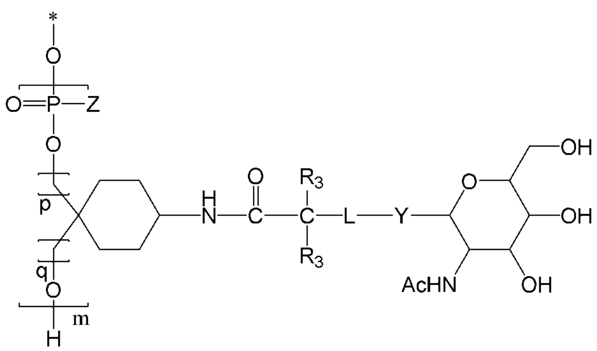

Herein, * represents a ligation site for attachment to oligonucleotide molecules; m is 1, 2, 3, or 4; Z, p, q, $R_3$, L, and Y are defined as follows: each Z is independently selected from hydroxyl or thiol; each p is independently 1, 2, or 3; each q is independently selected from 1, 2, or 3; each $R_3$ is independently selected from H, $C_1$-$C_6$ alkyl, $C_1$-$C_6$ haloalkyl, or $C_1$-$C_6$ alkoxy; L is a $C_1$-$C_{20}$ alkylene chain, or is a $C_1$-$C_{20}$ alkylene chain with one or more C atoms in the chain replaced by any substituent selected from the group consisting of O, S, NH, and -NH-C(O)-; and each Y is independently selected from NH, O, or S.

In some optional embodiments, the structure of the ligand is as follows:

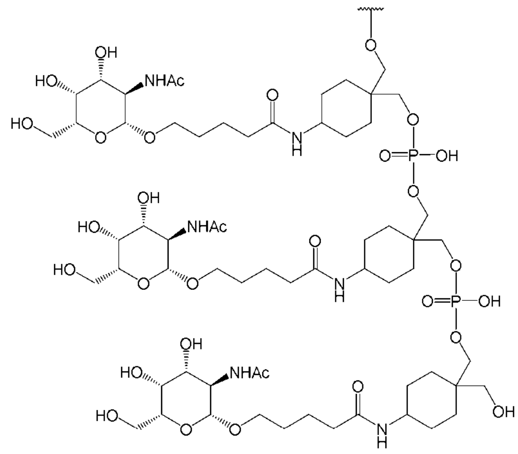

or

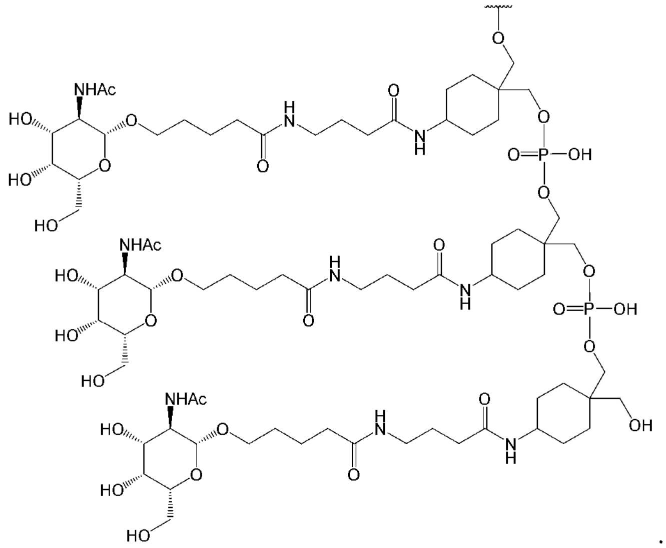

In some optional embodiments, the 3'-end of the sense strand in the double-stranded RNAi agent is covalently coupled to the ligand.

In some optional embodiments, the double-stranded RNAi agent of the present disclosure comprises a conjugate represented by a formula as follows:

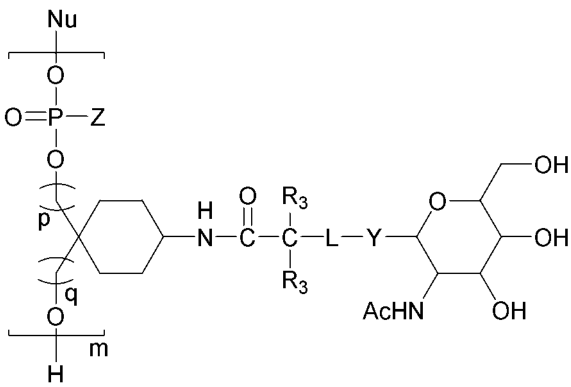

Herein, Nu represents a double-stranded oligonucleotide molecule;

the sense strand of the double-stranded oligonucleotide molecule comprises any modified nucleotide sequence in the sense strand as listed in Table 3; and/or the antisense strand comprises any modified nucleotide sequence in the

antisense strand as listed in Table 3; and
the ligand is attached to the 3'-end of the sense strand.

In some optional embodiments, the double-stranded RNAi agent of the present disclosure comprises a duplex selected from the group consisting of duplexes represented by RZ011304, RZ011305, RZ011306, and RZ011307.

In some optional embodiments, the sense strand in the double-stranded RNAi agent is: 5'-GmsGmsAmUmUmUmGfGfGfUfUmUmUmCmUmAmUmAmAm _ (CR01008×3)-3', or 5'-GmsGmsAmUmUmUmGfGfGfUfUmUmUmCmUmAmUmAmsAm _ (CR01008×3)-3'; and
the antisense strand is:

5'- UmsUfsAmUmAmGfAmAmAfAmCmCmCmAfAmAfUmCmCmsUmsCm -3', or
5'- UmsUfsAmUmAmGfAmAmAmAmCmCfCmAfAmAfUmCmCmsUmsCm -3', or
5'- UmsUfsAmUmAmGfAmAmAfAmCmCmCmAfA(moe)AfUmCmCmsUmsCm -3', or
5'- UmsUfsAmUmAmGfAmAmAmAmCmCfCmAfA(moe)AfUmCmCmsUmsCm -3' or
5'- UmsUfsAmUmAmGfAmAmAfAmsCmCmCmAfA(moe)AfUmCmCmsUmsCm -3', wherein

Am, Cm, Gm, and Um are 2'-O-methyladenosine-3'-phosphate, 2'-O-methylcytidine-3'-phosphate, 2'-O-methylguanosine phosphate, and 2'-O-methyluridine phosphate, respectively; Af, Cf, Gf, and Uf are 2'-fluoroadenosine-3'-phosphate, 2'-fluorocytidine-3'-phosphate, 2'-fluoroguanosine-3'-phosphate, and 2'-fluorouridine-3'-phosphate, respectively; A(moe) is 2'-O-methoxyethyladenosine-3'-phosphate; and s is phosphorothioate.

Herein, CR01008×3 is a ligand with a structure as follows:

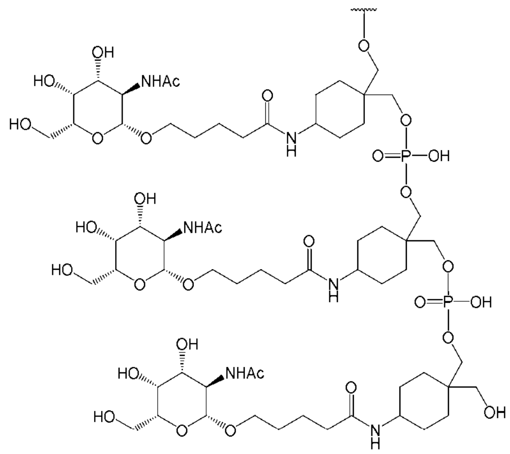

or

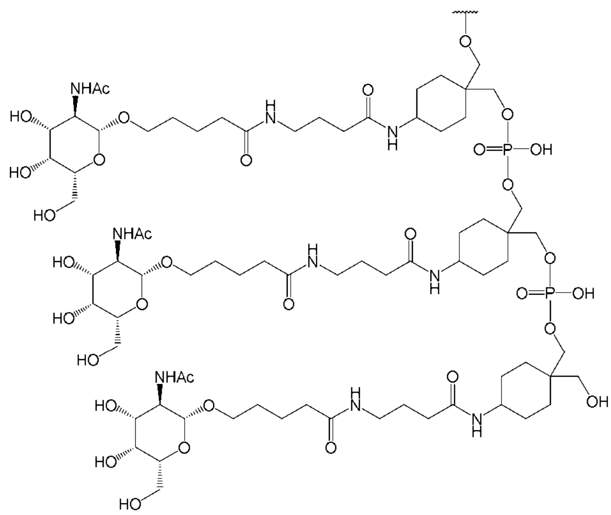

.

In some optional embodiments, the antisense strand and/or the sense strand further comprises a 3' and/or 5'

extension or overhang of 1-3 nucleotides in length.

In some optional embodiments, the sense strand of the double-stranded region is 17-21 nt in length, and the antisense strand is 19-23 nt in length. Preferably, the double-stranded region is 19 to 23 nucleotide pairs in length; or the double-stranded region is 19 to 21 nucleotide pairs in length.

In some optional embodiments, the sense strand and/or the antisense strand of the RNAi agent independently comprises one or more phosphorothioate internucleotide linkages.

In some optional embodiments, the sense strand comprises two contiguous phosphorothioate linkages between terminal nucleotides located at the 3'-end and 5'-end, or the antisense strand comprises two contiguous phosphorothioate linkages between terminal nucleotides located at the 3'-end and 5'-end.

In some optional embodiments, at least one strand within the double-stranded region of the RNAi agent comprises a 3' overhang of 1-2 nucleotides.

In some optional embodiments, the double-stranded RNAi agent comprises a sense strand and an antisense strand forming the double-stranded region, wherein each strand is 14-25 nucleotides, and the antisense strand comprises a region partially complementary to an mRNA encoding CFB (SEQ ID NO: 526), wherein the double-stranded region is represented by Formula II as follows:

$$\text{SS: } 5'\text{-}(N)_{a'}\text{-}(X)_{p'}\text{-}(N)_{b'}\text{-}(X)_{q'}\text{-}(N)_{c'}\text{-}(X)_{r'}\text{-}(N)_{d'}\text{-}3' \text{ AS: } 3'\text{-}(N)_{a}\text{-}(X)_{p}\text{-}(N)_{b}\text{-}(X)_{q}\text{-}(N)_{c}\text{-}5' \quad \text{(II)}$$

Herein, SS represents the sense strand, AS represents the antisense strand, and the SS strand can be conjugated to a ligand; each N independently represents a modified nucleotide selected from the group consisting of a 2'-O-methyl-modified nucleotide, a 2'-fluoro-modified nucleotide, and a 2'-deoxy-modified nucleotide; and each X independently represents a 2'-O-methoxyethyl-modified nucleotide.

Herein, a, a', p, p', b, b', q, q', c, c', r', and d' each independently represent the number of nucleotides, wherein a' is an integer selected from 3 to 8; p' is 0 or 1; b' is an integer selected from 4 to 13; q' is 0 or 1; c' is an integer selected from 3 to 9; r' is 0 or 1; d' is an integer selected from 1 to 8; a is an integer selected from 4 to 7; p is 1; b is an integer selected from 4 to 8; q is 0 or 1; c is an integer selected from 6 to 10; and p', q', r', p, and q are not simultaneously 0, and optionally, $0 \leq q' + r' \leq 2$.

Moreover, at least one fluoro-modified nucleotide is present in $(N)_a$, and position 16 of the antisense strand counting from the 5'-end is a fluoro-modified nucleotide; at least one fluoro-modified nucleotide is present in $(N)_b$, and position 14 of the antisense strand counting from the 5'-end is a fluoro-modified nucleotide; at least two fluoro-modified nucleotides are present in $(N)_c$, and positions 2 and 6 of the antisense strand counting from the 5'-end are both fluoro-modified nucleotides; and the first 4 nucleotides of $(N)_{b'}$ comprise at least two fluoro-modified nucleotides.

In some optional embodiments, in the direction from the 5'-end to the 3'-end, the nucleotides at positions 7-10 of the nucleotide sequence in the sense strand of Formula II are fluoro-modified nucleotides, and the nucleotides at the remaining positions are 2'-O-methyl-modified nucleotides; and positions 2, 6, 14, and 16 of the nucleotide sequence in the antisense strand are 2'-fluoro-modified nucleotides, a nucleotide at any position selected from position 9, 10, 11 or 12 is a 2'-fluoro-modified nucleotide, the nucleotide at position 15 is a 2'-O-methoxyethyl-modified nucleotide, and the nucleotides at the remaining positions are 2'-O-methyl-modified nucleotides.

In some optional embodiments, the double-stranded RNAi agent comprises a duplex selected from the group consisting of duplexes represented by RZ011304, RZ011305, RZ011306, and RZ011307.

In the third aspect, the present disclosure provides a double-stranded RNAi agent for inhibiting complement factor B (CFB) expression, wherein the double-stranded RNAi agent comprises a sense strand and an antisense strand forming a double-stranded region, wherein the sense strand comprises a nucleotide sequence as set forth in SEQ ID NOs: 1-255 in Table 1, and the antisense strand comprises a nucleotide sequence as set forth in SEQ ID NOs: 256-510 in Table 1. Herein, each strand is no more than 25 nucleotides in length, and the sense strand and the antisense strand are both modified nucleotides. The modified nucleotides are selected from the group consisting of 2'-O-methyl-modified nucleotides and 2'-fluoro-modified nucleotides. The sense strand comprises 1-3 phosphorothioate internucleotide linkages. The modified nucleotides in the antisense strand are selected from the group consisting of 2'-O-methyl-modified nucleotides, 2'-O-methoxyethyl-modified nucleotides, and 2'-fluoro-modified nucleotides. The antisense strand comprises 2-5 phosphorothioate internucleotide linkages. Moreover, the sense strand is conjugated to a GalNAc ligand at 3'-end.

In some optional embodiments, the double-stranded RNAi agent comprises a duplex selected from the group consisting of duplexes represented by RZ011304, RZ011305, RZ011306, and RZ011307.

In the fourth aspect, the present disclosure provides use of the double-stranded RNAi agent in the manufacture of a medicament for relieving, preventing and/or treating a disease or disorder mediated by a complement factor B gene.

In some optional embodiments, the disease or disorder includes kidney disease, systemic lupus erythematosus and related diseases, macular degeneration, atypical hemolytic uremic syndrome, thrombotic microangiopathy, myasthenia gravis, ischemia-reperfusion injury, paroxysmal nocturnal hemoglobinuria, and rheumatoid arthritis.

In some optional embodiments, the kidney disease includes C3 glomerulopathy, lupus nephritis, IgA nephropathy, diabetic nephropathy, membranous nephropathy, and polycystic kidney disease.

In the fifth aspect, the present disclosure provides a pharmaceutical composition, comprising the double-stranded RNAi agent of the present disclosure, and further comprising a pharmaceutically optional auxiliary material.

In some optional embodiments, the double-stranded RNAi agent of the present disclosure can be administered in an unbuffered solution or buffer. Herein, the unbuffered solution is saline or water, and the buffer comprises acetate, citrate, prolamine, carbonate, or phosphate or any combination thereof. Optionally, the buffer is phosphate buffered saline (PBS).

In the sixth aspect, the present disclosure provides a method for inhibiting complement factor B (CFB) expression in a cell *in vitro,* including:

(a) contacting the cell with the double-stranded RNAi agent of the present disclosure or the pharmaceutical composition thereof; and
(b) maintaining the cell produced in step (a) for a period sufficient to obtain degradation of an mRNA transcript of a complement factor B (CFB) gene, thereby inhibiting the complement factor B (CFB) gene expression in the cell, wherein the complement factor B (CFB) expression is inhibited by at least 70%.

In the seventh aspect, the present disclosure provides use of the double-stranded RNAi agent or the pharmaceutical composition thereof for the manufacture of a medicament for treating a subject suffering from dysregulation associated with complement factor B (CFB).

In the eighth aspect, the present disclosure provides use of the double-stranded RNAi agent for the manufacture of a medicament for treating a subject suffering from a disorder associated with deregulation of complement factor B (CFB), wherein the medicament is subcutaneously administered to the subject, and optionally, the subject is a human or other mammal.

In some optional embodiments, the double-stranded RNAi agent is administered at a dose of 0.01 mg/kg to 10 mg/kg or 0.5 mg/kg to 50 mg/kg.

In some optional embodiments, the double-stranded RNAi agent is administered subcutaneously or intravenously.

In the ninth aspect, the present disclosure provides a kit, wherein the kit comprises the double-stranded RNAi agent of the present disclosure or the pharmaceutical composition thereof.

In the tenth aspect, the present disclosure provides a method for inhibiting complement factor B gene expression, wherein the method includes administering the double-stranded RNAi agent of the present disclosure or the pharmaceutical composition thereof to a subject. In some embodiments, the complement factor B expression is inhibited by at least 50%, 60%, 70%, 80%, 90%, or 95%. In some embodiments, inhibition of the complement factor B expression reduces a complement factor B protein level in serum of the subject by at least 50%, 60%, 70%, 80%, 90%, or 95%.

In the eleventh aspect, the present disclosure provides a method for relieving, treating and/or preventing a disease or disorder mediated by complement factor B, comprising administering the double-stranded RNAi agent of the present disclosure or the pharmaceutical composition thereof to a subject.

In some optional embodiments, the disease or disorder mediated by the complement factor B includes a disease associated with mRNA level of complement factor B gene expression.

In some optional embodiments, the disease associated with the complement factor B is selected from the group consisting of paroxysmal nocturnal hemoglobinuria (PNH), asthma, rheumatoid arthritis, systemic lupus erythmatosis, glomerulonephritis, psoriasis, dermatomyositis bullous pemphigoid, atypical hemolytic uremic syndrome, Shiga toxin E. coli-related hemolytic uremic syndrome, myasthenia gravis, neuromyelistis optica, dense deposit disease, C3 neuropathy, age-related macular degeneration, cold agglutinin disease, anti-neutrophil cytoplasmic antibody-associated vasculitis, myocardial infarction, and sepsis.

In some optional embodiments, the disease or disorder includes, but is not limited to, kidney disease, systemic lupus erythematosus and related diseases, macular degeneration, atypical hemolytic uremic syndrome, thrombotic microangiopathy, myasthenia gravis, ischemia-reperfusion injury, paroxysmal nocturnal hemoglobinuria, and rheumatoid arthritis; and further preferably, the kidney disease includes C3 glomerulopathy, lupus nephritis, IgA nephropathy, diabetic nephropathy, membranous nephropathy, and polycystic kidney disease.

Experimental data in the embodiments demonstrate that the double-stranded RNAi agent or the pharmaceutical composition thereof provided by the present disclosure can effectively modulate the expression level of the complement factor B gene, and thus can effectively treat and/or prevent a disease or symptom associated with dysregulation of the complement factor B gene expression.

In some optional embodiments, the foregoing pharmaceutical composition can be administered by any suitable means, such as administration of the foregoing double-stranded oligonucleotide in the form of an injection or infusion solution (e.g., subcutaneously, intramuscularly, or intravenously), or a mode of administration of the foregoing pharmaceutical composition includes, but is not limited to, a single administration or multiple administrations. A dose ranges from 0.1 mg/kg to 100 mg/kg, or from 0.5 mg/kg to 50 mg/kg, such as 3 mg/kg, 10 mg/kg, or 33 mg/kg.

It would be clearly known to those skilled in the art that a modified nucleotide group can be introduced into the

double-stranded RNAi agent of the present disclosure using a nucleoside monomer having a corresponding modification: a method for preparing the nucleoside monomer having a corresponding modification and a method for introducing the modified nucleotide group into the double-stranded RNAi agent are also well known to those skilled in the art. All modified nucleoside monomers are either commercially available or prepared using known methods.

[Definitions]

Herein, unless particularly specified, capital letters A (adenine), U (uracil), G (guanine), C (cytosine), and T (thymine, also representing uracil in the sequence listing) represent base composition of nucleotides; lowercase letter m means that a nucleotide adjacent to the left of the letter m is a 2'-methoxy-modified nucleotide; lowercase letter f means that a nucleotide adjacent to the left of the letter f is a 2'-fluoro-modified nucleotide; (moe) means that a nucleotide adjacent to the left of combined identity is a 2'-O-methoxyethyl-modified nucleotide; and lowercase letter s means that two nucleotides adjacent to the left and right of the letter s are linked by a phosphorothioate group.

L96 represents a moiety linked to Nu (double-stranded oligonucleotide) represented by following formula.

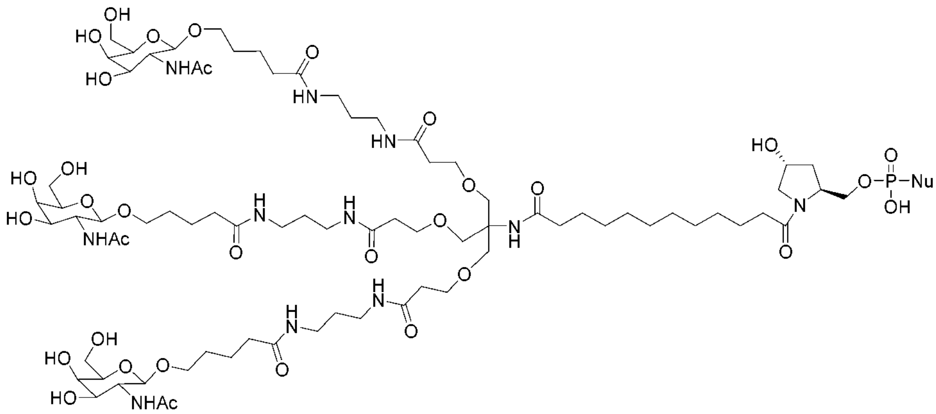

Herein, "fluoro-modified nucleotide" or "2'-fluoro-modified nucleotide" refers to a nucleotide formed by substituting 2'-hydroxyl of ribosyl group of nucleotide with fluorine, and "non-fluoro-modified nucleotide" refers to a nucleotide or a nucleotide analog formed by substituting 2'-hydroxyl of ribosyl group of nucleotide with a non-fluoro group. The "methoxy-modified nucleotide" or "2'-O-methoxyethyl-modified nucleotide" refers to a nucleotide formed by substituting 2'-hydroxy of ribosyl group with methoxy or methoxyethyl. "Methoxy-modified nucleotide" is also described as a 2'-OMe- or 2'-O-methyl-modified nucleotide, and "2'-O-methoxyethyl-modified nucleotide" can be used alternatively and is also described as a 2'-MOE-modified nucleotide. 2'-deoxynucleotide means that 2'-position of ribosyl group is hydrogen.

Herein, the double-stranded oligonucleotide consists of two strands, in which a strand that binds to target sequence is referred to as an antisense strand or a guide strand, and the other is referred to as a sense strand or a passenger strand. The term "antisense strand" refers to such a strand of the double-stranded oligonucleotide that comprises a region fully or substantially complementary to the target sequence. The term "sense strand" refers to such a strand of the double-stranded oligonucleotide that comprises a region substantially complementary to the region of the term antisense strand as defined herein. The term "region of complementarity" refers to a region on the antisense strand that is fully or substantially complementary to the target sequence. Where the region of complementarity and the target sequence are not fully complementary, mismatch(es) may be located within internal or terminal region of a molecule. As used herein, the term "complementary" refers to the ability of a first polynucleotide to hybridize with a second polynucleotide under certain conditions, e.g., stringent conditions. The double-stranded oligonucleotide and siRNA can be used interchangeably in the text.

Herein, the expressions "duplex," "duplex region," and "double-stranded region" are used interchangeably and have the meaning well-known to those skilled in the art, that is, a double-stranded region formed by a sense strand and an antisense strand fully or substantially complementary in a double-stranded nucleic acid molecule.

The expressions "complementary" and "reversely complementary" herein are used interchangeably, and have the meaning well-known to those skilled in the art, that is, bases of one strand are respectively paired with bases of the other strand in a complementary manner in a double-stranded nucleic acid molecule.

Herein, unless particularly specified, "substantially reversely complementary" or "substantially complementary" means that there are no more than three base mismatches between two nucleotide sequences involved; "virtually reversely complementary" means that there is no more than one base mismatch between two nucleotide sequences; and "fully reversely complementary" means that there is no base mismatch between two nucleotide sequences.

The term "Complement Factor B," used interchangeably with the term "CFB," refers to the well-known gene and

polypeptide, also known in the art as AHUS, BF, CFAB, BFD, FB, GBG, FBI12, B-Factor, Properdin, H2-Bf, Glycine-Rich β Glycoprotein, C3 Proaccelerator, Properdin Factor 2B, C3 Proactivator, PBF2, Glycine-Rich β-Glycoprotein, C3/C5 Convertase, EC 3.4.21, and EC 3.4.21.473. The term "CFB" includes human CFB, the amino acid and nucleotide sequence of which may be found in, for example, GenBank Accession No. GI: 189181756; mouse CFB, the amino acid and nucleotide sequence of which may be found in, for example, GenBank Accession Nos. GI: 218156288 and GI: 218156290; rat CFB, the amino acid and nucleotide sequence of which may be found in, for example, GenBank Accession No. GI: 218156284; and chimpanzee CFB, the amino acid and nucleotide sequence of which may be found in, for example, GenBank Accession No. GI: 57114201. The term "CFB" also includes Macaca fascicularis CFB, the amino acid and nucleotide sequence of which may be found in, for example, GenBank Accession No. GI: 544428919. Additional examples of CFB mRNA sequences are readily available using, e.g., GenBank, UniProt, Online Mendelian Inheritance in Man (OMIM), and the Macaca genome project web site.

Exemplary CFB nucleotide sequences of the present disclosure are listed in Tables 1-2. The term "CFB" also refers to naturally occurring DNA sequence variations of the CFB gene, and also refers to single nucleotide polymorphisms in the CFB gene. Numerous sequence variations within the CFB gene have been identified and may be found, which is known in the art.

The terms "RNAi," "iRNA," "RNAi agent," "RNAi reagent," "RNA interference agent," and "RNA inhibitor" in the present disclosure, used interchangeably herein, refer to a RNA-containing molecule or agent, which can mediate targeted cleavage of an RNA transcript via an RNA-induced silencing complex (RISC) pathway. RNA directs the sequence-specific degradation of mRNA through a process known as RNA interference (RNAi), which is well-known in the art. In an embodiment, the RNAi agent of the present disclosure comprises a single-stranded or double-stranded RNA that interacts with a target RNA sequence (e.g., a CFB, C3, or C9 target mRNA sequence), to direct the cleavage of the target RNA. Accordingly, in one aspect, the term "siRNA" involved in the present disclosure can also be used to refer to the RNAi as described above.

In some embodiments, an "RNAi" for use in the compositions, uses, and methods of the present disclosure is a double-stranded RNA, and the "RNAi agent" comprises the double-stranded RNA. The "RNAi agent" can be referred to as a "double-stranded RNAi agent," "double-stranded RNA (dsRNA) molecule," "dsRNA agent," "SiRNA agent" or "dsRNA reagent".

The term "dsRNA" or "SiRNA" refers to a complex of ribonucleic acid molecules, having a duplex structure comprising two anti-parallel and substantially complementary nucleic acid strands, referred to as having "sense" and "antisense" orientations with respect to a target RNA (e.g., a CFB, C3, or C9 gene).

In addition, as used in the present specification, "RNAi agent" or "RNAi reagent" may comprise ribonucleotides with chemical modifications and/or ligand; an RNAi agent may comprise substantial modifications at multiple nucleotides. The term "modified nucleotide" refers to a nucleotide having, independently, a modified sugar moiety, a modified internucleotide linkage, and/or a modified nucleobase. Thus, the term modified nucleotide encompasses substitutions, additions or removal of, e.g., a functional group or atom, to internucleoside linkages, sugar moieties, or nucleobases. The modifications suitable for use in the agents of the present disclosure include all types of modifications disclosed herein or known in the art. Any such modifications, as used in a siRNA molecule, can be encompassed by "RNAi agent".

The term "nucleotide overhang" or "overhang" refers to at least one unpaired nucleotide that protrudes from the duplex structure of an iRNA (e.g., a dsRNA). For example, when a 3'-end of one strand of a dsRNA extends beyond the 5'-end of the other strand, or vice versa, there is a nucleotide overhang. The overhang(s) can be on the sense strand, the antisense strand or any combination thereof. Furthermore, the nucleotide(s) of an overhang can be present on the 5'-end, 3'-end or both ends of either an antisense or sense strand of a dsRNA.

The term "ligand" generally refers to any compound or molecule capable of binding to a biologically active substance (such as an oligonucleotide) covalently or otherwise chemically. In certain embodiments, the ligand is capable of interacting directly or indirectly with another compound, such as a receptor, the receptor interacting with the ligand can be present on a cell surface, or alternatively can be an intracellular and/or an intercellular receptor, and interaction of the ligand with the receptor may cause a biochemical reaction, or may simply be a physical interaction or binding.

The term "administering (administration)" generally refers to introducing a pharmaceutical preparation of the present disclosure into a body of a subject by any route of introduction or delivery. Any method known to those skilled in the art for contacting cells, organs or tissue with the medicament can be employed. The administration may comprise, but is not limited to, intravenous, intraarterial, intranasal, intraperitoneal, intramuscular, subcutaneous or oral administration. A daily dosage can be divided into one, two or more suitable forms of dose to be administered at one, two or more time points in a certain period of time.

The term "contacting" generally means that two or more different types of substances are in contact in any order, in any manner, and for any length of time. Contacting can occur *in vivo, ex vivo,* or *in vitro.* In some embodiments, it can mean contacting the RNAi agent or composition of the present disclosure directly with a cell or tissue. In some other embodiments, the term means contacting the RNAi agent or composition of the present disclosure indirectly with a cell or tissue.

The term "subject" generally refers to a human or non-human animal (including mammals) requiring diagnosis, prognosis, amelioration, prevention, and/or treatment of a disease, such as humans, non-human primates (apes, gibbons, gorillas, chimpanzees, orangutans, and macaques), domestic animals (dogs and cats), farm animals (horses, cattle, goats, sheep, and pigs), and laboratory animals (mice, rats, rabbits, and guinea pigs). Human subjects encompass fetal, neonatal, infant, adolescent, and adult subjects. The subject also includes animal disease models.

In the present disclosure, the terms "comprising," "including," "having," "may," "containing," and variations thereof are generally intended to be open-ended transitional phrases, terms, or words that do not preclude the possibility of additional action or structure. The term "consisting of" generally means that no other components (or likewise, features, integers, steps, etc.) may be present. An indefinite number of terms also includes a plural reference unless the context clearly dictates otherwise.

mRNA sequences (SEQ ID NO: 526) encoding CFB involved in the present disclosure are as follows:

1 gggaagggaa tgtgaccagg tctaggtctg gagtttcagc ttggacactg agccaagcag
61 acaagcaaag caagccagga cacaccatcc tgccccaggc ccagcttctc tcctgccttc
121 caacgccatg gggagcaatc tcagccccca actctgcctg atgcccttta tcttgggcct
181 cttgtctgga ggtgtgacca ccactccatg gtctttggcc cggccccagg gatcctgctc
241 tctggagggg gtagagatca aaggcggctc cttccgactt ctccaagagg gccaggcact
301 ggagtacgtg tgtccttctg gcttctaccc gtaccctgtg cagacacgta cctgcagatc
361 tacggggtcc tggagcaccc tgaagactca agaccaaaag actgtcagga aggcagagtg
421 cagagcaatc cactgtccaa gaccacacga cttcgagaac ggggaatact ggccccggtc
481 tccctactac aatgtgagtg atgagatctc tttccactgc tatgacggtt acactctccg
541 gggctctgcc aatcgcacct gccaagtgaa tggccgatgg agtgggcaga cagcgatctg
601 tgacaacgga gcggggtact gctccaaccc gggcatcccc attggcacaa ggaaggtggg
661 cagccagtac cgccttgaag acagcgtcac ctaccactgc agccgggggc ttaccctgcg
721 tggctcccag cggcgaacgt gtcaggaagg tggctcttgg agcgggacgg agccttcctg
781 ccaagactcc ttcatgtacg acacccctca agaggtggcc gaagctttcc tgtcttccct
841 gacagagacc atagaaggag tcgatgctga ggatgggcac ggcccagggg aacaacagaa
901 gcggaagatc gtcctggacc cttcaggctc catgaacatc tacctggtgc tagatggatc

961 agacagcatt ggggccagca acttcacagg agccaaaaag tgtctagtca acttaattga
1021 gaaggtggca agttatggtg tgaagccaag atatggtcta gtgacatatg ccacataccc
1081 caaaatttgg gtcaaagtgt ctgaagcaga cagcagtaat gcagactggg tcacgaagca
1141 gctcaatgaa atcaattatg aagaccacaa gttgaagtca gggactaaca ccaagaaggc
1201 cctccaggca gtgtacagca tgatgagctg gccagatgac gtccctcctg aaggctggaa
1261 ccgcacccgc catgtcatca tcctcatgac tgatggattg cacaacatgg gcggggaccc
1321 aattactgtc attgatgaga tccgggactt gctatacatt ggcaaggatc gcaaaaaccc
1381 aagggaggat tatctggatg tctatgtgtt tggggtcggg cctttggtga accaagtgaa
1441 catcaatgct ttggcttcca agaaagacaa tgagcaacat gtgttcaaag tcaaggatat
1501 ggaaaacctg gaagatgttt tctaccaaat gatcgatgaa agccagtctc tgagtctctg
1561 tggcatggtt tgggaacaca ggaagggtac cgattaccac aagcaaccat ggcaggccaa
1621 gatctcagtc attcgccctt caaagggaca cgagagctgt atgggggctg tggtgtctga
1681 gtactttgtg ctgacagcag cacattgttt cactgtggat gacaaggaac actcaatcaa
1741 ggtcagcgta ggagggggaga agcgggacct ggagatagaa gtagtcctat ttcaccccaa
1801 ctacaacatt aatgggaaaa aagaagcagg aattcctgaa ttttatgact atgacgttgc
1861 cctgatcaag ctcaagaata agctgaaata tggccagact atcaggccca tttgtctccc
1921 ctgcaccgag ggaacaactc gagctttgag gcttcctcca actaccactt gccagcaaca
1981 aaaggaagag ctgctccctg cacaggatat caaagctctg tttgtgtctg aggaggagaa
2041 aaagctgact cggaaggagg tctacatcaa gaatggggat aagaaaggca gctgtgagag
2101 agatgctcaa tatgccccag gctatgacaa agtcaaggac atctcagagg tggtcacccc
2161 tcggttcctt tgtactggag gagtgagtcc ctatgctgac cccaatactt gcagaggtga
2221 ttctggcggc cccttgatag ttcacaagag aagtcgtttc attcaagttg gtgtaatcag
2281 ctggggagta gtggatgtct gcaaaaacca gaagcggcaa aagcaggtac ctgctcacgc
2341 ccgagacttt cacatcaacc tctttcaagt gctgccctgg ctgaaggaga aactccaaga
2401 tgaggatttg ggttttctat aaggggtttc ctgctggaca ggggcgtggg attgaattaa
2461 aacagctgcg acaaca

In the context of the present disclosure, unless otherwise stated, synthesis of siRNA sequences used in the present disclosure was entrusted to be completed by Kunshan Alltest Biotech Co., Ltd; synthesis of PCR primers used in the present disclosure was entrusted to be completed by Sangon Biotech (Shanghai) Co., Ltd.; human hepatocarcinoma cell line HepG2 used in the present disclosure was purchased from Wuhan Pricella Biotechnology Co., Ltd.; and experimental animals C57BL/6J mice used in the present disclosure were purchased from Zhejiang Vital River Laboratory Animal Technology Co., Ltd.

In the context of the present disclosure, unless otherwise stated, Real-time PCR detection data in the activity experiments involved in the present disclosure are all used for relative quantitative calculation of target gene mRNA in various test groups by the ΔΔCt method, and the calculation method is summarized as follows:

ΔCt (test group) = Ct (target gene in test group) - Ct (internal reference gene in test group);

ΔCt (control group)= Ct (target gene in control group) - Ct (internal reference gene in control group);

ΔΔCt (test group) = ΔCt (test group) - ΔCt (control group mean);

and

$$\Delta\Delta Ct\ (control\ group) = \Delta Ct\ (control\ group) - \Delta Ct\ (control\ group\ mean)$$

Taking the control group as benchmark, the expression level of the target gene mRNA in the test group is normalized, and the remaining expression level of the target gene mRNA in the control group is defined as 100%.

Relative remaining expression level of target gene mRNA in test group = $2^{-\Delta\Delta Ct}$ (test group) × 100%

Inhibitory rate of target gene mRNA in test group = 100% - relative expression level of target gene mRNA in test group

In the context of the present disclosure, unless otherwise stated, *in vivo* activity experimental data are all expressed by $\overline{X}\pm$STDEV, and experimental data are all plotted and analyzed using GraphPad prism 8.0 software.

In the context of the present disclosure, proportions of the reagents provided below are all calculated as volume ratio (v/v), unless otherwise stated.

Sequences of the double-stranded oligonucleotides (dsRNA) in the present disclosure are as listed in Table 1.

Table 1 Sequences of Unmodified Double-Stranded Oligonucleotides

| No. | Sense Strand | Sequence ID | Antisense Strand | Sequence ID |
|---|---|---|---|---|
| RN011001 | CCUUUAUCUUGGGCCUCUU | SEQ ID NO:1 | AAGAGGCCCAAGAUAAAGGGC | SEQ ID NO:256 |
| RN011002 | GGUGUGACCACCACUCCAU | SEQ ID NO:2 | AUGGAGUGGUGGUCACACCUC | SEQ ID NO:257 |
| RN011003 | GUAGAGAUCAAAGGCGGCU | SEQ ID NO:3 | AGCCGCCUUUGAUCUCUACCC | SEQ ID NO:258 |
| RN011004 | CAAAGGCGGCUCCUUCCGA | SEQ ID NO:4 | UCGGAAGGAGCCGCCUUUGAU | SEQ ID NO:259 |
| RN011005 | AAGGCGGCUCCUUCCGACU | SEQ ID NO:5 | AGUCGGAAGGAGCCGCCUUUG | SEQ ID NO:260 |
| RN011006 | GCGGCUCCUUCCGACUUCU | SEQ ID NO:6 | AGAAGUCGGAAGGAGCCGCCU | SEQ ID NO:261 |
| RN011007 | GCUCCUUCCGACUUCUCCA | SEQ ID NO:7 | UGGAGAAGUCGGAAGGAGCCG | SEQ ID NO:262 |
| RN011008 | CUCCUUCCGACUUCUCCAA | SEQ ID NO:8 | UUGGAGAAGUCGGAAGGAGCC | SEQ ID NO:263 |
| RN011009 | CCUUCCGACUUCUCCAAGA | SEQ ID NO:9 | UCUUGGAGAAGUCGGAAGGAG | SEQ ID NO:264 |
| RN011010 | CUUCCGACUUCUCCAAGAG | SEQ ID NO:10 | CUCUUGGAGAAGUCGGAAGGA | SEQ ID NO:265 |
| RN011011 | GACUUCUCCAAGAGGGCCA | SEQ ID NO: 11 | UGGCCCUCUUGGAGAAGUCGG | SEQ ID NO:266 |
| RN011012 | GCCAGGCACUGGAGUACGU | SEQ ID NO:12 | ACGUACUCCAGUGCCUGGCCC | SEQ ID NO:267 |
| RN011013 | GGAGUACGUGUGUCCUUCU | SEQ ID NO:13 | AGAAGGACACACGUACUCCAG | SEQ ID NO:268 |
| RN011014 | GUGUGUCCUUCUGGCUUCU | SEQ ID NO:14 | AGAAGCCAGAAGGACACACGU | SEQ ID NO:269 |
| RN011015 | CCUUCUGGCUUCUACCCGU | SEQ ID NO:15 | ACGGGUAGAAGCCAGAAGGAC | SEQ ID NO:270 |
| RN011016 | CUUCUGGCUUCUACCCGUA | SEQ ID NO:16 | UACGGGUAGAAGCCAGAAGGA | SEQ ID NO:271 |

(continued)

| No. | Sense Strand | Sequence ID | Antisense Strand | Sequence ID |
|---|---|---|---|---|
| RN011017 | GCUUCUACCCGUACCCUGU | SEQ ID NO:17 | ACAGGGUACGGGUAGAAGCCA | SEQ ID NO:272 |
| RN011018 | CCCGUACCCUGUGCAGACA | SEQ ID NO:18 | UGUCUGCACAGGGUACGGGUA | SEQ ID NO:273 |
| RN011019 | CCUGUGCAGACACGUACCU | SEQ ID NO:19 | AGGUACGUGUCUGCACAGGGU | SEQ ID NO:274 |
| RN011020 | GCAGACACGUACCUGCAGA | SEQ ID NO:20 | UCUGCAGGUACGUGUCUGCAC | SEQ ID NO:275 |
| RN011021 | CAGACACGUACCUGCAGAU | SEQ ID NO:21 | AUCUGCAGGUACGUGUCUGCA | SEQ ID NO:276 |
| RN011022 | ACACGUACCUGCAGAUCUA | SEQ ID NO:22 | UAGAUCUGCAGGUACGUGUCU | SEQ ID NO:277 |
| RN011023 | CGGGGUCCUGGAGCACCCU | SEQ ID NO:23 | AGGGUGCUCCAGGACCCCGUA | SEQ ID NO:278 |
| RN011024 | CUGGAGCACCCUGAAGACU | SEQ ID NO:24 | AGUCUUCAGGGUGCUCCAGGA | SEQ ID NO:279 |
| RN011025 | GGAGCACCCUGAAGACUCA | SEQ ID NO:25 | UGAGUCUUCAGGGUGCUCCAG | SEQ ID NO:280 |
| RN011026 | GCACCCUGAAGACUCAAGA | SEQ ID NO:26 | UCUUGAGUCUUCAGGGUGCUC | SEQ ID NO:281 |
| RN011027 | CAGGAAGGCAGAGUGCAGA | SEQ ID NO:27 | UCUGCACUCUGCCUUCCUGAC | SEQ ID NO:282 |
| RN011028 | AAGGCAGAGUGCAGAGCAA | SEQ ID NO:28 | UUGCUCUGCACUCUGCCUUCC | SEQ ID NO:283 |
| RN011029 | GUGCAGAGCAAUCCACUGU | SEQ ID NO:29 | ACAGUGGAUUGCUCUGCACUC | SEQ ID NO:284 |
| RN011030 | GACCACACGACUUCGAGAA | SEQ ID NO:30 | UUCUCGAAGUCGUGUGGUCUU | SEQ ID NO:285 |
| RN011031 | GGUCUCCCUACUACAAUGU | SEQ ID NO:31 | ACAUUGUAGUAGGGAGACCGG | SEQ ID NO:286 |
| RN011032 | CCUACUACAAUGUGAGUGA | SEQ ID NO:32 | UCACUCACAUUGUAGUAGGGA | SEQ ID NO:287 |
| RN011033 | CUACUACAAUGUGAGUGAU | SEQ ID NO:33 | AUCACUCACAUUGUAGUAGGG | SEQ ID NO:288 |
| RN011034 | CAAUGUGAGUGAUGAGAUC | SEQ ID NO:34 | GAUCUCAUCACUCACAUUGUA | SEQ ID NO:289 |
| RN011035 | GUGAUGAGAUCUCUUUCCA | SEQ ID NO:35 | UGGAAAGAGAUCUCAUCACUC | SEQ ID NO:290 |
| RN011036 | GAUGAGAUCUCUUUCCACU | SEQ ID NO:36 | AGUGGAAAGAGAUCUCAUCAC | SEQ ID NO:291 |
| RN011037 | GAGAUCUCUUUCCACUGCU | SEQ ID NO:37 | AGCAGUGGAAAGAGAUCUCAU | SEQ ID NO:292 |
| RN011038 | AGAUCUCUUUCCACUGCUA | SEQ ID NO:38 | UAGCAGUGGAAAGAGAUCUCA | SEQ ID NO:293 |

(continued)

| No. | Sense Strand | Sequence ID | Antisense Strand | Sequence ID |
|---|---|---|---|---|
| RN011039 | GAUCUCUUUCCACUGCUAU | SEQ ID NO:39 | AUAGCAGUGGAAAGAGAUCUC | SEQ ID NO:294 |
| RN011040 | GCUAUGACGGUUACACUCU | SEQ ID NO:40 | AGAGUGUAACCGUCAUAGCAG | SEQ ID NO:295 |
| RN011041 | CUGCCAAUCGCACCUGCCA | SEQ ID NO:41 | UGGCAGGUGCGAUUGGCAGAG | SEQ ID NO:296 |
| RN011042 | AAUCGCACCUGCCAAGUGA | SEQ ID NO:42 | UCACUUGGCAGGUGCGAUUGG | SEQ ID NO:297 |
| RN011043 | AUCGCACCUGCCAAGUGAA | SEQ ID NO:43 | UUCACUUGGCAGGUGCGAUUG | SEQ ID NO:298 |
| RN011044 | AGUGAAUGGCCGAUGGAGU | SEQ ID NO:44 | ACUCCAUCGGCCAUUCACUUG | SEQ ID NO:299 |
| RN011045 | GAAUGGCCGAUGGAGUGGG | SEQ ID NO:45 | CCCACUCCAUCGGCCAUUCAC | SEQ ID NO:300 |
| RN011046 | CCGAUGGAGUGGGCAGACA | SEQ ID NO:46 | UGUCUGCCCACUCCAUCGGCC | SEQ ID NO:301 |
| RN011047 | AUGGAGUGGGCAGACAGCG | SEQ ID NO:47 | CGCUGUCUGCCCACUCCAUCG | SEQ ID NO:302 |
| RN011048 | GGAGUGGGCAGACAGCGAU | SEQ ID NO:48 | AUCGCUGUCUGCCCACUCCAU | SEQ ID NO:303 |
| RN011049 | UGGGCAGACAGCGAUCUGU | SEQ ID NO:49 | ACAGAUCGCUGUCUGCCCACU | SEQ ID NO:304 |
| RN011050 | GGCAGACAGCGAUCUGUGA | SEQ ID NO:50 | UCACAGAUCGCUGUCUGCCCA | SEQ ID NO:305 |
| RN011051 | GCAGACAGCGAUCUGUGAC | SEQ ID NO:51 | GUCACAGAUCGCUGUCUGCCC | SEQ ID NO:306 |
| RN011052 | CAGACAGCGAUCUGUGACA | SEQ ID NO:52 | UGUCACAGAUCGCUGUCUGCC | SEQ ID NO:307 |
| RN011053 | GGCACAAGGAAGGUGGGCA | SEQ ID NO:53 | UGCCCACCUUCCUUGUGCCAA | SEQ ID NO:308 |
| RN011054 | GCACAAGGAAGGUGGGCAG | SEQ ID NO:54 | CUGCCCACCUUCCUUGUGCCA | SEQ ID NO:309 |
| RN011055 | CAAGGAAGGUGGGCAGCCA | SEQ ID NO:55 | UGGCUGCCCACCUUCCUUGUG | SEQ ID NO:310 |
| RN011056 | GCAGCCAGUACCGCCUUGA | SEQ ID NO:56 | UCAAGGCGGUACUGGCUGCCC | SEQ ID NO:311 |
| RN011057 | CUUGAAGACAGCGUCACCU | SEQ ID NO:57 | AGGUGACGCUGUCUUCAAGGC | SEQ ID NO:312 |
| RN011058 | GACAGCGUCACCUACCACU | SEQ ID NO:58 | AGUGGUAGGUGACGCUGUCUU | SEQ ID NO:313 |
| RN011059 | CCGGGGGCUUACCCUGCGU | SEQ ID NO:59 | ACGCAGGGUAAGCCCCCGGCU | SEQ ID NO:314 |
| RN011060 | GCGGCGAACGUGUCAGGAA | SEQ ID NO:60 | UUCCUGACACGUUCGCCGCUG | SEQ ID NO:315 |

(continued)

| No. | Sense Strand | Sequence ID | Antisense Strand | Sequence ID |
|---|---|---|---|---|
| RN011061 | GCGAACGUGUCAGGAAGGU | SEQ ID NO:61 | ACCUUCCUGACACGUUCGCCG | SEQ ID NO:316 |
| RN011062 | GUGUCAGGAAGGUGGCUCU | SEQ ID NO:62 | AGAGCCACCUUCCUGACACGU | SEQ ID NO:317 |
| RN011063 | GGAAGGUGGCUCUUGGAGC | SEQ ID NO:63 | GCUCCAAGAGCCACCUUCCUG | SEQ ID NO:318 |
| RN011064 | AGGUGGCUCUUGGAGCGGG | SEQ ID NO:64 | CCCGCUCCAAGAGCCACCUUC | SEQ ID NO:319 |
| RN011065 | GAGCGGGACGGAGCCUUCC | SEQ ID NO:65 | GGAAGGCUCCGUCCCGCUCCA | SEQ ID NO:320 |
| RN011066 | GACGGAGCCUUCCUGCCAA | SEQ ID NO:66 | UUGGCAGGAAGGCUCCGUCCC | SEQ ID NO:321 |
| RN011067 | GAGCCUUCCUGCCAAGACU | SEQ ID NO:67 | AGUCUUGGCAGGAAGGCUCCG | SEQ ID NO:322 |
| RN011068 | CCUUCCUGCCAAGACUCCU | SEQ ID NO:68 | AGGAGUCUUGGCAGGAAGGCU | SEQ ID NO:323 |
| RN011069 | CUUCCUGCCAAGACUCCUU | SEQ ID NO:69 | AAGGAGUCUUGGCAGGAAGGC | SEQ ID NO:324 |
| RN011070 | CCUGCCAAGACUCCUUCAU | SEQ ID NO:70 | AUGAAGGAGUCUUGGCAGGAA | SEQ ID NO:325 |
| RN011071 | GCCAAGACUCCUUCAUGUA | SEQ ID NO:71 | UACAUGAAGGAGUCUUGGCAG | SEQ ID NO:326 |
| RN011072 | GACUCCUUCAUGUACGACA | SEQ ID NO:72 | UGUCGUACAUGAAGGAGUCUU | SEQ ID NO:327 |
| RN011073 | ACUCCUUCAUGUACGACAC | SEQ ID NO:73 | GUGUCGUACAUGAAGGAGUCU | SEQ ID NO:328 |
| RN011074 | CUCCUUCAUGUACGACACC | SEQ ID NO:74 | GGUGUCGUACAUGAAGGAGUC | SEQ ID NO:329 |
| RN011075 | GGCCGAAGCUUUCCUGUCU | SEQ ID NO:75 | AGACAGGAAAGCUUCGGCCAC | SEQ ID NO:330 |
| RN011076 | GCCGAAGCUUUCCUGUCUU | SEQ ID NO:76 | AAGACAGGAAAGCUUCGGCCA | SEQ ID NO:331 |
| RN011077 | GCUUUCCUGUCUUCCCUGA | SEQ ID NO:77 | UCAGGGAAGACAGGAAAGCUU | SEQ ID NO:332 |
| RN011078 | CCUGACAGAGACCAUAGAA | SEQ ID NO:78 | UUCUAUGGUCUCUGUCAGGGA | SEQ ID NO:333 |
| RN011079 | GACAGAGACCAUAGAAGGA | SEQ ID NO:79 | UCCUUCUAUGGUCUCUGUCAG | SEQ ID NO:334 |
| RN011080 | CAGAGACCAUAGAAGGAGU | SEQ ID NO:80 | ACUCCUUCUAUGGUCUCUGUC | SEQ ID NO:335 |
| RN011081 | GACCAUAGAAGGAGUCGAU | SEQ ID NO:81 | AUCGACUCCUUCUAUGGUCUC | SEQ ID NO:336 |
| RN011082 | CAUAGAAGGAGUCGAUGCU | SEQ ID NO:82 | AGCAUCGACUCCUUCUAUGGU | SEQ ID NO:337 |

(continued)

| No. | Sense Strand | Sequence ID | Antisense Strand | Sequence ID |
|---|---|---|---|---|
| RN011083 | UAGAAGGAGUCGAUGCUGA | SEQ ID NO:83 | UCAGCAUCGACUCCUUCUAUG | SEQ ID NO:338 |
| RN011084 | AGGAGUCGAUGCUGAGGAU | SEQ ID NO:84 | AUCCUCAGCAUCGACUCCUUC | SEQ ID NO:339 |
| RN011085 | AUGGGCACGGCCCAGGGGA | SEQ ID NO:85 | UCCCCUGGGCCGUGCCCAUCC | SEQ ID NO:340 |
| RN011086 | GGGAACAACAGAAGCGGAA | SEQ ID NO:86 | UUCCGCUUCUGUUGUUCCCCU | SEQ ID NO:341 |
| RN011087 | GAACAACAGAAGCGGAAGA | SEQ ID NO:87 | UCUUCCGCUUCUGUUGUUCCC | SEQ ID NO:342 |
| RN011088 | CAACAGAAGCGGAAGAUCG | SEQ ID NO:88 | CGAUCUUCCGCUUCUGUUGUU | SEQ ID NO:343 |
| RN011089 | CUGGACCCUUCAGGCUCCA | SEQ ID NO:89 | UGGAGCCUGAAGGGUCCAGGA | SEQ ID NO:344 |
| RN011090 | CCUUCAGGCUCCAUGAACA | SEQ ID NO:90 | UGUUCAUGGAGCCUGAAGGGU | SEQ ID NO:345 |
| RN011091 | CAGGCUCCAUGAACAUCUA | SEQ ID NO:91 | UAGAUGUUCAUGGAGCCUGAA | SEQ ID NO:346 |
| RN011092 | GCUCCAUGAACAUCUACCU | SEQ ID NO:92 | AGGUAGAUGUUCAUGGAGCCU | SEQ ID NO:347 |
| RN011093 | CCAUGAACAUCUACCUGGU | SEQ ID NO:93 | ACCAGGUAGAUGUUCAUGGAG | SEQ ID NO:348 |
| RN011094 | AACAUCUACCUGGUGCUAG | SEQ ID NO:94 | CUAGCACCAGGUAGAUGUUCA | SEQ ID NO:349 |
| RN011095 | UACCUGGUGCUAGAUGGAU | SEQ ID NO:95 | AUCCAUCUAGCACCAGGUAGA | SEQ ID NO:350 |
| RN011096 | ACCUGGUGCUAGAUGGAUC | SEQ ID NO:96 | GAUCCAUCUAGCACCAGGUAG | SEQ ID NO:351 |
| RN011097 | CCUGGUGCUAGAUGGAUCA | SEQ ID NO:97 | UGAUCCAUCUAGCACCAGGUA | SEQ ID NO:352 |
| RN011098 | CUAGAUGGAUCAGACAGCA | SEQ ID NO:98 | UGCUGUCUGAUCCAUCUAGCA | SEQ ID NO:353 |
| RN011099 | UGGAUCAGACAGCAUUGGG | SEQ ID NO:99 | CCCAAUGCUGUCUGAUCCAUC | SEQ ID NO:354 |
| RN011100 | GCCAGCAACUUCACAGGAG | SEQ ID NO:100 | CUCCUGUGAAGUUGCUGGCCC | SEQ ID NO:355 |
| RN011101 | CAACUUCACAGGAGCCAAA | SEQ ID NO:101 | UUUGGCUCCUGUGAAGUUGCU | SEQ ID NO:356 |
| RN011102 | AAAGUGUCUAGUCAACUUA | SEQ ID NO:102 | UAAGUUGACUAGACACUUUUU | SEQ ID NO:357 |
| RN011103 | AAGUGUCUAGUCAACUUAA | SEQ ID NO:103 | UUAAGUUGACUAGACACUUUU | SEQ ID NO:358 |
| RN011104 | AGUGUCUAGUCAACUUAAU | SEQ ID NO: 104 | AUUAAGUUGACUAGACACUUU | SEQ ID NO:359 |

(continued)

| No. | Sense Strand | Sequence ID | Antisense Strand | Sequence ID |
|---|---|---|---|---|
| RN011105 | GUCUAGUCAACUUAAUUGA | SEQ ID NO:105 | UCAAUUAAGUUGACUAGACAC | SEQ ID NO:360 |
| RN011106 | CUAGUCAACUUAAUUGAGA | SEQ ID NO:106 | UCUCAAUUAAGUUGACUAGAC | SEQ ID NO:361 |
| RN011107 | UUGAGAAGGUGGCAAGUUA | SEQ ID NO:107 | UAACUUGCCACCUUCUCAAUU | SEQ ID NO:362 |
| RN011108 | GAAGGUGGCAAGUUAUGGU | SEQ ID NO:108 | ACCAUAACUUGCCACCUUCUC | SEQ ID NO:363 |
| RN011109 | GUGGCAAGUUAUGGUGUGA | SEQ ID NO: 109 | UCACACCAUAACUUGCCACCU | SEQ ID NO:364 |
| RN011110 | AGUUAUGGUGUGAAGCCAA | SEQ ID NO:110 | UUGGCUUCACACCAUAACUUG | SEQ ID NO:365 |
| RN011111 | GGUGUGAAGCCAAGAUAUG | SEQ ID NO:111 | CAUAUCUUGGCUUCACACCAU | SEQ ID NO:366 |
| RN011112 | GAAGCCAAGAUAUGGUCUA | SEQ ID NO:112 | UAGACCAUAUCUUGGCUUCAC | SEQ ID NO:367 |
| RN011113 | AAGAUAUGGUCUAGUGACA | SEQ ID NO:113 | UGUCACUAGACCAUAUCUUGG | SEQ ID NO:368 |
| RN011114 | GAUAUGGUCUAGUGACAUA | SEQ ID NO:114 | UAUGUCACUAGACCAUAUCUU | SEQ ID NO:369 |
| RN011115 | GGUCUAGUGACAUAUGCCA | SEQ ID NO:115 | UGGCAUAUGUCACUAGACCAU | SEQ ID NO:370 |
| RN011116 | CUAGUGACAUAUGCCACAU | SEQ ID NO:116 | AUGUGGCAUAUGUCACUAGAC | SEQ ID NO:371 |
| RN011117 | CCAAAAUUUGGGUCAAAGU | SEQ ID NO: 117 | ACUUUGACCCAAAUUUUGGGG | SEQ ID NO:372 |
| RN011118 | GACAGCAGUAAUGCAGACU | SEQ ID NO:118 | AGUCUGCAUUACUGCUGUCUG | SEQ ID NO:373 |
| RN011119 | GCAGUAAUGCAGACUGGGU | SEQ ID NO:119 | ACCCAGUCUGCAUUACUGCUG | SEQ ID NO:374 |
| RN011120 | AGUAAUGCAGACUGGGUCA | SEQ ID NO:120 | UGACCCAGUCUGCAUUACUGC | SEQ ID NO:375 |
| RN011121 | CAGACUGGGUCACGAAGCA | SEQ ID NO:121 | UGCUUCGUGACCCAGUCUGCA | SEQ ID NO:376 |
| RN011122 | GGGUCACGAAGCAGCUCAA | SEQ ID NO:122 | UUGAGCUGCUUCGUGACCCAG | SEQ ID NO:377 |
| RN011123 | GCAGCUCAAUGAAAUCAAU | SEQ ID NO:123 | AUUGAUUUCAUUGAGCUGCUU | SEQ ID NO:378 |
| RN011124 | GCUCAAUGAAAUCAAUUAU | SEQ ID NO:124 | AUAAUUGAUUUCAUUGAGCUG | SEQ ID NO:379 |
| RN011125 | CAAUGAAAUCAAUUAUGAA | SEQ ID NO:125 | UUCAUAAUUGAUUUCAUUGAG | SEQ ID NO:380 |
| RN011126 | UCAAUUAUGAAGACCACAA | SEQ ID NO:126 | UUGUGGUCUUCAUAAUUGAUU | SEQ ID NO:381 |

(continued)

| No. | Sense Strand | Sequence ID | Antisense Strand | Sequence ID |
|---|---|---|---|---|
| RN011127 | AUGAAGACCACAAGUUGAA | SEQ ID NO:127 | UUCAACUUGUGGUCUUCAUAA | SEQ ID NO:382 |
| RN011128 | GAAGACCACAAGUUGAAGU | SEQ ID NO:128 | ACUUCAACUUGUGGUCUUCAU | SEQ ID NO:383 |
| RN011129 | CACAAGUUGAAGUCAGGGA | SEQ ID NO:129 | UCCCUGACUUCAACUUGUGGU | SEQ ID NO:384 |
| RN011130 | AGUUGAAGUCAGGGACUAA | SEQ ID NO:130 | UUAGUCCCUGACUUCAACUUG | SEQ ID NO:385 |
| RN011131 | CAGGGACUAACACCAAGAA | SEQ ID NO:131 | UUCUUGGUGUUAGUCCCUGAC | SEQ ID NO:386 |
| RN011132 | CUAACACCAAGAAGGCCCU | SEQ ID NO: 132 | AGGGCCUUCUUGGUGUUAGUC | SEQ ID NO:387 |
| RN011133 | CAGGCAGUGUACAGCAUGA | SEQ ID NO:133 | UCAUGCUGUACACUGCCUGGA | SEQ ID NO:388 |
| RN011134 | GCAGUGUACAGCAUGAUGA | SEQ ID NO:134 | UCAUCAUGCUGUACACUGCCU | SEQ ID NO:389 |
| RN011135 | GUGUACAGCAUGAUGAGCU | SEQ ID NO:135 | AGCUCAUCAUGCUGUACACUG | SEQ ID NO:390 |
| RN011136 | CCUGAAGGCUGGAACCGCA | SEQ ID NO:136 | UGCGGUUCCAGCCUUCAGGAG | SEQ ID NO:391 |
| RN011137 | GCACCCGCCAUGUCAUCAU | SEQ ID NO:137 | AUGAUGACAUGGCGGGUGCGG | SEQ ID NO:392 |
| RN011138 | CCCGCCAUGUCAUCAUCCU | SEQ ID NO:138 | AGGAUGAUGACAUGGCGGGUG | SEQ ID NO:393 |
| RN011139 | CGCCAUGUCAUCAUCCUCA | SEQ ID NO: 139 | UGAGGAUGAUGACAUGGCGGG | SEQ ID NO:394 |
| RN011140 | GCCAUGUCAUCAUCCUCAU | SEQ ID NO:140 | AUGAGGAUGAUGACAUGGCGG | SEQ ID NO:395 |
| RN011141 | CAUGUCAUCAUCCUCAUGA | SEQ ID NO:141 | UCAUGAGGAUGAUGACAUGGC | SEQ ID NO:396 |
| RN011142 | CCUCAUGACUGAUGGAUUG | SEQ ID NO:142 | CAAUCCAUCAGUCAUGAGGAU | SEQ ID NO:397 |
| RN011143 | UGACUGAUGGAUUGCACAA | SEQ ID NO:143 | UUGUGCAAUCCAUCAGUCAUG | SEQ ID NO:398 |
| RN011144 | CUGAUGGAUUGCACAACAU | SEQ ID NO:144 | AUGUUGUGCAAUCCAUCAGUC | SEQ ID NO:399 |
| RN011145 | CCCAAUUACUGUCAUUGAU | SEQ ID NO:145 | AUCAAUGACAGUAAUUGGGUC | SEQ ID NO:400 |
| RN011146 | CAAUUACUGUCAUUGAUGA | SEQ ID NO:146 | UCAUCAAUGACAGUAAUUGGG | SEQ ID NO:401 |
| RN011147 | CCGGGACUUGCUAUACAUU | SEQ ID NO:147 | AAUGUAUAGCAAGUCCCGGAU | SEQ ID NO:402 |
| RN011148 | CAAGGGAGGAUUAUCUGGA | SEQ ID NO:148 | UCCAGAUAAUCCUCCCUUGGG | SEQ ID NO:403 |

(continued)

| No. | Sense Strand | Sequence ID | Antisense Strand | Sequence ID |
|---|---|---|---|---|
| RN011149 | GGGAGGAUUAUCUGGAUGU | SEQ ID NO:149 | ACAUCCAGAUAAUCCUCCCUU | SEQ ID NO:404 |
| RN011150 | AGGAUUAUCUGGAUGUCUA | SEQ ID NO:150 | UAGACAUCCAGAUAAUCCUCC | SEQ ID NO:405 |
| RN011151 | GGAUUAUCUGGAUGUCUAU | SEQ ID NO:151 | AUAGACAUCCAGAUAAUCCUC | SEQ ID NO:406 |
| RN011152 | UCUGGAUGUCUAUGUGUUU | SEQ ID NO:152 | AAACACAUAGACAUCCAGAUA | SEQ ID NO:407 |
| RN011153 | CUUUGGUGAACCAAGUGAA | SEQ ID NO:153 | UUCACUUGGUUCACCAAAGGC | SEQ ID NO:408 |
| RN011154 | UGAACCAAGUGAACAUCAA | SEQ ID NO:154 | UUGAUGUUCACUUGGUUCACC | SEQ ID NO:409 |
| RN011155 | CAAGUGAACAUCAAUGCUU | SEQ ID NO:155 | AAGCAUUGAUGUUCACUUGGU | SEQ ID NO:410 |
| RN011156 | AAGUGAACAUCAAUGCUUU | SEQ ID NO:156 | AAAGCAUUGAUGUUCACUUGG | SEQ ID NO:411 |
| RN011157 | GAACAUCAAUGCUUUGGCU | SEQ ID NO:157 | AGCCAAAGCAUUGAUGUUCAC | SEQ ID NO:412 |
| RN011158 | CUUCCAAGAAAGACAAUGA | SEQ ID NO:158 | UCAUUGUCUUUCUUGGAAGCC | SEQ ID NO:413 |
| RN011159 | CCAAGAAAGACAAUGAGCA | SEQ ID NO:159 | UGCUCAUUGUCUUUCUUGGAA | SEQ ID NO:414 |
| RN011160 | CAAGAAAGACAAUGAGCAA | SEQ ID NO:160 | UUGCUCAUUGUCUUUCUUGGA | SEQ ID NO:415 |
| RN011161 | AGAAAGACAAUGAGCAACA | SEQ ID NO:161 | UGUUGCUCAUUGUCUUUCUUG | SEQ ID NO:416 |
| RN011162 | GAAAGACAAUGAGCAACAU | SEQ ID NO:162 | AUGUUGCUCAUUGUCUUUCUU | SEQ ID NO:417 |
| RN011163 | CAACAUGUGUUCAAAGUCA | SEQ ID NO:163 | UGACUUUGAACACAUGUUGCU | SEQ ID NO:418 |
| RN011164 | AACAUGUGUUCAAAGUCAA | SEQ ID NO:164 | UUGACUUUGAACACAUGUUGC | SEQ ID NO:419 |
| RN011165 | GUGUUCAAAGUCAAGGAUA | SEQ ID NO:165 | UAUCCUUGACUUUGAACACAU | SEQ ID NO:420 |
| RN011166 | UGUUCAAAGUCAAGGAUAU | SEQ ID NO:166 | AUAUCCUUGACUUUGAACACA | SEQ ID NO:421 |
| RN011167 | CAAAGUCAAGGAUAUGGAA | SEQ ID NO:167 | UUCCAUAUCCUUGACUUUGAA | SEQ ID NO:422 |
| RN011168 | GGAAAACCUGGAAGAUGUU | SEQ ID NO:168 | AACAUCUUCCAGGUUUUCCAU | SEQ ID NO:423 |
| RN011169 | GAUCGAUGAAAGCCAGUCU | SEQ ID NO:169 | AGACUGGCUUUCAUCGAUCAU | SEQ ID NO:424 |
| RN011170 | GCCAGUCUCUGAGUCUCUG | SEQ ID NO:170 | CAGAGACUCAGAGACUGGCUU | SEQ ID NO:425 |

(continued)

| No. | Sense Strand | Sequence ID | Antisense Strand | Sequence ID |
|---|---|---|---|---|
| RN011171 | CUCUGAGUCUCUGUGGCAU | SEQ ID NO:171 | AUGCCACAGAGACUCAGAGAC | SEQ ID NO:426 |
| RN011172 | GAGUCUCUGUGGCAUGGUU | SEQ ID NO:172 | AACCAUGCCACAGAGACUCAG | SEQ ID NO:427 |
| RN011173 | GUGGCAUGGUUUGGGAACA | SEQ ID NO:173 | UGUUCCCAAACCAUGCCACAG | SEQ ID NO:428 |
| RN011174 | GGCAUGGUUUGGGAACACA | SEQ ID NO:174 | UGUGUUCCCAAACCAUGCCAC | SEQ ID NO:429 |
| RN011175 | GUACCGAUUACCACAAGCA | SEQ ID NO:175 | UGCUUGUGGUAAUCGGUACCC | SEQ ID NO:430 |
| RN011176 | GAUUACCACAAGCAACCAU | SEQ ID NO:176 | AUGGUUGCUUGUGGUAAUCGG | SEQ ID NO:431 |
| RN011177 | GGCAGGCCAAGAUCUCAGU | SEQ ID NO: 177 | ACUGAGAUCUUGGCCUGCCAU | SEQ ID NO:432 |
| RN011178 | CAGGCCAAGAUCUCAGUCA | SEQ ID NO:178 | UGACUGAGAUCUUGGCCUGCC | SEQ ID NO:433 |
| RN011179 | CUCAGUCAUUCGCCCUUCA | SEQ ID NO:179 | UGAAGGGCGAAUGACUGAGAU | SEQ ID NO:434 |
| RN011180 | GCUGUGGUGUCUGAGUACU | SEQ ID NO:180 | AGUACUCAGACACCACAGCCC | SEQ ID NO:435 |
| RN011181 | GCUGACAGCAGCACAUUGU | SEQ ID NO:181 | ACAAUGUGCUGCUGUCAGCAC | SEQ ID NO:436 |
| RN011182 | CACAUUGUUUCACUGUGGA | SEQ ID NO:182 | UCCACAGUGAAACAAUGUGCU | SEQ ID NO:437 |
| RN011183 | GUUUCACUGUGGAUGACAA | SEQ ID NO:183 | UUGUCAUCCACAGUGAAACAA | SEQ ID NO:438 |
| RN011184 | CACUGUGGAUGACAAGGAA | SEQ ID NO:184 | UUCCUUGUCAUCCACAGUGAA | SEQ ID NO:439 |
| RN011185 | CUGUGGAUGACAAGGAACA | SEQ ID NO:185 | UGUUCCUUGUCAUCCACAGUG | SEQ ID NO:440 |
| RN011186 | GUGGAUGACAAGGAACACU | SEQ ID NO:186 | AGUGUUCCUUGUCAUCCACAG | SEQ ID NO:441 |
| RN011187 | ACAAGGAACACUCAAUCAA | SEQ ID NO:187 | UUGAUUGAGUGUUCCUUGUCA | SEQ ID NO:442 |
| RN011188 | GAACACUCAAUCAAGGUCA | SEQ ID NO:188 | UGACCUUGAUUGAGUGUUCCU | SEQ ID NO:443 |
| RN011189 | GCGGGACCUGGAGAUAGAA | SEQ ID NO:189 | UUCUAUCUCCAGGUCCCGCUU | SEQ ID NO:444 |
| RN011190 | GGGACCUGGAGAUAGAAGU | SEQ ID NO:190 | ACUUCUAUCUCCAGGUCCCGC | SEQ ID NO:445 |
| RN011191 | GGAGAUAGAAGUAGUCCUA | SEQ ID NO:191 | UAGGACUACUUCUAUCUCCAG | SEQ ID NO:446 |
| RN011192 | CUAUUUCACCCCAACUACA | SEQ ID NO:192 | UGUAGUUGGGGUGAAAUAGGA | SEQ ID NO:447 |

(continued)

| No. | Sense Strand | Sequence ID | Antisense Strand | Sequence ID |
|---|---|---|---|---|
| RN011193 | GAAGCAGGAAUUCCUGAAU | SEQ ID NO:193 | AUUCAGGAAUUCCUGCUUCUU | SEQ ID NO:448 |
| RN011194 | CAGGAAUUCCUGAAUUUUA | SEQ ID NO:194 | UAAAAUUCAGGAAUUCCUGCU | SEQ ID NO:449 |
| RN011195 | GAAUUCCUGAAUUUUAUGA | SEQ ID NO:195 | UCAUAAAAUUCAGGAAUUCCU | SEQ ID NO:450 |
| RN011196 | CUGAAUUUUAUGACUAUGA | SEQ ID NO:196 | UCAUAGUCAUAAAAUUCAGGA | SEQ ID NO:451 |
| RN011197 | GUUGCCCUGAUCAAGCUCA | SEQ ID NO:197 | UGAGCUUGAUCAGGGCAACGU | SEQ ID NO:452 |
| RN011198 | GCCCUGAUCAAGCUCAAGA | SEQ ID NO:198 | UCUUGAGCUUGAUCAGGGCAA | SEQ ID NO:453 |
| RN011199 | CCCUGAUCAAGCUCAAGAA | SEQ ID NO:199 | UUCUUGAGCUUGAUCAGGGCA | SEQ ID NO:454 |
| RN011200 | CCUGAUCAAGCUCAAGAAU | SEQ ID NO:200 | AUUCUUGAGCUUGAUCAGGGC | SEQ ID NO:455 |
| RN011201 | UGAUCAAGCUCAAGAAUAA | SEQ ID NO:201 | UUAUUCUUGAGCUUGAUCAGG | SEQ ID NO:456 |
| RN011202 | GCUCAAGAAUAAGCUGAAA | SEQ ID NO:202 | UUUCAGCUUAUUCUUGAGCUU | SEQ ID NO:457 |
| RN011203 | GCCAGACUAUCAGGCCCAU | SEQ ID NO:203 | AUGGGCCUGAUAGUCUGGCCA | SEQ ID NO:458 |
| RN011204 | CCAGACUAUCAGGCCCAUU | SEQ ID NO:204 | AAUGGGCCUGAUAGUCUGGCC | SEQ ID NO:459 |
| RN011205 | CAGACUAUCAGGCCCAUUU | SEQ ID NO:205 | AAAUGGGCCUGAUAGUCUGGC | SEQ ID NO:460 |
| RN011206 | GACUAUCAGGCCCAUUUGU | SEQ ID NO:206 | ACAAAUGGGCCUGAUAGUCUG | SEQ ID NO:461 |
| RN011207 | CACCGAGGGAACAACUCGA | SEQ ID NO:207 | UCGAGUUGUUCCCUCGGUGCA | SEQ ID NO:462 |
| RN011208 | CGAGGGAACAACUCGAGCU | SEQ ID NO:208 | AGCUCGAGUUGUUCCCUCGGU | SEQ ID NO:463 |
| RN011209 | GAGGGAACAACUCGAGCUU | SEQ ID NO:209 | AAGCUCGAGUUGUUCCCUCGG | SEQ ID NO:464 |
| RN011210 | GCUUUGAGGCUUCCUCCAA | SEQ ID NO:210 | UUGGAGGAAGCCUCAAAGCUC | SEQ ID NO:465 |
| RN011211 | GCUUCCUCCAACUACCACU | SEQ ID NO:211 | AGUGGUAGUUGGAGGAAGCCU | SEQ ID NO:466 |
| RN011212 | CUUCCUCCAACUACCACUU | SEQ ID NO:212 | AAGUGGUAGUUGGAGGAAGCC | SEQ ID NO:467 |
| RN011213 | GCUGCUCCCUGCACAGGAU | SEQ ID NO:213 | AUCCUGUGCAGGGAGCAGCUC | SEQ ID NO:468 |
| RN011214 | CCCUGCACAGGAUAUCAAA | SEQ ID NO:214 | UUUGAUAUCCUGUGCAGGGAG | SEQ ID NO:469 |

(continued)

| No. | Sense Strand | Sequence ID | Antisense Strand | Sequence ID |
|---|---|---|---|---|
| RN011215 | CACAGGAUAUCAAAGCUCU | SEQ ID NO:215 | AGAGCUUUGAUAUCCUGUGCA | SEQ ID NO:470 |
| RN011216 | CAGGAUAUCAAAGCUCUGU | SEQ ID NO:216 | ACAGAGCUUUGAUAUCCUGUG | SEQ ID NO:471 |
| RN011217 | AGGAUAUCAAAGCUCUGUU | SEQ ID NO:217 | AACAGAGCUUUGAUAUCCUGU | SEQ ID NO:472 |
| RN011218 | GGAUAUCAAAGCUCUGUUU | SEQ ID NO:218 | AAACAGAGCUUUGAUAUCCUG | SEQ ID NO:473 |
| RN011219 | GAUAUCAAAGCUCUGUUUG | SEQ ID NO:219 | CAAACAGAGCUUUGAUAUCCU | SEQ ID NO:474 |
| RN011220 | CUCUGUUUGUGUCUGAGGA | SEQ ID NO:220 | UCCUCAGACACAAACAGAGCU | SEQ ID NO:475 |
| RN011221 | CUGACUCGGAAGGAGGUCU | SEQ ID NO:221 | AGACCUCCUUCCGAGUCAGCU | SEQ ID NO:476 |
| RN011222 | UGACUCGGAAGGAGGUCUA | SEQ ID NO:222 | UAGACCUCCUUCCGAGUCAGC | SEQ ID NO:477 |
| RN011223 | GACUCGGAAGGAGGUCUAC | SEQ ID NO:223 | GUAGACCUCCUUCCGAGUCAG | SEQ ID NO:478 |
| RN011224 | CUCGGAAGGAGGUCUACAU | SEQ ID NO:224 | AUGUAGACCUCCUUCCGAGUC | SEQ ID NO:479 |
| RN011225 | GGAAGGAGGUCUACAUCAA | SEQ ID NO:225 | UUGAUGUAGACCUCCUUCCGA | SEQ ID NO:480 |
| RN011226 | GAAGGAGGUCUACAUCAAG | SEQ ID NO:226 | CUUGAUGUAGACCUCCUUCCG | SEQ ID NO:481 |
| RN011227 | GGAGGUCUACAUCAAGAAU | SEQ ID NO:227 | AUUCUUGAUGUAGACCUCCUU | SEQ ID NO:482 |
| RN011228 | GAGGUCUACAUCAAGAAUG | SEQ ID NO:228 | CAUUCUUGAUGUAGACCUCCU | SEQ ID NO:483 |
| RN011229 | CAAGAAUGGGGAUAAGAAA | SEQ ID NO:229 | UUUCUUAUCCCCAUUCUUGAU | SEQ ID NO:484 |
| RN011230 | GCUGUGAGAGAGAUGCUCA | SEQ ID NO:230 | UGAGCAUCUCUCUCACAGCUG | SEQ ID NO:485 |
| RN011231 | CUGUGAGAGAGAUGCUCAA | SEQ ID NO:231 | UUGAGCAUCUCUCUCACAGCU | SEQ ID NO:486 |
| RN011232 | GUGAGAGAGAUGCUCAAUA | SEQ ID NO:232 | UAUUGAGCAUCUCUCUCACAG | SEQ ID NO:487 |
| RN011233 | CAGGCUAUGACAAAGUCAA | SEQ ID NO:233 | UUGACUUUGUCAUAGCCUGGG | SEQ ID NO:488 |
| RN011234 | GCUAUGACAAAGUCAAGGA | SEQ ID NO:234 | UCCUUGACUUUGUCAUAGCCU | SEQ ID NO:489 |
| RN011235 | GACAAAGUCAAGGACAUCU | SEQ ID NO:235 | AGAUGUCCUUGACUUUGUCAU | SEQ ID NO:490 |
| RN011236 | GUUCCUUUGUACUGGAGGA | SEQ ID NO:236 | UCCUCCAGUACAAAGGAACCG | SEQ ID NO:491 |

(continued)

| No. | Sense Strand | Sequence ID | Antisense Strand | Sequence ID |
|---|---|---|---|---|
| RN011237 | CUGGAGGAGUGAGUCCCUA | SEQ ID NO:237 | UAGGGACUCACUCCUCCAGUA | SEQ ID NO:492 |
| RN011238 | CUUGAUAGUUCACAAGAGA | SEQ ID NO:238 | UCUCUUGUGAACUAUCAAGGG | SEQ ID NO:493 |
| RN011239 | GUUCACAAGAGAAGUCGUU | SEQ ID NO:239 | AACGACUUCUCUUGUGAACUA | SEQ ID NO:494 |
| RN011240 | CACAAGAGAAGUCGUUUCA | SEQ ID NO:240 | UGAAACGACUUCUCUUGUGAA | SEQ ID NO:495 |
| RN011241 | CAAGAGAAGUCGUUUCAUU | SEQ ID NO:241 | AAUGAAACGACUUCUCUUGUG | SEQ ID NO:496 |
| RN011242 | AGAGAAGUCGUUUCAUUCA | SEQ ID NO:242 | UGAAUGAAACGACUUCUCUUG | SEQ ID NO:497 |
| RN011243 | GAGAAGUCGUUUCAUUCAA | SEQ ID NO:243 | UUGAAUGAAACGACUUCUCUU | SEQ ID NO:498 |
| RN011244 | GAAGUCGUUUCAUUCAAGU | SEQ ID NO:244 | ACUUGAAUGAAACGACUUCUC | SEQ ID NO:499 |
| RN011245 | GUUUCAUUCAAGUUGGUGU | SEQ ID NO:245 | ACACCAACUUGAAUGAAACGA | SEQ ID NO:500 |
| RN011246 | CCCGAGACUUUCACAUCAA | SEQ ID NO:246 | UUGAUGUGAAAGUCUCGGGCG | SEQ ID NO:501 |
| RN011247 | GAGACUUUCACAUCAACCU | SEQ ID NO:247 | AGGUUGAUGUGAAAGUCUCGG | SEQ ID NO:502 |
| RN011248 | GACUUUCACAUCAACCUCU | SEQ ID NO:248 | AGAGGUUGAUGUGAAAGUCUC | SEQ ID NO:503 |
| RN011249 | CCUGGCUGAAGGAGAAACU | SEQ ID NO:249 | AGUUUCUCCUUCAGCCAGGGC | SEQ ID NO:504 |
| RN011250 | GGCUGAAGGAGAAACUCCA | SEQ ID NO:250 | UGGAGUUUCUCCUUCAGCCAG | SEQ ID NO:505 |
| RN011251 | GCUGAAGGAGAAACUCCAA | SEQ ID NO:251 | UUGGAGUUUCUCCUUCAGCCA | SEQ ID NO:506 |
| RN011252 | GAAGGAGAAACUCCAAGAU | SEQ ID NO:252 | AUCUUGGAGUUUCUCCUUCAG | SEQ ID NO:507 |
| RN011253 | GAAACUCCAAGAUGAGGAU | SEQ ID NO:253 | AUCCUCAUCUUGGAGUUUCUC | SEQ ID NO:508 |
| RN011254 | GAGGAUUUGGGUUUUCUAU | SEQ ID NO:254 | AUAGAAAACCCAAAUCCUCAU | SEQ ID NO:509 |
| RN011255 | GGAUUUGGGUUUUCUAUAA | SEQ ID NO:255 | UUAUAGAAAACCCAAAUCCUC | SEQ ID NO:510 |

**Preparation of Ligands**

**Preparation Example 1 Preparation of CR01008 Ligand**

(1.1) Synthesis of Compound CR01008

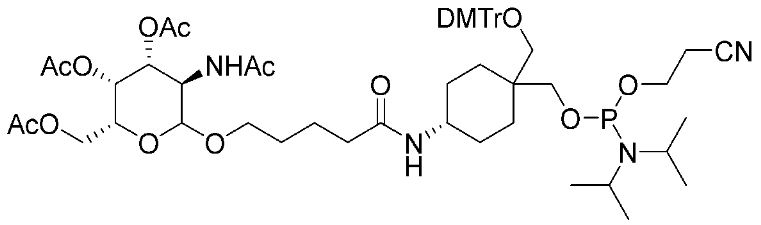

CR01008

The synthesis route of compound CR01008 was as follows:

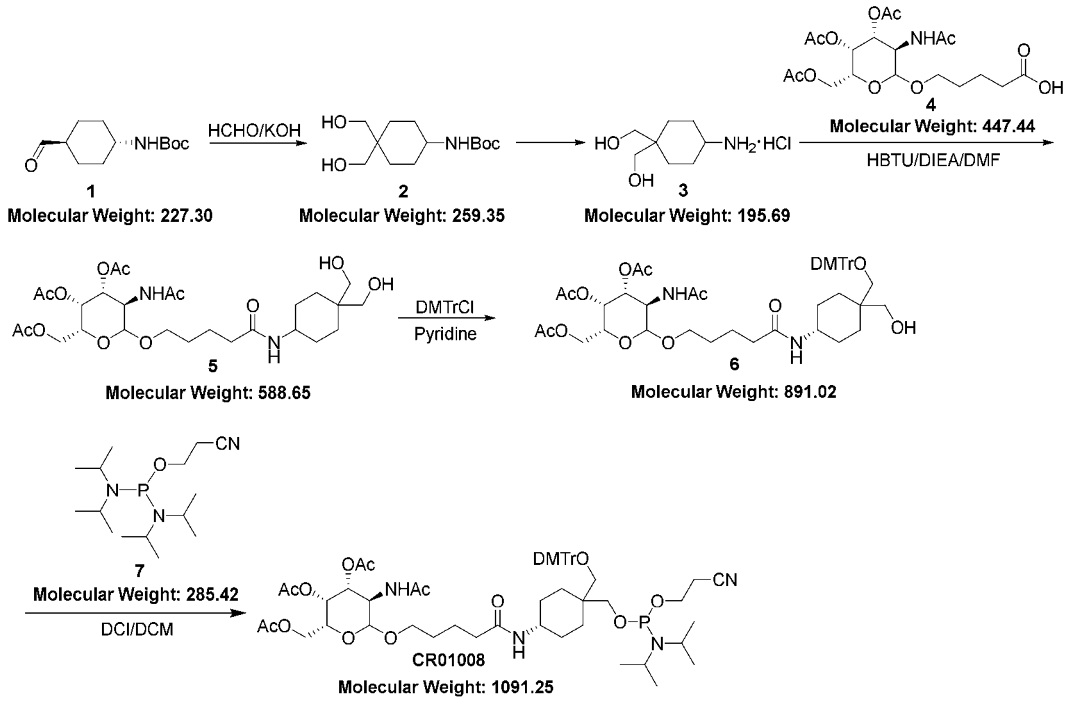


(1.1.1) Synthesis of Compound 2

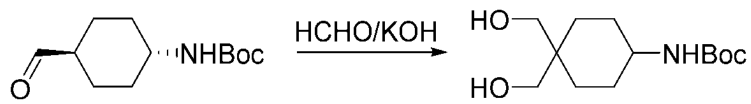


1 2

Molecular Weight: 227.30 Molecular Weight: 259.35

The compound 1 (trans-4-(Boc-amino)cyclohexanecarboxaldehyde, 10.0 g, 1.0 eq) and aqueous formaldehyde solution (8.9 g, 37 mass%, 2.4 eq) were dissolved in 33 ml of methanol. 13 ml of 45.3 wt% aqueous KOH solution was added dropwise. Upon completion of the dropwise addition, the mixture was stirred and reacted at 25 °C for 30 minutes, and resultant was heated to 60 °C and subjected to reflux reaction at 60 °C for 2 hours. After the reaction was quenched, when the reaction solution was cooled to room temperature, the reaction solution was evaporated to dryness under reduced pressure, providing the white solid-like crude product. The crude product was slurried by adding a small amount of water and filtered, to provide compound 2 as a white solid (9 g, in a yield of 78.9%). MS-ESI (*m*/*z*) = 260 [M - H]$^+$.

(1.1.2) Synthesis of Compound 3

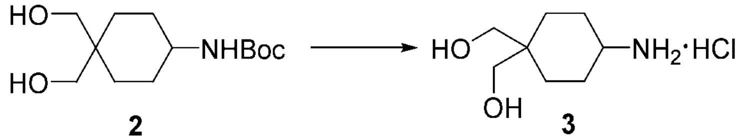

2 3

**Molecular Weight: 259.35** **Molecular Weight: 195.69**

The compound 2 (9 g, 1 eq) prepared according to step (1.1.1) was dissolved in 70 ml of 1,4-dioxane. A hydrogen chloride solution in 1,4-dioxane (45 ml, 4 M) was added. The mixture was stirred and reacted at 25 °C for 1 hour. After the reaction was quenched, the reaction solution was evaporated to dryness under reduced pressure, to provide compound 3 as a white solid (6.8 g, in a yield of 100%).

(1.1.3) Synthesis of Compound 5

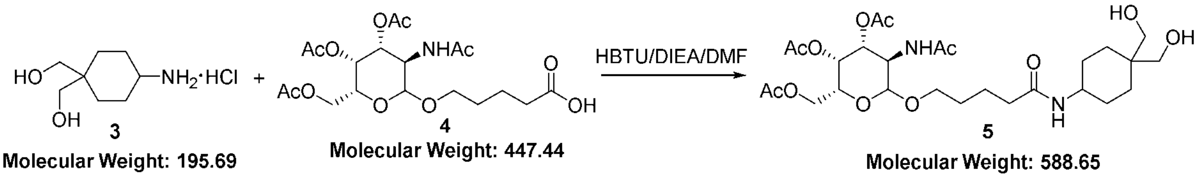


3 4 5

**Molecular Weight: 195.69** **Molecular Weight: 447.44** **Molecular Weight: 588.65**

The compound 3 (1.8 g, 2.0 eq) prepared according to step (1.1.2), the compound 4 (5-[[(2R,3R,4R,5R,6R)-3-acetamido-4,5-diacetoxy-6-(acetoxymethyl)-2-tetrahydropyranyl]oxy]pentanoic acid, 2.1 g, 1.0 eq), and DIEA (N,N-diisopropylethylamine, 3.5 g, 6.0 eq) were dissolved in 15 ml of DMF. HBTU (1.9 g, 1.1 eq) was added. The mixture was stirred and reacted at 25 °C for 3 hours in a $N_2$ atmosphere. After the reaction was quenched, the reaction solution was evaporated to dryness under reduced pressure and subjected to reverse phase purification (22% (v/v) acetonitrile in water), to provide compound 5 as a white solid (1.78 g, in a yield of 64.4%). MS-ESI (*m*/*z*) = 589[M + H]$^+$.

(1.1.4) Synthesis of Compound 6

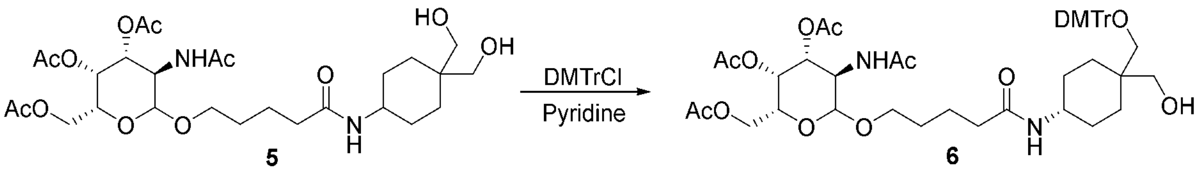


5 6

**Molecular Weight: 588.65** **Molecular Weight: 891.02**

The compound 5 (1.54 g, 1.0 eq) prepared according to step (1.1.3) was dissolved in 15 ml of pyridine. The reaction system was cooled to 0 °C using ice water bath, and DMTrCl (4,4'-dimethoxy triphenylchloromethane, 1.32 g, 1.5 eq) was added at 0 °C. The reaction was carried out at 25 °C for 3 hours, and 15 ml of methanol was added to the reaction solution to quench the reaction. After the reaction was quenched, the reaction solution was evaporated to dryness under reduced pressure and subjected to reverse phase purification (60% (v/v) acetonitrile in water), to provide compound 6 as a yellow solid (1 g, in a yield of 42.7%). MS-ESI (*m*/*z*) = 891 [M + H]$^+$.

(1.1.5) Synthesis of Compound CR01008

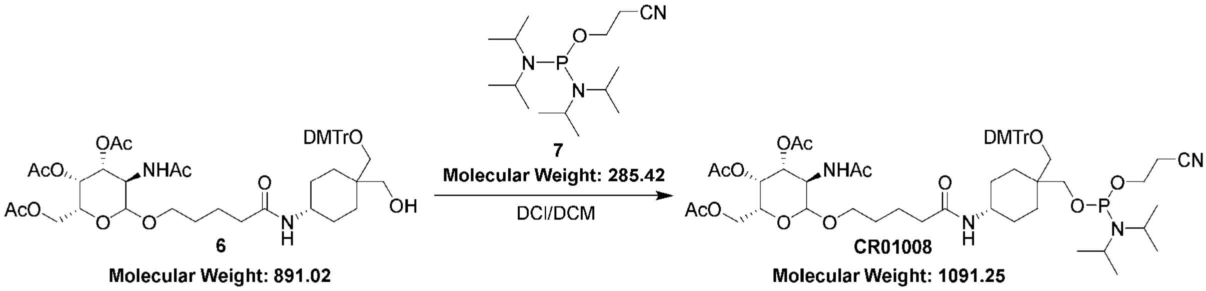


The compound 6 (1.08 g, 1.0 eq) prepared according to step (1.1.4) was dissolved in 20 ml of anhydrous dichloromethane. DCI (115 mg, 0.8 eq) and the compound 7 (bis(diisopropylamino)(2-cyanoethoxy)phosphine, 732 mg, 2.1 eq) were added respectively. Nitrogen replacement was carried out three times, and the resultant was stirred and reacted at 25 °C for 2 hours. After the reaction was quenched, 20 ml of saturated aqueous sodium bicarbonate solution was added to the reaction solution. Extraction was performed with 20 ml of dichloromethane three times (3×20 ml). The organic phases were combined. The organic phases were evaporated to dryness under reduced pressure, subjected to reverse phase purification (72% (v/v) acetonitrile in water), and then vacuum-dried for 12 hours, to provide compound CR01008 as a white powder (1 g, in a yield of 76.0%). MS-ESI (m/z) = 1091 $[M + Na]^+$.

$^1$H NMR (400 MHz, DMSO-$d_6$) δ 1.05 (d, *J* = 6.7 Hz, 6H).1.14 (d, *J* = 6.7 Hz, 6H), 1.37 - 1.17 (m, 5H), 1.60 - 1.40 (m, 6H),1.68 - 1.62 (m, 1H),1.80 (s, 3H),1.80 (s, 3H),1.92 (s, 3H), 2.02 (s, 5H),2.13 (s, 3H),2.71 (t, *J* = 5.9 Hz, 2H), 2.79 (d, *J* = 8.4 Hz, 1H), 2.87 (d, *J* = 8.4 Hz, 1H),3.36 (s, 1H), 3.58 - 3.39 (m, 3H), 3.69 - 3.60 (m, 2H), 3.75 (s, 7H), 3.90 (dt, *J* = 11.2, 8.8 Hz, 1H), 4.05 (s, 3H),4.51 (d, *J* = 8.4 Hz, 1H), 4.99 (dd, *J* = 11.3, 3.4 Hz, 1H), 5.24 (d, *J* = 3.4 Hz, 1H), 5.78 (s, 1H), 6.93 - 6.87 (m, 4H),7.35 - 7.21 (m, 7H), 7.44 - 7.37 (m, 2H), 7.66 (d, *J* = 7.8 Hz, 1H), 7.84 (d, *J* = 9.2 Hz, 1H).

(1.2) Synthesis of Compound CR01008Z

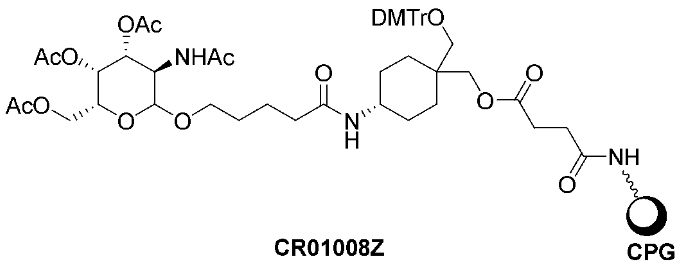

CR01008Z

The compound CR01008Z was obtained by linking the compound 6 synthesizing the compound CR01008 to solid-phase support CPG.

The synthesis route of the compound CR01008Z was as follows:

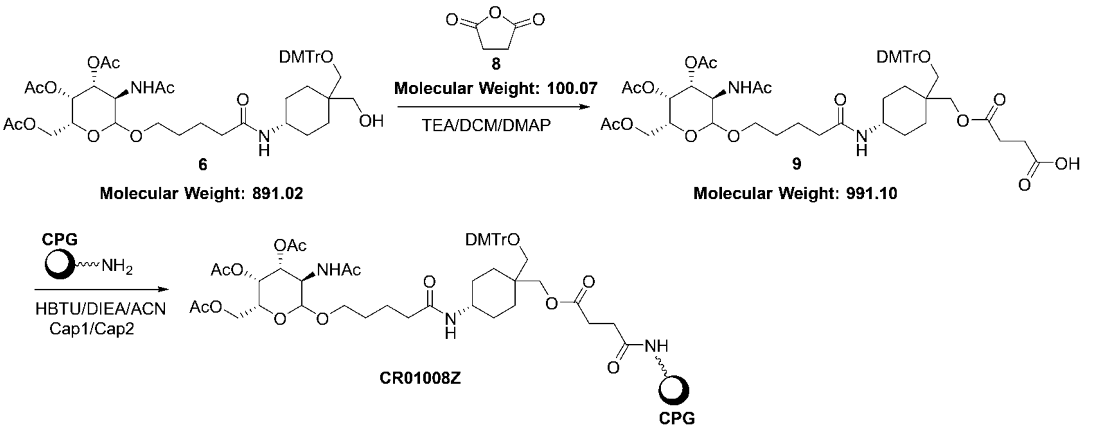

(1.2.1) Synthesis of Compound 9

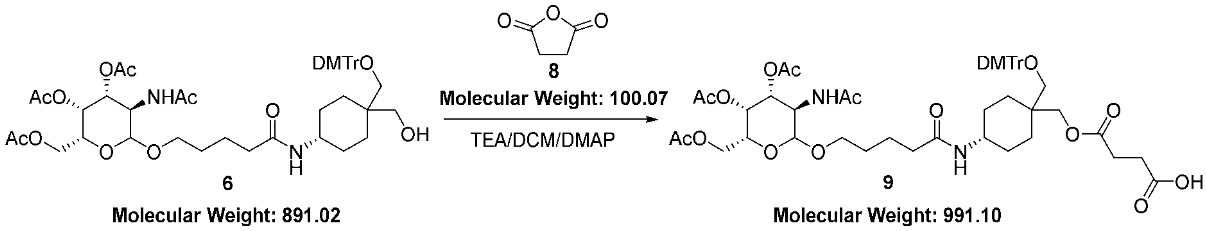


6

Molecular Weight: 891.02

9

Molecular Weight: 991.10

The compound 6 (500 mg) prepared according to step (1.1.4) was dissolved in 10 ml of dichloromethane. The compound 8 (succinic anhydride, 112 mg), DMAP (6.8 mg), and TEA (226.2 mg) were added. Nitrogen replacement was carried out three times. The resultant was stirred and reacted at 25 °C for 16 hours, and subjected to flash-purification, to provide compound 9 (300 mg, in a yield of 53.6%). MS-ESI (m/z) = 1013 $[M + Na]^+$.

(1.2.2) Synthesis of Compound CR01008Z

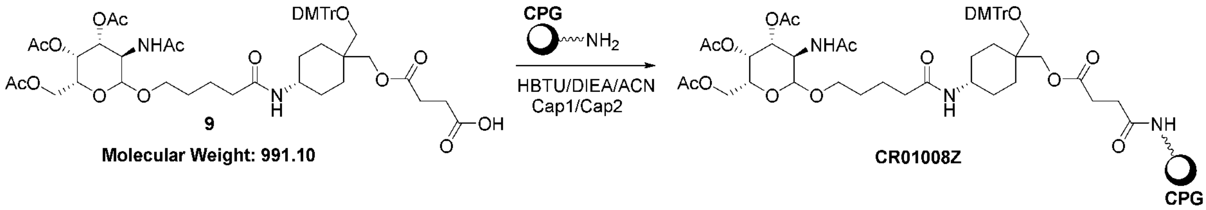


9

Molecular Weight: 991.10

CR01008Z

To a 20 ml sample vial, the compound 9 (50 mg) prepared according to step (1.2.1), amino CPG (1.25 g, 80 μmol/g, 0.1 mmol), HBTU (27 mg), and DIEA (12 mg) were added, and reacted on a shaker for 16 hours. After the reaction was quenched, a reaction solution was filtered, yielding a filter cake. The filter cake was first washed once with 10 ml of acetonitrile (1×10 ml), and then dried *in vacuo.* To the 20 ml sample vial, the dried filter cake, DMAP (3 mg), Cap1 (10 ml, 200 V), and Cap2 (1 ml, 20 V) were added and reacted on a shaker for 6 hours. After the reaction was quenched, the reaction solution was filtered, to provide the filter cake. The filter cake was first washed once with 10 ml of acetonitrile (1×10 ml), and then dried *in vacuo,* to provide compound CR01008Z (1.03 g, load amount 20-30 μmol/g).

Herein, Cap1 and Cap2 are capping reagents, Cap1 is a 20% (v/v) *N*-methylimidazole solution in mixed pyridine/acetonitrile, a volume ratio of pyridine to acetonitrile being 3:5; and Cap2 is a 20% (v/v) acetic anhydride solution in acetonitrile.

**Preparation Example 2 Preparation of (CR01008)×3 Ligand**

By the solid-phase synthetic method for phosphoramidite nucleic acids, the solid-phase support compound (CR01008Z) prepared according to Preparation Example 1 was used to start cycles to link the compounds (CR01008) not linked to the solid-phase support one by one.

The linking of each compound included a four-step reaction of deprotection, coupling, capping, oxidation or sulfurization. Synthesis conditions were given as follows.

Compounds CR01008 not linked to the solid-phase support were each formulated as a 0.1 M solution using acetonitrile.

The conditions for the deprotection reaction in each step were identical. The conditions for the deprotection reaction were as follows: the temperature was 25 °C, the reaction duration was 70 seconds, the deprotection reagent was a (3% v/v) dichloroacetic acid solution in dichloromethane, and the molar ratio of dichloroacetic acid to 4,4'-dimethoxytrityl protecting group on the solid-phase support was 5:1.

The conditions for the coupling reaction in each step were identical. The conditions for the coupling reaction were as follows: the temperature was 25 °C, the molar ratio of the compound linked to the solid-phase support to compounds not linked to the solid-phase support was 1:10, the molar ratio of the compound linked to the solid-phase support to the coupling reagent was 1:65, the reaction duration was 600 seconds, the coupling reagent was 0.5 M 5-ethylthio-1*H*-tetrazole in acetonitrile, and the sulfurization reagent was a solution of 0.2 M xanthane hydride in mixed acetonitrile/pyridine (the volume ratio of acetonitrile to pyridine was 1:1).

The conditions for the capping reaction in each step were identical. The conditions for the capping reaction were as follows: the temperature was 25 °C; the reaction duration was 2 minutes; the capping reagent solution was a mixed solution of Cap1 and Cap2 in the molar ratio of 1:1, where Cap1 was a solution of 20% (v/v) *N*-methylimidazole in mixed pyridine/acetonitrile, the volume ratio of pyridine to acetonitrile being 3:5, and Cap2 was a solution of 20% (v/v) acetic anhydride in acetonitrile; the molar ratio of *N*-methyl imidazole in Cap1 capping reagent, acetic anhydride in Cap2 capping reagent, and the compound linked to the solid-phase support was 1:1:1.

The conditions for the oxidation or sulfurization reaction in each step were identical. The conditions for the oxidation reaction were as follows: the temperature was 25 °C; the reaction duration was 3 seconds; the oxidation reagent was 0.05 M iodine water, the molar ratio of iodine to nucleic acid sequences linked to the solid-phase support in the coupling reaction was 30:1; and the oxidation reaction was carried out in the mixed water/pyridine solvent (the volume ratio of water to pyridine was 1:9). The conditions for the sulfurization reaction were as follows: the temperature was 25 °C; the reaction duration was 360 seconds; the sulfurization reagent was a solution of 0.2 M xanthane hydride in pyridine, the molar ratio of the sulfurization reagent to the compound linked to the solid-phase support in the coupling reaction was 4:1; and the sulfurization reaction was carried out in the mixed water/pyridine solvent (the volume ratio of water to pyridine was 1:9).

Trimer of CR01008 (denoted as (CR01008)×3) was obtained by the above method.

The structural formula of the trimer of CR01008 is as follows:

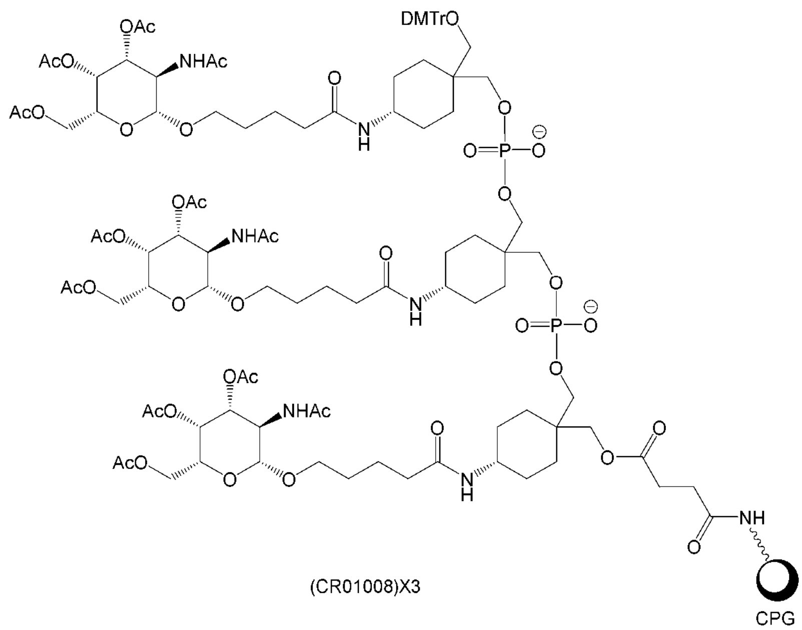

(CR01008)X3

## Preparation of Double-Stranded RNAi Agents

### Example 1 Preparation of Double-Stranded RNAi Agent with L96 as Ligand

Step 1: L96-PS

The compound L96-PS was purchased from Asymchem Laboratories (Tianjin) Co., Ltd., with a load amount of 120±12 μmol/g (the detection method was UV/HPLC). The structural formula of the compound L96-PS is as follows:

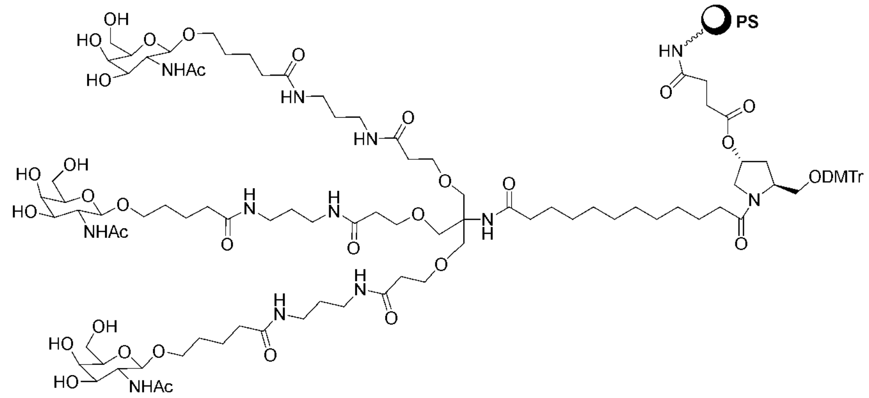

Herein, PS represents a polystyrene resin solid-phase support.

Step 2: Synthesis of Sense Strand

By the solid-phase synthetic method for phosphoramidite nucleic acids, the above compound L96-PS linked to the solid-phase support was used to start cycles to link the nucleoside monomers one by one in the 3'-5' direction according to the sequence of nucleotides. The linking of each nucleoside monomer included a four-step reaction of deprotection, coupling, capping, and oxidation or sulfurization.

By the solid-phase synthetic method for phosphoramidite nucleic acids, the solid-phase support compound was used to start cycles to link the nucleoside monomers one by one in the 3'-5' direction according to the sequence of nucleotides. The linking of each nucleoside monomer included a four-step reaction of deprotection, coupling, capping, and oxidation and sulfurization. Synthesis conditions were given as follows.

The nucleoside monomers were prepared as a solution of 0.1 M nucleoside monomers in acetonitrile.

The conditions for the deprotection reaction in each step were identical. The conditions for the deprotection reaction were as follows: the temperature was 25 °C, the reaction duration was 70 seconds, the deprotection reagent was a (3% v/v) dichloroacetic acid solution in dichloromethane, and the molar ratio of dichloroacetic acid to 4,4'-dimethoxytrityl protecting group on the solid support was 5:1.

The conditions for the coupling reaction in each step were identical. The conditions for the coupling reaction were as follows: the temperature was 25 °C, the molar ratio of nucleic acid sequences linked to the solid-phase support to the nucleoside monomers was 1:10, the molar ratio of nucleic acid sequences linked to the solid-phase support to the coupling reagent was 1:65, the reaction duration was 600 seconds, the coupling reagent was a solution of 0.5 M 5-ethylthio-1*H*-tetrazole in acetonitrile, and the sulfurization reagent was a solution of 0.2 M xanthane hydride in mixed acetonitrile/pyridine (the volume ratio of acetonitrile to pyridine was 1:1).

The conditions for the capping reaction in each step were identical. The conditions for the capping reaction were as follows: the temperature was 25 °C; the reaction duration was 2 minutes; the capping reagent solution was a mixed solution of Cap1 and Cap2 in the molar ratio of 1:1, where Cap1 was a solution of 20% v/v *N*-methylimidazole in mixed pyridine/acetonitrile, the volume ratio of pyridine to acetonitrile being 3:5, and Cap2 was a solution of 20% v/v acetic anhydride in acetonitrile; the molar ratio of *N*-methyl imidazole in Cap1 capping reagent, acetic anhydride in Cap2 capping reagent, and the nucleic acid sequences linked to the solid-phase support was 1:1:1.

The conditions for the oxidation reaction in each step were identical. The conditions for the oxidation reaction were as follows: the temperature was 25 °C; the reaction duration was 3 seconds; the oxidation reagent was 0.05 M iodine water, the molar ratio of iodine to the nucleic acid sequences linked to the solid-phase support in the coupling reaction was 30:1; and the oxidation reaction was carried out in the mixed water/pyridine solvent (the volume ratio of water to pyridine was 1:9). The conditions for the sulfurization reaction were as follows: the temperature was 25 °C; the reaction duration was 360 seconds; the sulfurization reagent was a solution of 0.2 M xanthane hydride in pyridine, the molar ratio of the sulfurization reagent to the nucleic acid sequences linked to the solid-phase support in the coupling reaction was 4:1; and the sulfurization reaction was carried out in the mixed water/pyridine solvent (the volume ratio of water to pyridine was 1:9).

Upon completion of linking of the last nucleoside monomer, the nucleic acid sequences linked on the solid-phase support were subjected to cleavage, deprotection, purification, desalination, and then lyophilization in sequence, to provide the sense strand.

The conditions for the cleavage and deprotection were as follows: adding the synthesized nucleotide sequences linked to the solid-phase support to 25 mass% aqueous ammonia, where the aqueous ammonia was in an amount of 0.5 ml/μmol, reacting at 55 °C for 16 hours, removing the solvent, and concentrating the resultant *in vacuo* to dryness; after the treatment with the aqueous ammonia, with respect to the amount of single-stranded nucleic acid, dissolving the product in

0.4 ml/μmol of *N*-methylpyrrolidone, followed by addition of 0.3 ml/μmol of trimethylamine and 0.6 ml/μmol of triethylamine trihydrofluoride, thereby removing the 2'-O-TBDMS protection on ribose.

The conditions for purification and desalination were as follows: the purification of nucleic acid was completed using a preparative ion chromatography column (Source 15Q) with a gradient elution of NaCl. Specifically, eluent 1 was 20 mM sodium phosphate (pH=8.1), and the solvent was a mixed water/acetonitrile solution (the volume ratio of water to acetonitrile was 9:1); eluent 2 was 1.5 M sodium chloride, 20 mM sodium phosphate (pH=8.1), and solvent was a mixed water/acetonitrile solution (the volume ratio of water to acetonitrile was 9:1); and elution gradient was eluent 1 : eluent 2 = (100:0) - (50:50). The product eluate was collected, combined, and desalted using a reversed phase chromatography purification column. The desalination conditions included using Sephadex column for desalination, Sephadex-G25 as filler, and deionized water for eluting.

Detection: The purity was determined using the ion exchange chromatography (IEX-HPLC); and the molecular weight was analyzed using the liquid chromatography-mass spectrometry (LC-MS). The measured value and theoretical value of the molecular weight were compared. If the measured value was approximately equal to the theoretical value, it is indicated that the ligand was conjugated to the 3'-end of the sense strand.

Step 3: Synthesis of Antisense Strand

The antisense strand was synthesized using the universal solid-phase support. The conditions for deprotection, coupling, capping, oxidation or sulfurization, conditions for cleavage and deprotection, and conditions for purification and desalination in the solid-phase synthesis method for the antisense strand were the same as those in the synthesis of the sense strand in step 2.

Detection: The purity was determined using the ion exchange chromatography (IEX-HPLC); and the molecular weight was analyzed using the liquid chromatography-mass spectrometry (LC-MS). The measured value and theoretical value of the molecular weight were compared. If the measured value was approximately equal to the theoretical value, it is indicated that the antisense strand was obtained.

Step 4: Synthesis of Double-Stranded RNAi Agents

The sense strand synthesized in step 2 and the antisense strand synthesized in step 3 were mixed in an equimolar ratio, dissolved in water for injection, heated to 95 °C, slowly cooled to room temperature, and held at the room temperature for 10 minutes, to allow the sense strand and the antisense strand to form the double-stranded structure via hydrogen bond, thereby to provide the RNAi agent having the sense strand and the antisense strand as listed in Table 2.

Information about the prepared double-stranded RNAi agents is listed in the following table.

Table 2 Sequences of Double-stranded RNAi Agents with L96 as Ligand

| No. | Modified Sense strand (5'-3') | Modified Antiense Strand (5'-3') |
|---|---|---|
| RZ000001 | UmsUmsCmUmCmCmGfAfAfCmGmUm GmUmCmAmCmGmUm L96 | AmsCfsGmUmGmAfCmAmCmGmUmUmC mGfGmAfGmAmAmsCmsUm |
| RZ011001 | CmsCmsUmUmUmAmUfCfUfUmGmGm GmCmCmUmCmUmUm L96 | AmsAfsGmAmGmGfCmCmCmAmAmGmA mUfAmAfAmGmGmsGmsCm |
| RZ011002 | GmsGmsUmGmUmGmAfCfCfAmCmCm AmCmUmCmCmAmUm L96 | AmsUfsGmGmAmGfUmGmGmUmGmGmU mCfAmCfAmCmCmsUmsCm |
| RZ011003 | GmsUmsAmGmAmGmAfUfCfAmAmAm GmGmCmGmGmCmUm L96 | AmsGfsCmCmGmCfCmUmUmUmGmAmU mCfUmCfUmAmCmsCmsCm |
| RZ011004 | CmsAmsAmAmGmGmCfGfGfCmUmCm CmUmUmCmCmGmAm L96 | UmsCfsGmGmAmAfGmGmAmGmCmCmG mCfCmUfUmUmGmsAmsUm |
| RZ011005 | AmsAmsGmGmCmGmGfCfUfCmCmUm UmCmCmGmAmCmUm L96 | AmsGfsUmCmGmGfAmAmGmGmAmGmC mCfGmCfCmUmUmsUmsGm |
| RZ011006 | GmsCmsGmGmCmUmCfCfUfUmCmCmG mAmCmUmUmCmUm L96 | AmsGfsAmAmGmUfCmGmGmAmAmGmG mAfGmCfCmGmCmsCmsUm |

(continued)

| No. | Modified Sense strand (5'-3') | Modified Antiense Strand (5'-3') |
|---|---|---|
| RZ011007 | GmsCmsUmCmCmUmUfCfCfGmAmCmUmUmCmUmCmCmAm L96 | UmsGfsGmAmGmAfAmGmUmCmGmGmAmAfGmGfAmGmCmsCmsGm |
| RZ011008 | CmsUmsCmCmUmUmCfCfGfAmCmUmUmCmUmCmCmAmAm L96 | UmsUfsGmGmAmGfAmAmGmUmCmGmGmAfAmGfGmAmGmsCmsCm |
| RZ011009 | CmsCmsUmUmCmCmGfAfCfUmUmCmUmCmCmAmAmGmAm L96 | UmsCfsUmUmGmGfAmGmAmAmGmUmCmGfGmAfAmGmGmsAmsGm |
| RZ011010 | CmsUmsUmCmCmGmAfCfUfUmCmUmCmCmAmAmGmAmGm L96 | CmsUfsCmUmUmGfGmAmGmAmAmGmUmCfGmGfAmAmGmsGmsAm |
| RZ011011 | GmsAmsCmUmUmCmUfCfCfAmAmGmAmGmGmGmCmCmAm L96 | UmsGfsGmCmCmCfUmCmUmUmGmGmAmGfAmAfGmUmCmsGmsGm |
| RZ011012 | GmsCmsCmAmGmGmCfAfCfUmGmGmAmGmUmAmCmGmUm L96 | AmsCfsGmUmAmCfUmCmCmAmGmUmGmCfCmUfGmGmCmsCmsCm |
| RZ011013 | GmsGmsAmGmUmAmCfGfUfGmUmGmUmCmCmUmUmCmUm L96 | AmsGfsAmAmGmGfAmCmAmCmAmCmGmUfAmCfUmCmCmsAmsGm |
| RZ011014 | GmsUmsGmUmGmUmCfCfUfUmCmUmGmGmCmUmUmCmUm L96 | AmsGfsAmAmGmCfCmAmGmAmAmGmGmAfCmAfCmAmCmsGmsUm |
| RZ011015 | CmsCmsUmUmCmUmGfGfCfUmUmCmUmAmCmCmCmGmUm L96 | AmsCfsGmGmGmUfAmGmAmAmGmCmCmAfGmAfAmGmGmsAmsCm |
| RZ011016 | CmsUmsUmCmUmGmGfCfUfUmCmUmAmCmCmCmGmUmAm L96 | UmsAfsCmGmGmGfUmAmGmAmAmGmCmCfAmGfAmAmGmsGmsAm |
| RZ011017 | GmsCmsUmUmCmUmAfCfCfCmGmUmAmCmCmCmUmGmUm L96 | AmsCfsAmGmGmGfUmAmCmGmGmGmUmAfGmAfAmGmCmsCmsAm |
| RZ011018 | CmsCmsCmGmUmAmCfCfCfUmGmUmGmCmAmGmAmCmAm L96 | UmsGfsUmCmUmGfCmAmCmAmGmGmGmUfAmCfGmGmGmsUmsAm |
| RZ011019 | CmsCmsUmGmUmGmCfAfGfAmCmAmCmGmUmAmCmCmUm L96 | AmsGfsGmUmAmCfGmUmGmUmCmUmGmCfAmCfAmGmGmsGmsUm |
| RZ011020 | GmsCmsAmGmAmCmAfCfGfUmAmCmCmUmGmCmAmGmAm L96 | UmsCfsUmGmCmAfGmGmUmAmCmGmUmGfUmCfUmGmCmsAmsCm |
| RZ011021 | CmsAmsGmAmCmAmCfGfUfAmCmCmUmGmCmAmGmAmUm L96 | AmsUfsCmUmGmCfAmGmGmUmAmCmGmUfGmUfCmUmGmsCmsAm |
| RZ011022 | AmsCmsAmCmGmUmAfCfCfUmGmCmAmGmAmUmCmUmAm L96 | UmsAfsGmAmUmCfUmGmCmAmGmGmUmAfCmGfUmGmUmsCmsUm |
| RZ011023 | CmsGmsGmGmGmUmCfCfUfGmGmAmGmCmAmCmCmCmUm L96 | AmsGfsGmGmUmGfCmUmCmCmAmGmGmAfCmCfCmCmGmsUmsAm |
| RZ011024 | CmsUmsGmGmAmGmCfAfCfCmCmUmGmAmAmGmAmCmUm L96 | AmsGfsUmCmUmUfCmAmGmGmGmUmGmCfUmCfCmAmGmsGmsAm |
| RZ011025 | GmsGmsAmGmCmAmCfCfCfUmGmAmAmGmAmCmUmCmAm L96 | UmsGfsAmGmUmCfUmUmCmAmGmGmGmUfGmCfUmCmCmsAmsGm |

(continued)

| No. | Modified Sense strand (5'-3') | Modified Antiense Strand (5'-3') |
|---|---|---|
| RZ011026 | GmsCmsAmCmCmCmUfGfAfAmGmAm CmUmCmAmAmGmAm L96 | UmsCfsUmUmGmAfGmUmCmUmUmCmA mGfGmGfUmGmCmsUmsCm |
| RZ011027 | CmsAmsGmGmAmAmGfGfCfAmGmAm GmUmGmCmAmGmAm L96 | UmsCfsUmGmCmAfCmUmCmUmGmCmC mUfUmCfCmUmGmsAmsCm |
| RZ011028 | AmsAmsGmGmCmAmGfAfGfUmGmCm AmGmAmGmCmAmAm L96 | UmsUfsGmCmUmCfUmGmCmAmCmUmC mUfGmCfCmUmUmsCmsCm |
| RZ011029 | GmsUmsGmCmAmGmAfGfCfAmAmUm CmCmAmCmUmGmUm L96 | AmsCfsAmGmUmGfGmAmUmUmGmCmU mCfUmGfCmAmCmsUmsCm |
| RZ011030 | GmsAmsCmCmAmCmAfCfGfAmCmUm UmCmGmAmGmAmAm L96 | UmsUfsCmUmCmGfAmAmGmUmCmGmU mGfUmGfGmUmCmsUmsUm |
| RZ011031 | GmsGmsUmCmUmCmCfCfUfAmCmUm AmCmAmAmUmGmUm L96 | AmsCfsAmUmUmGfUmAmGmUmAmGmG mGfAmGfAmCmCmsGmsGm |
| RZ011032 | CmsCmsUmAmCmUmAfCfAfAmUmGm UmGmAmGmUmGmAm L96 | UmsCfsAmCmUmCfAmCmAmUmUmGmU mAfGmUfAmGmGmsGmsAm |
| RZ011033 | CmsUmsAmCmUmAmCfAfAfUmGmUm GmAmGmUmGmAmUm L96 | AmsUfsCmAmCmUfCmAmCmAmUmUmG mUfAmGfUmAmGmsGmsGm |
| RZ011034 | CmsAmsAmUmGmUmGfAfGfUmGmAm UmGmAmGmAmUmCm L96 | GmsAfsUmCmUmCfAmUmCmAmCmUmC mAfCmAfUmUmGmsUmsAm |
| RZ011035 | GmsUmsGmAmUmGmAfGfAfUmCmUm CmUmUmUmCmCmAm L96 | UmsGfsGmAmAmAfGmAmGmAmUmCmU mCfAmUfCmAmCmsUmsCm |
| RZ011036 | GmsAmsUmGmAmGmAfUfCfUmCmUm UmUmCmCmAmCmUm L96 | AmsGfsUmGmGmAfAmAmGmAmGmAmU mCfUmCfAmUmCmsAmsCm |
| RZ011037 | GmsAmsGmAmUmCmUfCfUfUmUmCm CmAmCmUmGmCmUm L96 | AmsGfsCmAmGmUfGmGmAmAmAmGmA mGfAmUfCmUmCmsAmsUm |
| RZ011038 | AmsGmsAmUmCmUmCfUfUfUmCmCm AmCmUmGmCmUmAm L96 | UmsAfsGmCmAmGfUmGmGmAmAmAmG mAfGmAfUmCmUmsCmsAm |
| RZ011039 | GmsAmsUmCmUmCmUfUfUfCmCmAm CmUmGmCmUmAmUm L96 | AmsUfsAmGmCmAfGmUmGmGmAmAmA mGfAmGfAmUmCmsUmsCm |
| RZ011040 | GmsCmsUmAmUmGmAfCfGfGmUmUm AmCmAmCmUmCmUm L96 | AmsGfsAmGmUmGfUmAmAmCmCmGmU mCfAmUfAmGmCmsAmsGm |
| RZ011041 | CmsUmsGmCmCmAmAfUfCfGmCmAmC mCmUmGmCmCmAm L96 | UmsGfsGmCmAmGfGmUmGmCmGmAmU mUfGmGfCmAmGmsAmsGm |
| RZ011042 | AmsAmsUmCmGmCmAfCfCfUmGmCmC mAmAmGmUmGmAm L96 | UmsCfsAmCmUmUfGmGmCmAmGmGmU mGfCmGfAmUmUmsGmsGm |
| RZ011043 | AmsUmsCmGmCmAmCfCfUfGmCmCmA mAmGmUmGmAmAm L96 | UmsUfsCmAmCmUfUmGmGmCmAmGmG mUfGmCfGmAmUmsUmsGm |
| RZ011044 | AmsGmsUmGmAmAmUfGfGfCmCmGm AmUmGmGmAmGmUm L96 | AmsCfsUmCmCmAfUmCmGmGmCmCmA mUfUmCfAmCmUmsUmsGm |

(continued)

| No. | Modified Sense strand (5'-3') | Modified Antiense Strand (5'-3') |
|---|---|---|
| RZ011045 | GmsAmsAmUmGmGmCfCfGfAmUmGm GmAmGmUmGmGmGm L96 | CmsCfsCmAmCmUfCmCmAmUmCmGmG mCfCmAfUmUmCmsAmsCm |
| RZ011046 | CmsCmsGmAmUmGmGfAfGfUmGmGm GmCmAmGmAmCmAm L96 | UmsGfsUmCmUmGfCmCmCmAmCmUmC mCfAmUfCmGmGmsCmsCm |
| RZ011047 | AmsUmsGmGmAmGmUfGfGfGmCmAm GmAmCmAmGmCmGm L96 | CmsGfsCmUmGmUfCmUmGmCmCmCmA mCfUmCfCmAmUmsCmsGm |
| RZ011048 | GmsGmsAmGmUmGmGfGfCfAmGmAm CmAmGmCmGmAmUm L96 | AmsUfsCmGmCmUfGmUmCmUmGmCmC mCfAmCfUmCmCmsAmsUm |
| RZ011049 | UmsGmsGmGmCmAmGfAfCfAmGmCm GmAmUmCmUmGmUm L96 | AmsCfsAmGmAmUfCmGmCmUmGmUmC mUfGmCfCmCmAmsCmsUm |
| RZ011050 | GmsGmsCmAmGmAmCfAfGfCmGmAm UmCmUmGmUmGmAm L96 | UmsCfsAmCmAmGfAmUmCmGmCmUmG mUfCmUfGmCmCmsCmsAm |
| RZ011051 | GmsCmsAmGmAmCmAfGfCfGmAmUm CmUmGmUmGmAmCm L96 | GmsUfsCmAmCmAfGmAmUmCmGmCmU mGfUmCfUmGmCmsCmsCm |
| RZ011052 | CmsAmsGmAmCmAmGfCfGfAmUmCm UmGmUmGmAmCmAm L96 | UmsGfsUmCmAmCfAmGmAmUmCmGmC mUfGmUfCmUmGmsCmsCm |
| RZ011053 | GmsGmsCmAmCmAmAfGfGfAmAmGm GmUmGmGmGmCmAm L96 | UmsGfsCmCmCmAfCmCmUmUmCmCmU mUfGmUfGmCmCmsAmsAm |
| RZ011054 | GmsCmsAmCmAmAmGfGfAfAmGmGm UmGmGmGmCmAmGm L96 | CmsUfsGmCmCmCfAmCmCmUmUmCmCm UfUmGfUmGmCmsCmsAm |
| RZ011055 | CmsAmsAmGmGmAmAfGfGfUmGmGm GmCmAmGmCmCmAm L96 | UmsGfsGmCmUmGfCmCmCmAmCmCmU mUfCmCfUmUmGmsUmsGm |
| RZ011056 | GmsCmsAmGmCmCmAfGfUfAmCmCm GmCmCmUmUmGmAm L96 | UmsCfsAmAmGmGfCmGmGmUmAmCmU mGfGmCfUmGmCmsCmsCm |
| RZ011057 | CmsUmsUmGmAmAmGfAfCfAmGmCm GmUmCmAmCmCmUm L96 | AmsGfsGmUmGmAfCmGmCmUmGmUmC mUfUmCfAmAmGmsGmsCm |
| RZ011058 | GmsAmsCmAmGmCmGfUfCfAmCmCm UmAmCmCmAmCmUm L96 | AmsGfsUmGmGmUfAmGmGmUmGmAmC mGfCmUfGmUmCmsUmsUm |
| RZ011059 | CmsCmsGmGmGmGmGfCfUfUmAmCm CmCmUmGmCmGmUm L96 | AmsCfsGmCmAmGfGmGmUmAmAmGmC mCfCmCfCmGmGmsCmsUm |
| RZ011060 | GmsCmsGmGmCmGmAfAfCfGmUmGm UmCmAmGmGmAmAm L96 | UmsUfsCmCmUmGfAmCmAmCmGmUmU mCfGmCfCmGmCmsUmsGm |
| RZ011061 | GmsCmsGmAmAmCmGfUfGfUmCmAm GmGmAmAmGmGmUm L96 | AmsCfsCmUmUmCfCmUmGmAmCmAmC mGfUmUfCmGmCmsCmsGm |
| RZ011062 | GmsUmsGmUmCmAmGfGfAfAmGmGm UmGmGmCmUmCmUm L96 | AmsGfsAmGmCmCfAmCmCmUmUmCmC mUfGmAfCmAmCmsGmsUm |
| RZ011063 | GmsGmsAmAmGmGmUfGfGfCmUmCm UmUmGmGmAmGmCm L96 | GmsCfsUmCmCmAfAmGmAmGmCmCmA mCfCmUfUmCmCmsUmsGm |

(continued)

| No. | Modified Sense strand (5'-3') | Modified Antiense Strand (5'-3') |
|---|---|---|
| RZ011064 | AmsGmsGmUmGmGmCfUfCfUmUmGmGmAmGmCmGmGmGm L96 | CmsCfsCmGmCmUfCmCmAmAmGmAmGmCfCmAfCmCmUmsUmsCm |
| RZ011065 | GmsAmsGmCmGmGmGfAfCfGmGmAmGmCmCmUmUmCmCm L96 | GmsGfsAmAmGmGfCmUmCmCmGmUmCmCfCmGfCmUmCmsCmsAm |
| RZ011066 | GmsAmsCmGmGmAmGfCfCfUmUmCmCmUmGmCmCmAmAm L96 | UmsUfsGmGmCmAfGmGmAmAmGmGmCmUfCmCfGmUmCmsCmsCm |
| RZ011067 | GmsAmsGmCmCmUmUfCfCfUmGmCmCmAmAmGmAmCmUm L96 | AmsGfsUmCmUmUfGmGmCmAmGmGmAmAfGmGfCmUmCmsCmsGm |
| RZ011068 | CmsCmsUmUmCmCmUfGfCfCmAmAmGmAmCmUmCmCmUm L96 | AmsGfsGmAmGmUfCmUmUmGmGmCmAmGfGmAfAmGmGmsCmsUm |
| RZ011069 | CmsUmsUmCmCmUmGfCfCfAmAmGmAmCmUmCmCmUmUm L96 | AmsAfsGmGmAmGfUmCmUmUmGmGmCmAfGmGfAmAmGmsGmsCm |
| RZ011070 | CmsCmsUmGmCmCmAfAfGfAmCmUmCmCmUmUmCmAmUm L96 | AmsUfsGmAmAmGfGmAmGmUmCmUmUmGfGmCfAmGmGmsAmsAm |
| RZ011071 | GmsCmsCmAmAmGmAfCfUfCmCmUmUmCmAmUmGmUmAm L96 | UmsAfsCmAmUmGfAmAmGmGmAmGmUmCfUmUfGmGmCmsAmsGm |
| RZ011072 | GmsAmsCmUmCmCmUfUfCfAmUmGmUmAmCmGmAmCmAm L96 | UmsGfsUmCmGmUfAmCmAmUmGmAmAmGfGmAfGmUmCmsUmsUm |
| RZ011073 | AmsCmsUmCmCmUmUfCfAfUmGmUmAmCmGmAmCmAmCm L96 | GmsUfsGmUmCmGfUmAmCmAmUmGmAmAfGmGfAmGmUmsCmsUm |
| RZ011074 | CmsUmsCmCmUmUmCfAfUfGmUmAmCmGmAmCmAmCmCm L96 | GmsGfsUmGmUmCfGmUmAmCmAmUmGmAfAmGfGmAmGmsUmsCm |
| RZ011075 | GmsGmsCmCmGmAmAfGfCfUmUmUmCmCmUmGmUmCmUm L96 | AmsGfsAmCmAmGfGmAmAmAmGmCmUmUfCmGfGmCmCmsAmsCm |
| RZ011076 | GmsCmsCmGmAmAmGfCfUfUmUmCmCmUmGmUmCmUmUm L96 | AmsAfsGmAmCmAfGmGmAmAmAmGmCmUfUmCfGmGmCmsCmsAm |
| RZ011077 | GmsCmsUmUmUmCmCfUfGfUmCmUmUmCmCmCmUmGmAm L96 | UmsCfsAmGmGmGfAmAmGmAmCmAmGmGfAmAfAmGmCmsUmsUm |
| RZ011078 | CmsCmsUmGmAmCmAfGfAfGmAmCmCmAmUmAmGmAmAm L96 | UmsUfsCmUmAmUfGmGmUmCmUmCmUmGfUmCfAmGmGmsGmsAm |
| RZ011079 | GmsAmsCmAmGmAmGfAfCfCmAmUmAmGmAmAmGmGmAm L96 | UmsCfsCmUmUmCfUmAmUmGmGmUmCmUfCmUfGmUmCmsAmsGm |
| RZ011080 | CmsAmsGmAmGmAmCfCfAfUmAmGmAmAmGmGmAmGmUm L96 | AmsCfsUmCmCmUfUmCmUmAmUmGmGmUfCmUfCmUmGmsUmsCm |
| RZ011081 | GmsAmsCmCmAmUmAfGfAfAmGmGmAmGmUmCmGmAmUm L96 | AmsUfsCmGmAmCfUmCmCmUmUmCmUmAfUmGfGmUmCmsUmsCm |
| RZ011082 | CmsAmsUmAmGmAmAfGfGfAmGmUmCmGmAmUmGmCmUm L96 | AmsGfsCmAmUmCfGmAmCmUmCmCmUmUfCmUfAmUmGmsGmsUm |

(continued)

| No. | Modified Sense strand (5'-3') | Modified Antiense Strand (5'-3') |
|---|---|---|
| RZ011083 | UmsAmsGmAmAmGmGfAfGfUmCmGmAmUmGmCmUmGmAm L96 | UmsCfsAmGmCmAfUmCmGmAmCmUmCmCfUmUfCmUmAmsUmsGm |
| RZ011084 | AmsGmsGmAmGmUmCfGfAfUmGmCmUmGmAmGmGmAmUm L96 | AmsUfsCmCmUmCfAmGmCmAmUmCmGmAfCmUfCmCmUmsUmsCm |
| RZ011085 | AmsUmsGmGmGmCmAfCfGfGmCmCmCmAmGmGmGmGmAm L96 | UmsCfsCmCmCmUfGmGmGmCmCmGmUmGfCmCfCmAmUmsCmsCm |
| RZ011086 | GmsGmsGmAmAmCmAfAfCfAmGmAmAmGmCmGmGmAmAm L96 | UmsUfsCmCmGmCfUmUmCmUmGmUmUmGfUmUfCmCmCmsCmsUm |
| RZ011087 | GmsAmsAmCmAmAmCfAfGfAmAmGmCmGmGmAmAmGmAm L96 | UmsCfsUmUmCmCfGmCmUmUmCmUmGmUfUmGfUmUmCmsCmsCm |
| RZ011088 | CmsAmsAmCmAmGmAfAfGfCmGmGmAmAmGmAmUmCmGm L96 | CmsGfsAmUmCmUfUmCmCmGmCmUmUmCfUmGfUmUmGmsUmsUm |
| RZ011089 | CmsUmsGmGmAmCmCfCfUfUmCmAmGmGmCmUmCmCmAm L96 | UmsGfsGmAmGmCfCmUmGmAmAmGmGmGfUmCfCmAmGmsGmsAm |
| RZ011090 | CmsCmsUmUmCmAmGfGfCfUmCmCmAmUmGmAmAmCmAm L96 | UmsGfsUmUmCmAfUmGmGmAmGmCmCmUfGmAfAmGmGmsGmsUm |
| RZ011091 | CmsAmsGmGmCmUmCfCfAfUmGmAmAmCmAmUmCmUmAm L96 | UmsAfsGmAmUmGfUmUmCmAmUmGmGmAfGmCfCmUmGmsAmsAm |
| RZ011092 | GmsCmsUmCmCmAmUfGfAfAmCmAmUmCmUmAmCmCmUm L96 | AmsGfsGmUmAmGfAmUmGmUmUmCmAmUfGmGfAmGmCmsCmsUm |
| RZ011093 | CmsCmsAmUmGmAmAfCfAfUmCmUmAmCmCmUmGmGmUm L96 | AmsCfsCmAmGmGfUmAmGmAmUmGmUmUfCmAfUmGmGmsAmsGm |
| RZ011094 | AmsAmsCmAmUmCmUfAfCfCmUmGmGmUmGmCmUmAmGm L96 | CmsUfsAmGmCmAfCmCmAmGmGmUmAmGfAmUfGmUmUmsCmsAm |
| RZ011095 | UmsAmsCmCmUmGmGfUfGfCmUmAmGmAmUmGmGmAmUm L96 | AmsUfsCmCmAmUfCmUmAmGmCmAmCmCfAmGfGmUmAmsGmsAm |
| RZ011096 | AmsCmsCmUmGmGmUfGfCfUmAmGmAmUmGmGmAmUmCm L96 | GmsAfsUmCmCmAfUmCmUmAmGmCmAmCfCmAfGmGmUmsAmsGm |
| RZ011097 | CmsCmsUmGmGmUmGfCfUfAmGmAmUmGmGmAmUmCmAm L96 | UmsGfsAmUmCmCfAmUmCmUmAmGmCmAfCmCfAmGmGmsUmsAm |
| RZ011098 | CmsUmsAmGmAmUmGfGfAfUmCmAmGmAmCmAmGmCmAm L96 | UmsGfsCmUmGmUfCmUmGmAmUmCmCmAfUmCfUmAmGmsCmsAm |
| RZ011099 | UmsGmsGmAmUmCmAfGfAfCmAmGmCmAmUmUmGmGmGm L96 | CmsCfsCmAmAmUfGmCmUmGmUmCmUmGfAmUfCmCmAmsUmsCm |
| RZ011100 | GmsCmsCmAmGmCmAfAfCfUmUmCmAmCmAmGmGmAmGm L96 | CmsUfsCmCmUmGfUmGmAmAmGmUmUmGfCmUfGmGmCmsCmsCm |
| RZ011101 | CmsAmsAmCmUmUmCfAfCfAmGmGmAmGmCmCmAmAmAm L96 | UmsUfsUmGmGmCfUmCmCmUmGmUmGmAfAmGfUmUmGmsCmsUm |

(continued)

| No. | Modified Sense strand (5'-3') | Modified Antiense Strand (5'-3') |
|---|---|---|
| RZ011102 | AmsAmsAmGmUmGmUfCfUfAmGmUmCmAmAmCmUmUmAm L96 | UmsAfsAmGmUmUfGmAmCmUmAmGmAmCfAmCfUmUmUmsUmsUm |
| RZ011103 | AmsAmsGmUmGmUmCfUfAfGmUmCmAmAmCmUmUmAmAm L96 | UmsUfsAmAmGmUfUmGmAmCmUmAmGmAfCmAfCmUmUmsUmsUm |
| RZ011104 | AmsGmsUmGmUmCmUfAfGfUmCmAmAmCmUmUmAmAmUm L96 | AmsUfsUmAmAmGfUmUmGmAmCmUmAmGfAmCfAmCmUmsUmsUm |
| RZ011105 | GmsUmsCmUmAmGmUfCfAfAmCmUmUmAmAmUmUmGmAm L96 | UmsCfsAmAmUmUfAmAmGmUmUmGmAmCfUmAfGmAmCmsAmsCm |
| RZ011106 | CmsUmsAmGmUmCmAfAfCfUmUmAmAmUmUmGmAmGmAm L96 | UmsCfsUmCmAmAfUmUmAmAmGmUmUmGfAmCfUmAmGmsAmsCm |
| RZ011107 | UmsUmsGmAmGmAmAfGfGfUmGmGmCmAmAmGmUmUmAm L96 | UmsAfsAmCmUmUfGmCmCmAmCmCmUmUfCmUfCmAmAmsUmsUm |
| RZ011108 | GmsAmsAmGmGmUmGfGfCfAmAmGmUmUmAmUmGmGmUm L96 | AmsCfsCmAmUmAfAmCmUmUmGmCmCmAfCmCfUmUmCmsUmsCm |
| RZ011109 | GmsUmsGmGmCmAmAfGfUfUmAmUmGmGmUmGmUmGmAm L96 | UmsCfsAmCmAmCfCmAmUmAmAmCmUmUfGmCfCmAmCmsCmsUm |
| RZ011110 | AmsGmsUmUmAmUmGfGfUfGmUmGmAmAmGmCmCmAmAm L96 | UmsUfsGmGmCmUfUmCmAmCmAmCmCmAfUmAfAmCmUmsUmsGm |
| RZ011111 | GmsGmsUmGmUmGmAfAfGfCmCmAmAmGmAmUmAmUmGm L96 | CmsAfsUmAmUmCfUmUmGmGmCmUmUmCfAmCfAmCmCmsAmsUm |
| RZ011112 | GmsAmsAmGmCmCmAfAfGfAmUmAmUmGmGmUmCmUmAm L96 | UmsAfsGmAmCmCfAmUmAmUmCmUmUmGfGmCfUmUmCmsAmsCm |
| RZ011113 | AmsAmsGmAmUmAmUfGfGfUmCmUmAmGmUmGmAmCmAm L96 | UmsGfsUmCmAmCfUmAmGmAmCmCmAmUfAmUfCmUmUmsGmsGm |
| RZ011114 | GmsAmsUmAmUmGmGfUfCfUmAmGmUmGmAmCmAmUmAm L96 | UmsAfsUmGmUmCfAmCmUmAmGmAmCmCfAmUfAmUmCmsUmsUm |
| RZ011115 | GmsGmsUmCmUmAmGfUfGfAmCmAmUmAmUmGmCmCmAm L96 | UmsGfsGmCmAmUfAmUmGmUmCmAmCmUfAmGfAmCmCmsAmsUm |
| RZ011116 | CmsUmsAmGmUmGmAfCfAfUmAmUmGmCmCmAmCmAmUm L96 | AmsUfsGmUmGmGfCmAmUmAmUmGmUmCfAmCfUmAmGmsAmsCm |
| RZ011117 | CmsCmsAmAmAmAmUfUfUfGmGmGmUmCmAmAmAmGmUm L96 | AmsCfsUmUmUmGfAmCmCmCmAmAmAmUfUmUfUmGmGmsGmsGm |
| RZ011118 | GmsAmsCmAmGmCmAfGfUfAmAmUmGmCmAmGmAmCmUm L96 | AmsGfsUmCmUmGfCmAmUmUmAmCmUmGfCmUfGmUmCmsUmsGm |
| RZ011119 | GmsCmsAmGmUmAmAfUfGfCmAmGmAmCmUmGmGmGmUm L96 | AmsCfsCmCmAmGfUmCmUmGmCmAmUmUfAmCfUmGmCmsUmsGm |
| RZ011120 | AmsGmsUmAmAmUmGfCfAfGmAmCmUmGmGmGmUmCmAm L96 | UmsGfsAmCmCmCfAmGmUmCmUmGmCmAfUmUfAmCmUmsGmsCm |

(continued)

| No. | Modified Sense strand (5'-3') | Modified Antiense Strand (5'-3') |
|---|---|---|
| RZ011121 | CmsAmsGmAmCmUmGfGfGfUmCmAm CmGmAmAmGmCmAm L96 | UmsGfsCmUmUmCfGmUmGmAmCmCmC mAfGmUfCmUmGmsCmsAm |
| RZ011122 | GmsGmsGmUmCmAmCfGfAfAmGmCm AmGmCmUmCmAmAm L96 | UmsUfsGmAmGmCfUmGmCmUmUmCmG mUfGmAfCmCmCmsAmsGm |
| RZ011123 | GmsCmsAmGmCmUmCfAfAfUmGmAm AmAmUmCmAmAmUm L96 | AmsUfsUmGmAmUfUmUmCmAmUmUmG mAfGmCfUmGmCmsUmsUm |
| RZ011124 | GmsCmsUmCmAmAmUfGfAfAmAmUm CmAmAmUmUmAmUm L96 | AmsUfsAmAmUmUfGmAmUmUmUmCmA mUfUmGfAmGmCmsUmsGm |
| RZ011125 | CmsAmsAmUmGmAmAfAfUfCmAmAm UmUmAmUmGmAmAm L96 | UmsUfsCmAmUmAfAmUmUmGmAmUmU mUfCmAfUmUmGmsAmsGm |
| RZ011126 | UmsCmsAmAmUmUmAfUfGfAmAmGm AmCmCmAmCmAmAm L96 | UmsUfsGmUmGmGfUmCmUmUmCmAmU mAfAmUfUmGmAmsUmsUm |
| RZ011127 | AmsUmsGmAmAmGmAfCfCfAmCmAm AmGmUmUmGmAmAm L96 | UmsUfsCmAmAmCfUmUmGmUmGmGmU mCfUmUfCmAmUmsAmsAm |
| RZ011128 | GmsAmsAmGmAmCmCfAfCfAmAmGm UmUmGmAmAmGmUm L96 | AmsCfsUmUmCmAfAmCmUmUmGmUmG mGfUmCfUmUmCmsAmsUm |
| RZ011129 | CmsAmsCmAmAmGmUfUfGfAmAmGm UmCmAmGmGmGmAm L96 | UmsCfsCmCmUmGfAmCmUmUmCmAmA mCfUmUfGmUmGmsGmsUm |
| RZ011130 | AmsGmsUmUmGmAmAfGfUfCmAmGm GmGmAmCmUmAmAm L96 | UmsUfsAmGmUmCfCmCmUmGmAmCmU mUfCmAfAmCmUmsUmsGm |
| RZ011131 | CmsAmsGmGmGmAmCfUfAfAmCmAm CmCmAmAmGmAmAm L96 | UmsUfsCmUmUmGfGmUmGmUmUmAmG mUfCmCfCmUmGmsAmsCm |
| RZ011132 | CmsUmsAmAmCmAmCfCfAfAmGmAm AmGmGmCmCmCmUm L96 | AmsGfsGmGmCmCfUmUmCmUmUmGmG mUfGmUfUmAmGmsUmsCm |
| RZ011133 | CmsAmsGmGmCmAmGfUfGfUmAmCm AmGmCmAmUmGmAm L96 | UmsCfsAmUmGmCfUmGmUmAmCmAmC mUfGmCfCmUmGmsGmsAm |
| RZ011134 | GmsCmsAmGmUmGmUfAfCfAmGmCm AmUmGmAmUmGmAm L96 | UmsCfsAmUmCmAfUmGmCmUmGmUmA mCfAmCfUmGmCmsCmsUm |
| RZ011135 | GmsUmsGmUmAmCmAfGfCfAmUmGm AmUmGmAmGmCmUm L96 | AmsGfsCmUmCmAfUmCmAmUmGmCmU mGfUmAfCmAmCmsUmsGm |
| RZ011136 | CmsCmsUmGmAmAmGfGfCfUmGmGm AmAmCmCmGmCmAm L96 | UmsGfsCmGmGmUfUmCmCmAmGmCmC mUfUmCfAmGmGmsAmsGm |
| RZ011137 | GmsCmsAmCmCmCmGfCfCfAmUmGmU mCmAmUmCmAmUm L96 | AmsUfsGmAmUmGfAmCmAmUmGmGmC mGfGmGfUmGmCmsGmsGm |
| RZ011138 | CmsCmsCmGmCmCmAfUfGfUmCmAmU mCmAmUmCmCmUm L96 | AmsGfsGmAmUmGfAmUmGmAmCmAmU mGfGmCfGmGmGmsUmsGm |
| RZ011139 | CmsGmsCmCmAmUmGfUfCfAmUmCm AmUmCmCmUmCmAm L96 | UmsGfsAmGmGmAfUmGmAmUmGmAmC mAfUmGfGmCmGmsGmsGm |

(continued)

| No. | Modified Sense strand (5'-3') | Modified Antiense Strand (5'-3') |
|---|---|---|
| RZ011140 | GmsCmsCmAmUmGmUfCfAfUmCmAm UmCmCmUmCmAmUm L96 | AmsUfsGmAmGmGfAmUmGmAmUmGmA mCfAmUfGmGmCmsGmsGm |
| RZ011141 | CmsAmsUmGmUmCmAfUfCfAmUmCm CmUmCmAmUmGmAm L96 | UmsCfsAmUmGmAfGmGmAmUmGmAmU mGfAmCfAmUmGmsGmsCm |
| RZ011142 | CmsCmsUmCmAmUmGfAfCfUmGmAm UmGmGmAmUmUmGm L96 | CmsAfsAmUmCmCfAmUmCmAmGmUmC mAfUmGfAmGmGmsAmsUm |
| RZ011143 | UmsGmsAmCmUmGmAfUfGfGmAmUm UmGmCmAmCmAmAm L96 | UmsUfsGmUmGmCfAmAmUmCmCmAmU mCfAmGfUmCmAmsUmsGm |
| RZ011144 | CmsUmsGmAmUmGmGfAfUfUmGmCm AmCmAmAmCmAmUm L96 | AmsUfsGmUmUmGfUmGmCmAmAmUmC mCfAmUfCmAmGmsUmsCm |
| RZ011145 | CmsCmsCmAmAmUmUfAfCfUmGmUm CmAmUmUmGmAmUm L96 | AmsUfsCmAmAmUfGmAmCmAmGmUmA mAfUmUfGmGmGmsUmsCm |
| RZ011146 | CmsAmsAmUmUmAmCfUfGfUmCmAm UmUmGmAmUmGmAm L96 | UmsCfsAmUmCmAfAmUmGmAmCmAmG mUfAmAfUmUmGmsGmsGm |
| RZ011147 | CmsCmsGmGmGmAmCfUfUfGmCmUm AmUmAmCmAmUmUm L96 | AmsAfsUmGmUmAfUmAmGmCmAmAmG mUfCmCfCmGmGmsAmsUm |
| RZ011148 | CmsAmsAmGmGmGmAfGfGfAmUmUm AmUmCmUmGmGmAm L96 | UmsCfsCmAmGmAfUmAmAmUmCmCmU mCfCmCfUmUmGmsGmsGm |
| RZ011149 | GmsGmsGmAmGmGmAfUfUfAmUmCm UmGmGmAmUmGmUm L96 | AmsCfsAmUmCmCfAmGmAmUmAmAmU mCfCmUfCmCmCmsUmsUm |
| RZ011150 | AmsGmsGmAmUmUmAfUfCfUmGmGm AmUmGmUmCmUmAm L96 | UmsAfsGmAmCmAfUmCmCmAmGmAmU mAfAmUfCmCmUmsCmsCm |
| RZ011151 | GmsGmsAmUmUmAmUfCfUfGmGmAm UmGmUmCmUmAmUm L96 | AmsUfsAmGmAmCfAmUmCmCmAmGmA mUfAmAfUmCmCmsUmsCm |
| RZ011152 | UmsCmsUmGmGmAmUfGfUfCmUmAm UmGmUmGmUmUmUm L96 | AmsAfsAmCmAmCfAmUmAmGmAmCmA mUfCmCfAmGmAmsUmsAm |
| RZ011153 | CmsUmsUmUmGmGmUfGfAfAmCmCm AmAmGmUmGmAmAm L96 | UmsUfsCmAmCmUfUmGmGmUmUmCmA mCfCmAfAmAmGmsGmsCm |
| RZ011154 | UmsGmsAmAmCmCmAfAfGfUmGmAm AmCmAmUmCmAmAm L96 | UmsUfsGmAmUmGfUmUmCmAmCmUmU mGfGmUfUmCmAmsCmsCm |
| RZ011155 | CmsAmsAmGmUmGmAfAfCfAmUmCm AmAmUmGmCmUmUm L96 | AmsAfsGmCmAmUfUmGmAmUmGmUmU mCfAmCfUmUmGmsGmsUm |
| RZ011156 | AmsAmsGmUmGmAmAfCfAfUmCmAm AmUmGmCmUmUmUm L96 | AmsAfsAmGmCmAfUmUmGmAmUmGmU mUfCmAfCmUmUmsGmsGm |
| RZ011157 | GmsAmsAmCmAmUmCfAfAfUmGmCm UmUmUmGmGmCmUm L96 | AmsGfsCmCmAmAfAmGmCmAmUmUmG mAfUmGfUmUmCmsAmsCm |
| RZ011158 | CmsUmsUmCmCmAmAfGfAfAmAmGm AmCmAmAmUmGmAm L96 | UmsCfsAmUmUmGfUmCmUmUmUmCmU mUfGmGfAmAmGmsCmsCm |

(continued)

| No. | Modified Sense strand (5'-3') | Modified Antiense Strand (5'-3') |
|---|---|---|
| RZ011159 | CmsCmsAmAmGmAmAfAfGfAmCmAm AmUmGmAmGmCmAm L96 | UmsGfsCmUmCmAfUmUmGmUmCmUmU mUfCmUfUmGmGmsAmsAm |
| RZ011160 | CmsAmsAmGmAmAmAfGfAfCmAmAm UmGmAmGmCmAmAm L96 | UmsUfsGmCmUmCfAmUmUmGmUmCmU mUfUmCfUmUmGmsGmsAm |
| RZ011161 | AmsGmsAmAmAmGmAfCfAfAmUmGm AmGmCmAmAmCmAm L96 | UmsGfsUmUmGmCfUmCmAmUmUmGmU mCfUmUfUmCmUmsUmsGm |
| RZ011162 | GmsAmsAmAmGmAmCfAfAfUmGmAm GmCmAmAmCmAmUm L96 | AmsUfsGmUmUmGfCmUmCmAmUmUmG mUfCmUfUmUmCmsUmsUm |
| RZ011163 | CmsAmsAmCmAmUmGfUfGfUmUmCm AmAmAmGmUmCmAm L96 | UmsGfsAmCmUmUfUmGmAmAmCmAmC mAfUmGfUmUmGmsCmsUm |
| RZ011164 | AmsAmsCmAmUmGmUfGfUfUmCmAm AmAmGmUmCmAmAm L96 | UmsUfsGmAmCmUfUmUmGmAmAmCmA mCfAmUfGmUmUmsGmsCm |
| RZ011165 | GmsUmsGmUmUmCmAfAfAfGmUmCm AmAmGmGmAmUmAm L96 | UmsAfsUmCmCmUfUmGmAmCmUmUmU mGfAmAfCmAmCmsAmsUm |
| RZ011166 | UmsGmsUmUmCmAmAfAfGfUmCmAm AmGmGmAmUmAmUm L96 | AmsUfsAmUmCmCfUmUmGmAmCmUmU mUfGmAfAmCmAmsCmsAm |
| RZ011167 | CmsAmsAmAmGmUmCfAfAfGmGmAm UmAmUmGmGmAmAm L96 | UmsUfsCmCmAmUfAmUmCmCmUmUmG mAfCmUfUmUmGmsAmsAm |
| RZ011168 | GmsGmsAmAmAmAmCfCfUfGmGmAm AmGmAmUmGmUmUm L96 | AmsAfsCmAmUmCfUmUmCmCmAmGmG mUfUmUfUmCmCmsAmsUm |
| RZ011169 | GmsAmsUmCmGmAmUfGfAfAmAmGm CmCmAmGmUmCmUm L96 | AmsGfsAmCmUmGfGmCmUmUmUmCmA mUfCmGfAmUmCmsAmsUm |
| RZ011170 | GmsCmsCmAmGmUmCfUfCfUmGmAm GmUmCmUmCmUmGm L96 | CmsAfsGmAmGmAfCmUmCmAmGmAmG mAfCmUfGmGmCmsUmsUm |
| RZ011171 | CmsUmsCmUmGmAmGfUfCfUmCmUm GmUmGmGmCmAmUm L96 | AmsUfsGmCmCmAfCmAmGmAmGmAmC mUfCmAfGmAmGmsAmsCm |
| RZ011172 | GmsAmsGmUmCmUmCfUfGfUmGmGm CmAmUmGmGmUmUm L96 | AmsAfsCmCmAmUfGmCmCmAmCmAmG mAfGmAfCmUmCmsAmsGm |
| RZ011173 | GmsUmsGmGmCmAmUfGfGfUmUmUm GmGmGmAmAmCmAm L96 | UmsGfsUmUmCmCfCmAmAmAmCmCmA mUfGmCfCmAmCmsAmsGm |
| RZ011174 | GmsGmsCmAmUmGmGfUfUfUmGmGm GmAmAmCmAmCmAm L96 | UmsGfsUmGmUmUfCmCmCmAmAmAmC mCfAmUfGmCmCmsAmsCm |
| RZ011175 | GmsUmsAmCmCmGmAfUfUfAmCmCm AmCmAmAmGmCmAm L96 | UmsGfsCmUmUmGfUmGmGmUmAmAmU mCfGmGfUmAmCmsCmsCm |
| RZ011176 | GmsAmsUmUmAmCmCfAfCfAmAmGm CmAmAmCmCmAmUm L96 | AmsUfsGmGmUmUfGmCmUmUmGmUmG mGfUmAfAmUmCmsGmsGm |
| RZ011177 | GmsGmsCmAmGmGmCfCfAfAmGmAm UmCmUmCmAmGmUm L96 | AmsCfsUmGmAmGfAmUmCmUmUmGmG mCfCmUfGmCmCmsAmsUm |

(continued)

| No. | Modified Sense strand (5'-3') | Modified Antiense Strand (5'-3') |
|---|---|---|
| RZ011178 | CmsAmsGmGmCmCmAfAfGfAmUmCmUmCmAmGmUmCmAm L96 | UmsGfsAmCmUmGfAmGmAmUmCmUmUmGfGmCfCmUmGmsCmsCm |
| RZ011179 | CmsUmsCmAmGmUmCfAfUfUmCmGmCmCmCmUmUmCmAm L96 | UmsGfsAmAmGmGfGmCmGmAmAmUmGmAfCmUfGmAmGmsAmsUm |
| RZ011180 | GmsCmsUmGmUmGmGfUfGfUmCmUmGmAmGmUmAmCmUm L96 | AmsGfsUmAmCmUfCmAmGmAmCmAmCmCfAmCfAmGmCmsCmsCm |
| RZ011181 | GmsCmsUmGmAmCmAfGfCfAmGmCmAmCmAmUmUmGmUm L96 | AmsCfsAmAmUmGfUmGmCmUmGmCmUmGfUmCfAmGmCmsAmsCm |
| RZ011182 | CmsAmsCmAmUmUmGfUfUfUmCmAmCmUmGmUmGmGmAm L96 | UmsCfsCmAmCmAfGmUmGmAmAmAmCmAfAmUfGmUmGmsCmsUm |
| RZ011183 | GmsUmsUmUmCmAmCfUfGfUmGmGmAmUmGmAmCmAmAm L96 | UmsUfsGmUmCmAfUmCmCmAmCmAmGmUfGmAfAmAmCmsAmsAm |
| RZ011184 | CmsAmsCmUmGmUmGfGfAfUmGmAmCmAmAmGmGmAmAm L96 | UmsUfsCmCmUmUfGmUmCmAmUmCmCmAfCmAfGmUmGmsAmsAm |
| RZ011185 | CmsUmsGmUmGmGmAfUfGfAmCmAmAmGmGmAmAmCmAm L96 | UmsGfsUmUmCmCfUmUmGmUmCmAmUmCfCmAfCmAmGmsUmsGm |
| RZ011186 | GmsUmsGmGmAmUmGfAfCfAmAmGmGmAmAmCmAmCmUm L96 | AmsGfsUmGmUmUfCmCmUmUmGmUmCmAfUmCfCmAmCmsAmsGm |
| RZ011187 | AmsCmsAmAmGmGmAfAfCfAmCmUmCmAmAmUmCmAmAm L96 | UmsUfsGmAmUmUfGmAmGmUmGmUmUmCfCmUfUmGmUmsCmsAm |
| RZ011188 | GmsAmsAmCmAmCmUfCfAfAmUmCmAmAmGmGmUmCmAm L96 | UmsGfsAmCmCmUfUmGmAmUmUmGmAmGfUmGfUmUmCmsCmsUm |
| RZ011189 | GmsCmsGmGmGmAmCfCfUfGmGmAmGmAmUmAmGmAmAm L96 | UmsUfsCmUmAmUfCmUmCmCmAmGmGmUfCmCfCmGmCmsUmsUm |
| RZ011190 | GmsGmsGmAmCmCmUfGfGfAmGmAmUmAmGmAmAmGmUm L96 | AmsCfsUmUmCmUfAmUmCmUmCmCmAmGfGmUfCmCmCmsGmsCm |
| RZ011191 | GmsGmsAmGmAmUmAfGfAfAmGmUmAmGmUmCmCmUmAm L96 | UmsAfsGmGmAmCfUmAmCmUmUmCmUmAfUmCfUmCmCmsAmsGm |
| RZ011192 | CmsUmsAmUmUmUmCfAfCfCmCmCmAmAmCmUmAmCmAm L96 | UmsGfsUmAmGmUfUmGmGmGmGmUmGmAfAmAfUmAmGmsGmsAm |
| RZ011193 | GmsAmsAmGmCmAmGfGfAfAmUmUmCmCmUmGmAmAmUm L96 | AmsUfsUmCmAmGfGmAmAmUmUmCmCmUfGmCfUmUmCmsUmsUm |
| RZ011194 | CmsAmsGmGmAmAmUfUfCfCmUmGmAmAmUmUmUmUmAm L96 | UmsAfsAmAmAmUfUmCmAmGmGmAmAmUfUmCfCmUmGmsCmsUm |
| RZ011195 | GmsAmsAmUmUmCmCfUfGfAmAmUmUmUmUmAmUmGmAm L96 | UmsCfsAmUmAmAfAmAmUmUmCmAmGmGfAmAfUmUmCmsCmsUm |
| RZ011196 | CmsUmsGmAmAmUmUfUfUfAmUmGmAmCmUmAmUmGmAm L96 | UmsCfsAmUmAmGfUmCmAmUmAmAmAmAfUmUfCmAmGmsGmsAm |

(continued)

| No. | Modified Sense strand (5'-3') | Modified Antiense Strand (5'-3') |
|---|---|---|
| RZ011197 | GmsUmsUmGmCmCmCfUfGfAmUmCmAmAmGmCmUmCmAm L96 | UmsGfsAmGmCmUfUmGmAmUmCmAmGmGfGmCfAmAmCmsGmsUm |
| RZ011198 | GmsCmsCmCmUmGmAfUfCfAmAmGmCmUmCmAmAmGmAm L96 | UmsCfsUmUmGmAfGmCmUmUmGmAmUmCfAmGfGmGmCmsAmsAm |
| RZ011199 | CmsCmsCmUmGmAmUfCfAfAmGmCmUmCmAmAmGmAmAm L96 | UmsUfsCmUmUmGfAmGmCmUmUmGmAmUfCmAfGmGmGmsCmsAm |
| RZ011200 | CmsCmsUmGmAmUmCfAfAfGmCmUmCmAmAmGmAmAmUm L96 | AmsUfsUmCmUmUfGmAmGmCmUmUmGmAfUmCfAmGmGmsGmsCm |
| RZ011201 | UmsGmsAmUmCmAmAfGfCfUmCmAmAmGmAmAmUmAmAm L96 | UmsUfsAmUmUmCfUmUmGmAmGmCmUmUfGmAfUmCmAmsGmsGm |
| RZ011202 | GmsCmsUmCmAmAmGfAfAfUmAmAmGmCmUmGmAmAmAm L96 | UmsUfsUmCmAmGfCmUmUmAmUmUmCmUfUmGfAmGmCmsUmsUm |
| RZ011203 | GmsCmsCmAmGmAmCfUfAfUmCmAmGmGmCmCmCmAmUm L96 | AmsUfsGmGmGmCfCmUmGmAmUmAmGmUfCmUfGmGmCmsCmsAm |
| RZ011204 | CmsCmsAmGmAmCmUfAfUfCmAmGmGmCmCmCmAmUmUm L96 | AmsAfsUmGmGmGfCmCmUmGmAmUmAmGfUmCfUmGmGmsCmsCm |
| RZ011205 | CmsAmsGmAmCmUmAfUfCfAmGmGmCmCmCmAmUmUmUm L96 | AmsAfsAmUmGmGfGmCmCmUmGmAmUmAfGmUfCmUmGmsGmsCm |
| RZ011206 | GmsAmsCmUmAmUmCfAfGfGmCmCmCmAmUmUmUmGmUm L96 | AmsCfsAmAmAmUfGmGmGmCmCmUmGmAfUmAfGmUmCmsUmsGm |
| RZ011207 | CmsAmsCmCmGmAmGfGfGfAmAmCmAmAmCmUmCmGmAm L96 | UmsCfsGmAmGmUfUmGmUmUmCmCmCmUfCmGfGmUmGmsCmsAm |
| RZ011208 | CmsGmsAmGmGmGmAfAfCfAmAmCmUmCmGmAmGmCmUm L96 | AmsGfsCmUmCmGfAmGmUmUmGmUmUmCfCmCfUmCmGmsGmsUm |
| RZ011209 | GmsAmsGmGmGmAmAfCfAfAmCmUmCmGmAmGmCmUmUm L96 | AmsAfsGmCmUmCfGmAmGmUmUmGmUmUfCmCfCmUmCmsGmsGm |
| RZ011210 | GmsCmsUmUmUmGmAfGfGfCmUmUmCmCmUmCmCmAmAm L96 | UmsUfsGmGmAmGfGmAmAmGmCmCmUmCfAmAfAmGmCmsUmsCm |
| RZ011211 | GmsCmsUmUmCmCmUfCfCfAmAmCmUmAmCmCmAmCmUm L96 | AmsGfsUmGmGmUfAmGmUmUmGmGmAmGfGmAfAmGmCmsCmsUm |
| RZ011212 | CmsUmsUmCmCmUmCfCfAfAmCmUmAmCmCmAmCmUmUm L96 | AmsAfsGmUmGmGfUmAmGmUmUmGmGmAfGmGfAmAmGmsCmsCm |
| RZ011213 | GmsCmsUmGmCmUmCfCfCfUmGmCmAmCmAmGmGmAmUm L96 | AmsUfsCmCmUmGfUmGmCmAmGmGmGmAfGmCfAmGmCmsUmsCm |
| RZ011214 | CmsCmsCmUmGmCmAfCfAfGmGmAmUmAmUmCmAmAmAm L96 | UmsUfsUmGmAmUfAmUmCmCmUmGmUmGfCmAfGmGmGmsAmsGm |
| RZ011215 | CmsAmsCmAmGmGmAfUfAfUmCmAmAmAmGmCmUmCmUm L96 | AmsGfsAmGmCmUfUmUmGmAmUmAmUmCfCmUfGmUmGmsCmsAm |

(continued)

| No. | Modified Sense strand (5'-3') | Modified Antiense Strand (5'-3') |
|---|---|---|
| RZ011216 | CmsAmsGmGmAmUmAfUfCfAmAmAm GmCmUmCmUmGmUm L96 | AmsCfsAmGmAmGfCmUmUmUmGmAmU mAfUmCfCmUmGmsUmsGm |
| RZ011217 | AmsGmsGmAmUmAmUfCfAfAmAmGm CmUmCmUmGmUmUm L96 | AmsAfsCmAmGmAfGmCmUmUmUmGmA mUfAmUfCmCmUmsGmsUm |
| RZ011218 | GmsGmsAmUmAmUmCfAfAfAmGmCm UmCmUmGmUmUmUm L96 | AmsAfsAmCmAmGfAmGmCmUmUmUmG mAfUmAfUmCmCmsUmsGm |
| RZ011219 | GmsAmsUmAmUmCmAfAfAfGmCmUm CmUmGmUmUmUmGm L96 | CmsAfsAmAmCmAfGmAmGmCmUmUmU mGfAmUfAmUmCmsCmsUm |
| RZ011220 | CmsUmsCmUmGmUmUfUfGfUmGmUm CmUmGmAmGmGmAm L96 | UmsCfsCmUmCmAfGmAmCmAmCmAmA mAfCmAfGmAmGmsCmsUm |
| RZ011221 | CmsUmsGmAmCmUmCfGfGfAmAmGm GmAmGmGmUmCmUm L96 | AmsGfsAmCmCmUfCmCmUmUmCmCmG mAfGmUfCmAmGmsCmsUm |
| RZ011222 | UmsGmsAmCmUmCmGfGfAfAmGmGm AmGmGmUmCmUmAm L96 | UmsAfsGmAmCmCfUmCmCmUmUmCmC mGfAmGfUmCmAmsGmsCm |
| RZ011223 | GmsAmsCmUmCmGmGfAfAfGmGmAm GmGmUmCmUmAmCm L96 | GmsUfsAmGmAmCfCmUmCmCmUmUmC mCfGmAfGmUmCmsAmsGm |
| RZ011224 | CmsUmsCmGmGmAmAfGfGfAmGmGm UmCmUmAmCmAmUm L96 | AmsUfsGmUmAmGfAmCmCmUmCmCmU mUfCmCfGmAmGmsUmsCm |
| RZ011225 | GmsGmsAmAmGmGmAfGfGfUmCmUm AmCmAmUmCmAmAm L96 | UmsUfsGmAmUmGfUmAmGmAmCmCmU mCfCmUfUmCmCmsGmsAm |
| RZ011226 | GmsAmsAmGmGmAmGfGfUfCmUmAm CmAmUmCmAmAmGm L96 | CmsUfsUmGmAmUfGmUmAmGmAmCmC mUfCmCfUmUmCmsCmsGm |
| RZ011227 | GmsGmsAmGmGmUmCfUfAfCmAmUm CmAmAmGmAmAmUm L96 | AmsUfsUmCmUmUfGmAmUmGmUmAmG mAfCmCfUmCmCmsUmsUm |
| RZ011228 | GmsAmsGmGmUmCmUfAfCfAmUmCm AmAmGmAmAmUmGm L96 | CmsAfsUmUmCmUfUmGmAmUmGmUmA mGfAmCfCmUmCmsCmsUm |
| RZ011229 | CmsAmsAmGmAmAmUfGfGfGmGmAm UmAmAmGmAmAmAm L96 | UmsUfsUmCmUmUfAmUmCmCmCmCmA mUfUmCfUmUmGmsAmsUm |
| RZ011230 | GmsCmsUmGmUmGmAfGfAfGmAmGm AmUmGmCmUmCmAm L96 | UmsGfsAmGmCmAfUmCmUmCmUmCmU mCfAmCfAmGmCmsUmsGm |
| RZ011231 | CmsUmsGmUmGmAmGfAfGfAmGmAm UmGmCmUmCmAmAm L96 | UmsUfsGmAmGmCfAmUmCmUmCmUmC mUfCmAfCmAmGmsCmsUm |
| RZ011232 | GmsUmsGmAmGmAmGfAfGfAmUmGm CmUmCmAmAmUmAm L96 | UmsAfsUmUmGmAfGmCmAmUmCmUmC mUfCmUfCmAmCmsAmsGm |
| RZ011233 | CmsAmsGmGmCmUmAfUfGfAmCmAm AmAmGmUmCmAmAm L96 | UmsUfsGmAmCmUfUmUmGmUmCmAmU mAfGmCfCmUmGmsGmsGm |
| RZ011234 | GmsCmsUmAmUmGmAfCfAfAmAmGm UmCmAmAmGmGmAm L96 | UmsCfsCmUmUmGfAmCmUmUmUmGmU mCfAmUfAmGmCmsCmsUm |

(continued)

| No. | Modified Sense strand (5'-3') | Modified Antiense Strand (5'-3') |
|---|---|---|
| RZ011235 | GmsAmsCmAmAmAmGfUfCfAmAmGmGmAmCmAmUmCmUm L96 | AmsGfsAmUmGmUfCmCmUmUmGmAmCmUfUmUfGmUmCmsAmsUm |
| RZ011236 | GmsUmsUmCmCmUmUfUfGfUmAmCmUmGmGmAmGmGmAm L96 | UmsCfsCmUmCmCfAmGmUmAmCmAmAmAfGmGfAmAmCmsCmsGm |
| RZ011237 | CmsUmsGmGmAmGmGfAfGfUmGmAmGmUmCmCmCmUmAm L96 | UmsAfsGmGmGmAfCmUmCmAmCmUmCmCfUmCfCmAmGmsUmsAm |
| RZ011238 | CmsUmsUmGmAmUmAfGfUfUmCmAmCmAmAmGmAmGmAm L96 | UmsCfsUmCmUmUfGmUmGmAmAmCmUmAfUmCfAmAmGmsGmsGm |
| RZ011239 | GmsUmsUmCmAmCmAfAfGfAmGmAmAmGmUmCmGmUmUm L96 | AmsAfsCmGmAmCfUmUmCmUmCmUmUmGfUmGfAmAmCmsUmsAm |
| RZ011240 | CmsAmsCmAmAmGmAfGfAfAmGmUmCmGmUmUmUmCmAm L96 | UmsGfsAmAmAmCfGmAmCmUmUmCmUmCfUmUfGmUmGmsAmsAm |
| RZ011241 | CmsAmsAmGmAmGmAfAfGfUmCmGmUmUmUmCmAmUmUm L96 | AmsAfsUmGmAmAfAmCmGmAmCmUmUmCfUmCfUmUmGmsUmsGm |
| RZ011242 | AmsGmsAmGmAmAmGfUfCfGmUmUmUmCmAmUmUmCmAm L96 | UmsGfsAmAmUmGfAmAmAmCmGmAmCmUfUmCfUmCmUmsUmsGm |
| RZ011243 | GmsAmsGmAmAmGmUfCfGfUmUmUmCmAmUmUmCmAmAm L96 | UmsUfsGmAmAmUfGmAmAmAmCmGmAmCfUmUfCmUmCmsUmsUm |
| RZ011244 | GmsAmsAmGmUmCmGfUfUfUmCmAmUmUmCmAmAmGmUm L96 | AmsCfsUmUmGmAfAmUmGmAmAmAmCmGfAmCfUmUmCmsUmsCm |
| RZ011245 | GmsUmsUmUmCmAmUfUfCfAmAmGmUmUmGmGmUmGmUm L96 | AmsCfsAmCmCmAfAmCmUmUmGmAmAmUfGmAfAmAmCmsGmsAm |
| RZ011246 | CmsCmsCmGmAmGmAfCfUfUmUmCmAmCmAmUmCmAmAm L96 | UmsUfsGmAmUmGfUmGmAmAmAmGmUmCfUmCfGmGmGmsCmsGm |
| RZ011247 | GmsAmsGmAmCmUmUfUfCfAmCmAmUmCmAmAmCmCmUm L96 | AmsGfsGmUmUmGfAmUmGmUmGmAmAmAfGmUfCmUmCmsGmsGm |
| RZ011248 | GmsAmsCmUmUmUmCfAfCfAmUmCmAmAmCmCmUmCmUm L96 | AmsGfsAmGmGmUfUmGmAmUmGmUmGmAfAmAfGmUmCmsUmsCm |
| RZ011249 | CmsCmsUmGmGmCmUfGfAfAmGmGmAmGmAmAmAmCmUm L96 | AmsGfsUmUmUmCfUmCmCmUmUmCmAmGfCmCfAmGmGmsGmsCm |
| RZ011250 | GmsGmsCmUmGmAmAfGfGfAmGmAmAmAmCmUmCmCmAm L96 | UmsGfsGmAmGmUfUmUmCmUmCmCmUmUfCmAfGmCmCmsAmsGm |
| RZ011251 | GmsCmsUmGmAmAmGfGfAfGmAmAmAmCmUmCmCmAmAm L96 | UmsUfsGmGmAmGfUmUmUmCmUmCmCmUfUmCfAmGmCmsCmsAm |
| RZ011252 | GmsAmsAmGmGmAmGfAfAfAmCmUmCmCmAmAmGmAmUm L96 | AmsUfsCmUmUmGfGmAmGmUmUmUmCmUfCmCfUmUmCmsAmsGm |
| RZ011253 | GmsAmsAmAmCmUmCfCfAfAmGmAmUmGmAmGmGmAmUm L96 | AmsUfsCmCmUmCfAmUmCmUmUmGmGmAfGmUfUmUmCmsUmsCm |

(continued)

| No. | Modified Sense strand (5'-3') | Modified Antiense Strand (5'-3') |
|---|---|---|
| RZ011254 | GmsAmsGmGmAmUmUfUfGfGmGmUm UmUmUmCmUmAmUm L96 | AmsUfsAmGmAmAfAmAmCmCmCmAmA mAfUmCfCmUmCmsAmsUm |
| RZ011255 | GmsGmsAmUmUmUmGfGfGfUmUmUm UmCmUmAmUmAmAm L96 | UmsUfsAmUmAmGfAmAmAmAmCmCmC mAfAmAfUmCmCmsUmsCm |
| RZ011256 | CmsCmsUmUmUmAmUfCfUfUfGmGmG mCmCmUmCmUmUm L96 | AmsAfsGmAmGmGfCmCmCfAmAmGmAm UfA(moe)AfAmGmGmsGmsCm |
| RZ011257 | AmsAmsGmGmCmGmGfCfUfCfCmUmU mCmCmGmAmCmUm L96 | AmsGfsUmCmGmGfAmAmGfGmAmGmCm CfG(moe)CfCmUmUmsUmsGm |
| RZ011258 | GmsCmsGmGmCmUmCfCfUfUfCmCmG mAmCmUmUmCmUm L96 | AmsGfsAmAmGmUfCmGmGfAmAmGmGm AfG(moe)CfCmGmCmsCmsUm |
| RZ011259 | GmsCmsUmCmCmUmUfCfCfGfAmCmU mUmCmUmCmCmAm L96 | UmsGfsGmAmGmAfAmGmUfCmGmGmAm AfG(moe)GfAmGmCmsCmsGm |
| RZ011260 | CmsCmsCmGmUmAmCfCfCfUfGmUmG mCmAmGmAmCmAm L96 | UmsGfsUmCmUmGfCmAmCfAmGmGmGm UfA(moe)CfGmGmGmsUmsAm |
| RZ011261 | CmsAmsGmAmCmAmCfGfUfAfCmCmU mGmCmAmGmAmUm L96 | AmsUfsCmUmGmCfAmGmGfUmAmCmGm UfG(moe)UfCmUmGmsCmsAm |
| RZ011262 | AmsCmsAmCmGmUmAfCfCfUfGmCmA mGmAmUmCmUmAm L96 | UmsAfsGmAmUmCfUmGmCfAmGmGmUm AfC(moe)GfUmGmUmsCmsUm |
| RZ011263 | CmsUmsAmCmUmAmCfAfAfUfGmUmG mAmGmUmGmAmUm L96 | AmsUfsCmAmCmUfCmAmCfAmUmUmGm UfA(moe)GfUmAmGmsGmsGm |
| RZ011264 | CmsAmsAmUmGmUmGfAfGfUfGmAmU mGmAmGmAmUmCm L96 | GmsAfsUmCmUmCfAmUmCfAmCmUmCm AfC(moe)AfUmUmGmsUmsAm |
| RZ011265 | GmsUmsGmAmUmGmAfGfAfUfCmUmC mUmUmUmCmCmAm L96 | UmsGfsGmAmAmAfGmAmGfAmUmCmUm CfA(moe)UfCmAmCmsUmsCm |
| RZ011266 | AmsGmsAmUmCmUmCfUfUfUfCmCmA mCmUmGmCmUmAm L96 | UmsAfsGmCmAmGfUmGmGfAmAmAmGm AfG(moe)AfUmCmUmsCmsAm |
| RZ011267 | CmsAmsGmAmCmAmGfCfGfAfUmCmU mGmUmGmAmCmAm L96 | UmsGfsUmCmAmCfAmGmAfUmCmGmCm UfG(moe)UfCmUmGmsCmsCm |
| RZ011268 | GmsCmsCmGmAmAmGfCfUfUfUmCmC mUmGmUmCmUmUm L96 | AmsAfsGmAmCmAfGmGmAfAmAmGmCm UfU(moe)CfGmGmCmsCmsAm |
| RZ011269 | CmsCmsUmGmGmUmGfCfUfAfGmAmU mGmGmAmUmCmAm L96 | UmsGfsAmUmCmCfAmUmCfUmAmGmCm AfC(moe)CfAmGmGmsUmsAm |
| RZ011270 | CmsUmsAmGmAmUmGfGfAfUfCmAmG mAmCmAmGmCmAm L96 | UmsGfsCmUmGmUfCmUmGfAmUmCmCm AfU(moe)CfUmAmGmsCmsAm |
| RZ011271 | CmsAmsAmCmUmUmCfAfCfAfGmGmA mGmCmCmAmAmAm L96 | UmsUfsUmGmGmCfUmCmCfUmGmUmGm AfA(moe)GfUmUmGmsCmsUm |
| RZ011272 | AmsAmsAmGmUmGmUfCfUfAfGmUmC mAmAmCmUmUmAm L96 | UmsAfsAmGmUmUfGmAmCfUmAmGmAm CfA(moe)CfUmUmUmsUmsUm |

(continued)

| No. | Modified Sense strand (5'-3') | Modified Antiense Strand (5'-3') |
|---|---|---|
| RZ011273 | AmsAmsGmUmGmUmCfUfAfGfUmCmAmAmCmUmUmAmAm L96 | UmsUfsAmAmGmUfUmGmAfCmUmAmGmAfC(moe)AfCmUmUmsUmsUm |
| RZ011274 | AmsGmsUmGmUmCmUfAfGfUfCmAmAmCmUmUmAmAmUm L96 | AmsUfsUmAmAmGfUmUmGfAmCmUmAmGfA(moe)CfAmCmUmsUmsUm |
| RZ011275 | CmsUmsAmGmUmCmAfAfCfUfUmAmAmUmUmGmAmGmAm L96 | UmsCfsUmCmAmAfUmUmAfAmGmUmUmGfA(moe)CfUmAmGmsAmsCm |
| RZ011276 | UmsUmsGmAmGmAmAfGfGfUfGmGmCmAmAmGmUmUmAm L96 | UmsAfsAmCmUmUfGmCmCfAmCmCmUmUfC(moe)UfCmAmAmsUmsUm |
| RZ011277 | GmsUmsGmGmCmAmAfGfUfUfAmUmGmGmUmGmUmGmAm L96 | UmsCfsAmCmAmCfCmAmUfAmAmCmUmUfG(moe)CfCmAmCmsCmsUm |
| RZ011278 | AmsAmsGmAmUmAmUfGfGfUfCmUmAmGmUmGmAmCmAm L96 | UmsGfsUmCmAmCfUmAmGfAmCmCmAmUfA(moe)UfCmUmUmsGmsGm |
| RZ011279 | GmsAmsUmAmUmGmGfUfCfUfAmGmUmGmAmCmAmUmAm L96 | UmsAfsUmGmUmCfAmCmUfAmGmAmCmCfA(moe)UfAmUmCmsUmsUm |
| RZ011280 | CmsCmsAmAmAmAmUfUfUfGfGmGmUmCmAmAmAmGmUm L96 | AmsCfsUmUmUmGfAmCmCfCmAmAmAmUfU(moe)UfUmGmGmsGmsGm |
| RZ011281 | GmsCmsAmGmCmUmCfAfAfUfGmAmAmAmUmCmAmAmUm L96 | AmsUfsUmGmAmUfUmUmCfAmUmUmGmAfG(moe)CfUmGmCmsUmsUm |
| RZ011282 | GmsCmsUmCmAmAmUfGfAfAfAmUmCmAmAmUmUmAmUm L96 | AmsUfsAmAmUmUfGmAmUfUmUmCmAmUfU(moe)GfAmGmCmsUmsGm |
| RZ011283 | CmsAmsAmUmGmAmAfAfUfCfAmAmUmUmAmUmGmAmAm L96 | UmsUfsCmAmUmAfAmUmUfGmAmUmUmUfC(moe)AfUmUmGmsAmsGm |
| RZ011284 | GmsCmsCmAmUmGmUfCfAfUfCmAmUmCmCmUmCmAmUm L96 | AmsUfsGmAmGmGfAmUmGfAmUmGmAmCfA(moe)UfGmGmCmsGmsGm |
| RZ011285 | AmsGmsGmAmUmUmAfUfCfUfGmGmAmUmGmUmCmUmAm L96 | UmsAfsGmAmCmAfUmCmCfAmGmAmUmAfA(moe)UfCmCmUmsCmsCm |
| RZ011286 | GmsGmsAmUmUmAmUfCfUfGfGmAmUmGmUmCmUmAmUm L96 | AmsUfsAmGmAmCfAmUmCfCmAmGmAmUfA(moe)AfUmCmCmsUmsCm |
| RZ011287 | UmsCmsUmGmGmAmUfGfUfCfUmAmUmGmUmGmUmUmUm L96 | AmsAfsAmCmAmCfAmUmAfGmAmCmAmUfC(moe)CfAmGmAmsUmsAm |
| RZ011288 | AmsAmsGmUmGmAmAfCfAfUfCmAmAmUmGmCmUmUmUm L96 | AmsAfsAmGmCmAfUmUmGfAmUmGmUmUfC(moe)AfCmUmUmsGmsGm |
| RZ011289 | CmsAmsAmGmAmAmAfGfAfCfAmAmUmGmAmGmCmAmAm L96 | UmsUfsGmCmUmCfAmUmUfGmUmCmUmUfU(moe)CfUmUmGmsGmsAm |
| RZ011290 | CmsUmsCmUmGmAmGfUfCfUfCmUmGmUmGmGmCmAmUm L96 | AmsUfsGmCmCmAfCmAmGfAmGmAmCmUfC(moe)AfGmAmGmsAmsCm |
| RZ011291 | GmsUmsUmUmCmAmCfUfGfUfGmGmAmUmGmAmCmAmAm L96 | UmsUfsGmUmCmAfUmCmCfAmCmAmGmUfG(moe)AfAmAmCmsAmsAm |

(continued)

| No. | Modified Sense strand (5'-3') | Modified Antiense Strand (5'-3') |
|---|---|---|
| RZ011292 | GmsAmsAmGmCmAmGfGfAfAfUmUmCmCmUmGmAmAmUm L96 | AmsUfsUmCmAmGfGmAmAfUmUmCmCmUfG(moe)CfUmUmCmsUmsUm |
| RZ011293 | CmsAmsGmGmAmAmUfUfCfCfUmGmAmAmUmUmUmUmAm L96 | UmsAfsAmAmAmUfUmCmAfGmGmAmAmUfU(moe)CfCmUmGmsCmsUm |
| RZ011294 | CmsCmsCmUmGmAmUfCfAfAfGmCmUmCmAmAmGmAmAm L96 | UmsUfsCmUmUmGfAmGmCfUmUmGmAmUfC(moe)AfGmGmGmsCmsAm |
| RZ011295 | UmsGmsAmUmCmAmAfGfCfUfCmAmAmGmAmAmUmAmAm L96 | UmsUfsAmUmUmCfUmUmGfAmGmCmUmUfG(moe)AfUmCmAmsGmsGm |
| RZ011296 | GmsCmsUmCmAmAmGfAfAfUfAmAmGmCmUmGmAmAmAm L96 | UmsUfsUmCmAmGfCmUmUfAmUmUmCmUfU(moe)GfAmGmCmsUmsUm |
| RZ011297 | CmsAmsGmGmCmUmAfUfGfAfCmAmAmAmGmUmCmAmAm L96 | UmsUfsGmAmCmUfUmUmGfUmCmAmUmAfG(moe)CfCmUmGmsGmsGm |
| RZ011298 | CmsUmsUmGmAmUmAfGfUfUfCmAmCmAmAmGmAmGmAm L96 | UmsCfsUmCmUmUfGmUmGfAmAmCmUmAfU(moe)CfAmAmGmsGmsGm |
| RZ011299 | CmsAmsAmGmAmGmAfAfGfUfCmGmUmUmUmCmAmUmUm L96 | AmsAfsUmGmAmAfAmCmGfAmCmUmUmCfU(moe)CfUmUmGmsUmsGm |
| RZ011300 | AmsGmsAmGmAmAmGfUfCfGfUmUmUmCmAmUmUmCmAm L96 | UmsGfsAmAmUmGfAmAmAfCmGmAmCmUfU(moe)CfUmCmUmsUmsGm |
| RZ011301 | GmsAmsGmAmAmGmUfCfGfUfUmUmCmAmUmUmCmAmAm L96 | UmsUfsGmAmAmUfGmAmAfAmCmGmAmCfU(moe)UfCmUmCmsUmsUm |
| RZ011302 | GmsAmsGmGmAmUmUfUfGfGfGmUmUmUmUmCmUmAmUm L96 | AmsUfsAmGmAmAfAmAmCfCmCmAmAmAfU(moe)CfCmUmCmsAmsUm |
| RZ011303 | GmsGmsAmUmUmUmGfGfGfUfUmUmUmCmUmAmUmAmAm L96 | UmsUfsAmUmAmGfAmAmAfAmCmCmCmAfA(moe)AfUmCmCmsUmsCm |
| RZM11001 | GmsGmsAmGmGmGmAfGfUfAmGmAmGmAmUmCmAmAmAm L96 | UmsUfsUmGmAmUfCmUmCmUmAmCmUmCfCmCfUmCmCmsAmsGm |
| RZM11002 | CmsCmsCmUmCmUmGfGfCfUmUmCmUmAmCmCmCmAmUm L96 | AmsUfsGmGmGmUfAmGmAmAmGmCmCmAfGmAfGmGmGmsAmsCm |
| RZM11003 | GmsGmsCmGmGmAmAfUfGfCmAmGmAmGmCmAmAmUmAm L96 | UmsAfsUmUmGmCfUmCmUmGmCmAmUmUfCmCfGmCmCmsUmsUm |
| RZM11004 | GmsAmsGmAmAmUmGfGfCfCmGmGmUmGmGmGmAmUmGm L96 | CmsAfsUmCmCmCfAmCmCmGmGmCmCmAfUmUfCmUmCmsUmsUm |
| RZM11005 | CmsCmsAmAmUmCmCfCfGfGmUmAmUmUmCmCmUmAmUm L96 | AmsUfsAmGmGmAfAmUmAmCmCmGmGmGfAmUfUmGmGmsGmsAm |
| RZM 11006 | CmsGmsGmUmAmUmUfCfCfUmAmUmUmGmGmGmAmCmAm L96 | UmsGfsUmCmCmCfAmAmUmAmGmGmAmAfUmAfCmCmGmsGmsGm |
| RZM11007 | GmsGmsUmAmUmUmCfCfUfAmUmUmGmGmGmAmCmAmAm L96 | UmsUfsGmUmCmCfCmAmAmUmAmGmGmAfAmUfAmCmCmsGmsGm |

(continued)

| No. | Modified Sense strand (5'-3') | Modified Antiense Strand (5'-3') |
|---|---|---|
| RZM11008 | CmsCmsAmGmAmAmGfCfGfAmAmAmGmUmGmUmCmAmAm L96 | UmsUfsGmAmCmAfCmUmUmUmCmGmCmUfUmCfUmGmGmsGmsAm |
| RZM11009 | GmsAmsAmGmCmGmAfAfAfGmUmGmUmCmAmAmGmAmAm L96 | UmsUfsCmUmUmGfAmCmAmCmUmUmUmCfGmCfUmUmCmsUmsGm |
| RZM11010 | GmsCmsGmAmAmAmGfUfGfUmCmAmAmGmAmAmGmGmUm L96 | AmsCfsCmUmUmCfUmUmGmAmCmAmCmUfUmUfCmGmCmsUmsUm |
| RZM11011 | CmsGmsGmGmCmUmCfCfAfUmGmAmAmUmAmUmCmUmAm L96 | UmsAfsGmAmUmAfUmUmCmAmUmGmGmAfGmCfCmCmGmsAmsGm |
| RZM11012 | GmsGmsAmUmCmAmGfAfCfAmGmCmAmUmCmGmGmAmAm L96 | UmsUfsCmCmGmAfUmGmCmUmGmUmCmUfGmAfUmCmCmsAmsUm |
| RZM11013 | GmsCmsAmUmCmGmGfAfAfGmCmAmGmCmAmAmCmUmUm L96 | AmsAfsGmUmUmGfCmUmGmCmUmUmCmCfGmAfUmGmCmsUmsGm |
| RZM11014 | CmsAmsGmAmGmAmAfGfCfUmCmAmAmCmCmAmAmAmUm L96 | AmsUfsUmUmGmGfUmUmGmAmGmCmUmUfCmUfCmUmGmsUmsGm |
| RZM11015 | GmsCmsUmCmAmAmCfCfAfAmAmUmCmAmGmUmUmAmUm L96 | AmsUfsAmAmCmUfGmAmUmUmUmGmGmUfUmGfAmGmCmsUmsUm |
| RZM11016 | CmsAmsAmCmCmAmAfAfUfCmAmGmUmUmAmUmGmAmAm L96 | UmsUfsCmAmUmAfAmCmUmGmAmUmUmUfGmGfUmUmGmsAmsGm |
| RZM11017 | CmsCmsUmGmAmAmGfGfCfUmGmGmAmAmUmAmGmAmAm L96 | UmsUfsCmUmAmUfUmCmCmAmGmCmCmUfUmCfAmGmGmsCmsGm |
| RZM11018 | CmsAmsUmCmAmUmGfUfGfUmUmUmAmAmAmGmUmCmAm L96 | UmsGfsAmCmUmUfUmAmAmAmCmAmCmAfUmGfAmUmGmsCmsUm |
| RZM11019 | GmsUmsGmUmUmUmAfAfAfGmUmCmAmAmGmGmAmUmAm L96 | UmsAfsUmCmCmUfUmGmAmCmUmUmUmAfAmAfCmAmCmsAmsUm |
| RZM11020 | CmsAmsAmCmGmAmUfUfAfUmCmAmUmAmAmGmCmAmAm L96 | UmsUfsGmCmUmUfAmUmGmAmUmAmAmUfCmGfUmUmGmsCmsCm |
| RZM11021 | CmsCmsGmUmGmGmUfGfUfCmUmGmAmGmUmAmCmUmUm L96 | AmsAfsGmUmAmCfUmCmAmGmAmCmAmCfCmAfCmGmGmsCmsCm |
| RZM11022 | GmsCmsUmUmCmAmUfGfGfUmGmGmAmUmGmAmUmCmAm L96 | UmsGfsAmUmCmAfUmCmCmAmCmCmAmUfGmAfAmGmCmsAmsGm |
| RZM11023 | CmsAmsUmGmGmUmGfGfAfUmGmAmUmCmAmGmAmAmAm L96 | UmsUfsUmCmUmGfAmUmCmAmUmCmCmAfCmCfAmUmGmsAmsAm |
| RZM11024 | CmsCmsCmUmAmGmUfCfAfAmGmCmUmCmAmAmGmAmAm L96 | UmsUfsCmUmUmGfAmGmCmUmUmGmAmCfUmAfGmGmGmsCmsCm |
| RZM11025 | UmsAmsGmUmCmAmAfGfCfUmCmAmAmGmAmAmCmAmAm L96 | UmsUfsGmUmUmCfUmUmGmAmGmCmUmUfGmAfCmUmAmsGmsGm |
| RZM11026 | AmsGmsCmUmCmAmAfGfAfAmCmAmAmGmCmUmCmAmAm L96 | UmsUfsGmAmGmCfUmUmGmUmUmCmUmUfGmAfGmCmUmsUmsGm |

(continued)

| No. | Modified Sense strand (5'-3') | Modified Antiense Strand (5'-3') |
|---|---|---|
| RZM11027 | CmsAmsAmGmAmAmCfAfAfGmCmUm CmAmAmGmUmAmUm L96 | AmsUfsAmCmUmUfGmAmGmCmUmUmG mUfUmCfUmUmGmsAmsGm |
| RZM11028 | GmsCmsCmAmGmAmCfUfCfUmCmAm GmGmCmCmCmAmUm L96 | AmsUfsGmGmGmCfCmUmGmAmGmAmG mUfCmUfGmGmCmsCmsAm |
| RZM11029 | CmsCmsAmCmCmUmGfCfAfAmGmCmA mGmCmAmCmAmAm L96 | UmsUfsGmUmGmCfUmGmCmUmUmGmC mAfGmGfUmGmGmsCmsUm |
| RZM11030 | CmsCmsCmUmGmUmGfAfAfGmGmAm UmGmUmCmAmAmAm L96 | UmsUfsUmGmAmCfAmUmCmCmUmUmC mAfCmAfGmGmGmsAmsGm |
| RZM11031 | GmsGmsAmAmGmAmGfCfCfUmGmAm CmUmCmGmGmAmAm L96 | UmsUfsCmCmGmAfGmUmCmAmGmGmC mUfCmUfUmCmCmsCmsUm |
| RZM11032 | GmsGmsAmAmGmGmAfGfGfUmGmUm AmCmAmUmCmAmAm L96 | UmsUfsGmAmUmGfUmAmCmAmCmCmU mCfCmUfUmCmCmsGmsAm |
| RZM11033 | GmsGmsAmGmGmUmGfUfAfCmAmUm CmAmAmGmAmAmUm L96 | AmsUfsUmCmUmUfGmAmUmGmUmAmC mAfCmCfUmCmCmsUmsUm |
| RZM11034 | GmsCmsUmAmUmGmAfGfAfAmGmGm UmCmAmAmAmGmAm L96 | UmsCfsUmUmUmGfAmCmCmUmUmCmU mCfAmUfAmGmCmsCmsUm |
| RZM11035 | GmsCmsCmCmUmCmUfCfAfUmUmGm UmUmCmAmCmAmAm L96 | UmsUfsGmUmGmAfAmCmAmAmUmGmA mGfAmGfGmGmCmsCmsCm |
| RZM11036 | GmsGmsCmUmAmAmAfGfGfAmCmAm AmGmCmUmCmAmAm L96 | UmsUfsGmAmGmCfUmUmGmUmCmCmU mUfUmAfGmCmCmsAmsGm |
| RZM11037 | AmsGmsGmAmCmAmAfGfCfUmCmAm AmAmGmAmUmGmAm L96 | UmsCfsAmUmCmUfUmUmGmAmGmCmU mUfGmUfCmCmUmsUmsUm |
| RZM11038 | GmsAmsGmUmGmUmGfAfGfGmAmCm AmGmAmUmUmAmAm L96 | UmsUfsAmAmUmCfUmGmUmCmCmUmC mAfCmAfCmUmCmsUmsCm |
| RZM11039 | GmsAmsUmUmAmAmAfGfCfAmGmUm UmAmCmAmAmUmAm L96 | UmsAfsUmUmGmUfAmAmCmUmGmCmU mUfUmAfAmUmCmsUmsGm |

In the sequences, meanings of base composition and modifications are as follows: capital letters A, U, G, C, and T represent base composition of the nucleotides; lowercase letter m indicates that the nucleotide adjacent to the left side of the letter m is a 2'-methoxy-modified nucleotide; lowercase letter f indicates that the nucleotide adjacent to the left side of the letter f is 2'-fluoro-modified nucleotide; lowercase letter s indicates phosphorothioate linkage between two nucleotides adjacent to both left and right sides of the letter s; (moe) indicates that the nucleotide adjacent to the left side of combined identity (moe) is a nucleotide modified with 2'-O-methoxyethyl (i.e., 2'-O-MOE); and L96 is an *N*-acetylgalactosamine.

**Example 2 Preparation of Double-Stranded RNAi Agents with (CR01008)×3 as Ligand**

Step 1: (CR01008)×3 ligand was prepared by the method described in Preparation Example 2.

Step 2: synthesis of the sense strand: by the solid-phase synthetic method for phosphoramidite nucleic acids, the above trimer of compound CR01008 linked to the solid-phase support was used to start cycles to link the nucleoside monomers one by one in the 3'-5' direction according to the sequence of nucleotides. The linking of each nucleoside monomer included a four-step reaction of deprotection, coupling, capping, and oxidation or sulfurization. For the synthesis method and conditions, reference is made to the synthesis method for the sense strand in Example 1.

Step 3: Synthesis of the antisense strand: The antisense strand was synthesized according to the synthesis method and conditions for the antisense strand described in Example 1.

Step 4: Synthesis of double-stranded RNAi agents: prepared with reference to the method in Example 1.

Information about the obtained double-stranded RNAi agents is listed in the following table.

Table 3 Sequences of Double-Stranded RNAi Agents with (CR01008)×3 as Ligand

| No. | Modified Sense Strand (5'-3') | Modified Antisense Strand (5'-3') |
|---|---|---|
| RZ011304 | GmsGmsAmUmUmUmGfGfGfUfUmUmUmCm UmAmUmAmAm (CR01008×3) | UmsUfsAmUmAmGfAmAmAfAmCmCmCmA fAmAfUmCmCmsUmsCm |
| RZ011305 | GmsGmsAmUmUmUmGfGfGfUfUmUmUmCm UmAmUmAmAm (CR01008×3) | UmsUfsAmUmAmGfAmAmAmAmCmCfCmA fAmAfUmCmCmsUmsCm |
| RZ011306 | GmsGmsAmUmUmUmGfGfGfUfUmUmUmCm UmAmUmAmAm (CR01008×3) | UmsUfsAmUmAmGfAmAmAfAmCmCmCmA fA(moe)AfUmCmCmsUmsCm |
| RZ011307 | GmsGmsAmUmUmUmGfGfGfUfUmUmUmCm UmAmUmAmsAm (CR01008×3) | UmsUfsAmUmAmGfAmAmAfAmsCmCmCm AfA(moe)AfUmCmCmsUmsCm |
| RZ011308 | CmsCmsUmUmUmAmUfCfUfUfGmGmGmCmC mUmCmUmUm CR01008×3 | AmsAfsGmAmGmGfCmCmCfAmAmGmAmU fA(moe)AfAmGmGmsGmsCm |
| RZ011309 | AmsAmsGmGmCmGmGfCfUfCfCmUmUmCmC mGmAmCmUm CR01008×3 | AmsGfsUmCmGmGfAmAmGfGmAmGmCmC fG(moe)CfCmUmUmsUmsGm |
| RZ011310 | GmsCmsGmGmCmUmCfCfUfUfCmCmGmAmC mUmUmCmUm CR01008×3 | AmsGfsAmAmGmUfCmGmGfAmAmGmGmA fG(moe)CfCmGmCmsCmsUm |
| RZ011311 | GmsCmsUmCmCmUmUfCfCfGfAmCmUmUmC mUmCmCmAm CR01008×3 | UmsGfsGmAmGmAfAmGmUfCmGmGmAmA fG(moe)GfAmGmCmsCmsGm |
| RZ011312 | CmsCmsCmGmUmAmCfCfCfUfGmUmGmCmA mGmAmCmAm CR01008×3 | UmsGfsUmCmUmGfCmAmCfAmGmGmGmU fA(moe)CfGmGmGmsUmsAm |
| RZ011313 | CmsAmsGmAmCmAmCfGfUfAfCmCmUmGmC mAmGmAmUm CR01008×3 | AmsUfsCmUmGmCfAmGmGfUmAmCmGmU fG(moe)UfCmUmGmsCmsAm |
| RZ011314 | AmsCmsAmCmGmUmAfCfCfUfGmCmAmGmA mUmCmUmAm CR01008×3 | UmsAfsGmAmUmCfUmGmCfAmGmGmUmA fC(moe)GfUmGmUmsCmsUm |
| RZ011315 | CmsUmsAmCmUmAmCfAfAfUfGmUmGmAmG mUmGmAmUm CR01008×3 | AmsUfsCmAmCmUfCmAmCfAmUmUmGmU fA(moe)GfUmAmGmsGmsGm |
| RZ011316 | CmsAmsAmUmGmUmGfAfGfUfGmAmUmGm AmGmAmUmCm CR01008×3 | GmsAfsUmCmUmCfAmUmCfAmCmUmCmA fC(moe)AfUmUmGmsUmsAm |

(continued)

| No. | Modified Sense Strand (5'-3') | Modified Antisense Strand (5'-3') |
|---|---|---|
| RZ011317 | GmsUmsGmAmUmGmAfGfAfUfCmUmCmUmUmUmCmCmAm CR01008×3 | UmsGfsGmAmAmAfGmAmGfAmUmCmUmCfA(moe)UfCmAmCmsUmsCm |
| RZ011318 | AmsGmsAmUmCmUmCfUfUfUfCmCmAmCmUmGmCmUmAm CR01008×3 | UmsAfsGmCmAmGfUmGmGfAmAmAmGmAfG(moe)AfUmCmUmsCmsAm |
| RZ011319 | CmsAmsGmAmCmAmGfCfGfAfUmCmUmGmUmGmAmCmAm CR01008×3 | UmsGfsUmCmAmCfAmGmAfUmCmGmCmUfG(moe)UfCmUmGmsCmsCm |
| RZ011320 | GmsCmsCmGmAmAmGfCfUfUfUmCmCmUmGmUmCmUmUm CR01008×3 | AmsAfsGmAmCmAfGmGmAfAmAmGmCmUfU(moe)CfGmGmCmsCmsAm |
| RZ011321 | CmsCmsUmGmGmUmGfCfUfAfGmAmUmGmGmAmUmCmAm CR01008×3 | UmsGfsAmUmCmCfAmUmCfUmAmGmCmAfC(moe)CfAmGmGmsUmsAm |
| RZ011322 | CmsUmsAmGmAmUmGfGfAfUfCmAmGmAmCmAmGmCmAm CR01008×3 | UmsGfsCmUmGmUfCmUmGfAmUmCmCmAfU(moe)CfUmAmGmsCmsAm |
| RZ011323 | CmsAmsAmCmUmUmCfAfCfAfGmGmAmGmCmCmAmAmAm CR01008×3 | UmsUfsUmGmGmCfUmCmCfUmGmUmGmAfA(moe)GfUmUmGmsCmsUm |
| RZ011324 | AmsAmsAmGmUmGmUfCfUfAfGmUmCmAmAmCmUmUmAm CR01008×3 | UmsAfsAmGmUmUfGmAmCfUmAmGmAmCfA(moe)CfUmUmUmsUmsUm |
| RZ011325 | AmsAmsGmUmGmUmCfUfAfGfUmCmAmAmCmUmUmAmAm CR01008×3 | UmsUfsAmAmGmUfUmGmAfCmUmAmGmAfC(moe)AfCmUmUmsUmsUm |
| RZ011326 | AmsGmsUmGmUmCmUfAfGfUfCmAmAmCmUmUmAmAmUm CR01008×3 | AmsUfsUmAmAmGfUmUmGfAmCmUmAmGfA(moe)CfAmCmUmsUmsUm |
| RZ011327 | CmsUmsAmGmUmCmAfAfCfUfUmAmAmUmUmGmAmGmAm CR01008×3 | UmsCfsUmCmAmAfUmUmAfAmGmUmUmGfA(moe)CfUmAmGmsAmsCm |
| RZ011328 | UmsUmsGmAmGmAmAfGfGfUfGmGmCmAmAmGmUmUmAm CR01008×3 | UmsAfsAmCmUmUfGmCmCfAmCmCmUmUfC(moe)UfCmAmAmsUmsUm |
| RZ011329 | GmsUmsGmGmCmAmAfGfUfUfAmUmGmGmUmGmUmGmAm CR01008×3 | UmsCfsAmCmAmCfCmAmUfAmAmCmUmUfG(moe)CfCmAmCmsCmsUm |

(continued)

| No. | Modified Sense Strand (5'-3') | Modified Antisense Strand (5'-3') |
|---|---|---|
| RZ011330 | AmsAmsGmAmUmAmUfGfGfUfCmUmAmGmUmGmAmCmAm CR01008×3 | UmsGfsUmCmAmCfUmAmGfAmCmCmAmUfA(moe)UfCmUmUmsGmsGm |
| RZ011331 | GmsAmsUmAmUmGmGfUfCfUfAmGmUmGmAmCmAmUmAm CR01008×3 | UmsAfsUmGmUmCfAmCmUfAmGmAmCmCfA(moe)UfAmUmCmsUmsUm |
| RZ011332 | CmsCmsAmAmAmAmUfUfUfGfGmGmUmCmAmAmAmGmUm CR01008×3 | AmsCfsUmUmUmGfAmCmCfCmAmAmAmUfU(moe)UfUmGmGmsGmsGm |
| RZ011333 | GmsCmsAmGmCmUmCfAfAfUfGmAmAmAmUmCmAmAmUm CR01008×3 | AmsUfsUmGmAmUfUmUmCfAmUmUmGmAfG(moe)CfUmGmCmsUmsUm |
| RZ011334 | GmsCmsUmCmAmAmUfGfAfAfAmUmCmAmAmUmUmAmUm CR01008×3 | AmsUfsAmAmUmUfGmAmUfUmUmCmAmUfU(moe)GfAmGmCmsUmsGm |
| RZ011335 | CmsAmsAmUmGmAmAfAfUfCfAmAmUmUmAmUmGmAmAm CR01008×3 | UmsUfsCmAmUmAfAmUmUfGmAmUmUmUfC(moe)AfUmUmGmsAmsGm |
| RZ011336 | GmsCmsCmAmUmGmUfCfAfUfCmAmUmCmCmUmCmAmUm CR01008×3 | AmsUfsGmAmGmGfAmUmGfAmUmGmAmCfA(moe)UfGmGmCmsGmsGm |
| RZ011337 | AmsGmsGmAmUmUmAfUfCfUfGmGmAmUmGmUmCmUmAm CR01008×3 | UmsAfsGmAmCmAfUmCmCfAmGmAmUmAfA(moe)UfCmCmUmsCmsCm |
| RZ011338 | GmsGmsAmUmUmAmUfCfUfGfGmAmUmGmUmCmUmAmUm CR01008×3 | AmsUfsAmGmAmCfAmUmCfCmAmGmAmUfA(moe)AfUmCmCmsUmsCm |
| RZ011339 | UmsCmsUmGmGmAmUfGfUfCfUmAmUmGmUmGmUmUmUm CR01008×3 | AmsAfsAmCmAmCfAmUmAfGmAmCmAmUfC(moe)CfAmGmAmsUmsAm |
| RZ011340 | AmsAmsGmUmGmAmAfCfAfUfCmAmAmUmGmCmUmUmUm CR01008×3 | AmsAfsAmGmCmAfUmUmGfAmUmGmUmUfC(moe)AfCmUmUmsGmsGm |
| RZ011341 | CmsAmsAmGmAmAmAfGfAfCfAmAmUmGmAmGmCmAmAm CR01008×3 | UmsUfsGmCmUmCfAmUmUfGmUmCmUmUfU(moe)CfUmUmGmsGmsAm |
| RZ011342 | CmsUmsCmUmGmAmGfUfCfUfCmUmGmUmGmGmCmAmUm CR01008×3 | AmsUfsGmCmCmAfCmAmGfAmGmAmCmUfC(moe)AfGmAmGmsAmsCm |

(continued)

| No. | Modified Sense Strand (5'-3') | Modified Antisense Strand (5'-3') |
|---|---|---|
| RZ011343 | GmsUmsUmUmCmAmCfUfGfUfGmGmAmUmGmAmCmAmAm CR01008×3 | UmsUfsGmUmCmAfUmCmCfAmCmAmGmUfG(moe)AfAmAmCmsAmsAm |
| RZ011344 | GmsAmsAmGmCmAmGfGfAfAfUmUmCmCmUmGmAmAmUm CR01008×3 | AmsUfsUmCmAmGfGmAmAfUmUmCmCmUfG(moe)CfUmUmCmsUmsUm |
| RZ011345 | CmsAmsGmGmAmAmUfUfCfCfUmGmAmAmUmUmUmUmAm CR01008×3 | UmsAfsAmAmAmUfUmCmAfGmGmAmAmUfU(moe)CfCmUmGmsCmsUm |
| RZ011346 | CmsCmsCmUmGmAmUfCfAfAfGmCmUmCmAmAmGmAmAm CR01008×3 | UmsUfsCmUmUmGfAmGmCfUmUmGmAmUfC(moe)AfGmGmGmsCmsAm |
| RZ011347 | UmsGmsAmUmCmAmAfGfCfUfCmAmAmGmAmAmUmAmAm CR01008×3 | UmsUfsAmUmUmCfUmUmGfAmGmCmUmUfG(moe)AfUmCmAmsGmsGm |
| RZ011348 | GmsCmsUmCmAmAmGfAfAfUfAmAmGmCmUmGmAmAmAm CR01008×3 | UmsUfsUmCmAmGfCmUmUfAmUmUmCmUfU(moe)GfAmGmCmsUmsUm |
| RZ011349 | CmsAmsGmGmCmUmAfUfGfAfCmAmAmAmGmUmCmAmAm CR01008×3 | UmsUfsGmAmCmUfUmUmGfUmCmAmUmAfG(moe)CfCmUmGmsGmsGm |
| RZ011350 | CmsUmsUmGmAmUmAfGfUfUfCmAmCmAmAmGmAmGmAm CR01008×3 | UmsCfsUmCmUmUfGmUmGfAmAmCmUmAfU(moe)CfAmAmGmsGmsGm |
| RZ011351 | CmsAmsAmGmAmGmAfAfGfUfCmGmUmUmUmCmAmUmUm CR01008×3 | AmsAfsUmGmAmAfAmCmGfAmCmUmUmCfU(moe)CfUmUmGmsUmsGm |
| RZ011352 | AmsGmsAmGmAmAmGfUfCfGfUmUmUmCmAmUmUmCmAm CR01008×3 | UmsGfsAmAmUmGfAmAmAfCmGmAmCmUfU(moe)CfUmCmUmsUmsGm |
| RZ011353 | GmsAmsGmAmAmGmUfCfGfUfUmUmCmAmUmUmCmAmAm CR01008×3 | UmsUfsGmAmAmUfGmAmAfAmCmGmAmCfU(moe)UfCmUmCmsUmsUm |
| RZ011354 | GmsAmsGmGmAmUmUfUfGfGfGmUmUmUmUmCmUmAmUm CR01008×3 | AmsUfsAmGmAmAfAmAmCfCmCmAmAmAfU(moe)CfCmUmCmsAmsUm |
| RZ011355 | CmsCmsUmUmUmAmUfCfUfUfGmGmGmCmCmUmCmUmUm CR01008×3 | AmsAfsGmAmGmGfCmCmCmAmAmGfAmUfA(moe)AfAmGmGmsGmsCm |

(continued)

| No. | Modified Sense Strand (5'-3') | Modified Antisense Strand (5'-3') |
|---|---|---|
| RZ011356 | AmsAmsGmGmCmGmGfCfUfCfCmUmUmCmC mGmAmCmUm CR01008×3 | AmsGfsUmCmGmGfAmAmGmGmAmGfCmC fG(moe)CfCmUmUmsUmsGm |
| RZ011357 | GmsCmsGmGmCmUmCfCfUfUfCmCmGmAmC mUmUmCmUm CR01008×3 | AmsGfsAmAmGmUfCmGmGmAmAmGfGmA fG(moe)CfCmGmCmsCmsUm |
| RZ011358 | GmsCmsUmCmCmUmUfCfCfGfAmCmUmUmC mUmCmCmAm CR01008×3 | UmsGfsGmAmGmAfAmGmUmCmGmGfAmA fG(moe)GfAmGmCmsCmsGm |
| RZ011359 | CmsCmsCmGmUmAmCfCfCfUfGmUmGmCmA mGmAmCmAm CR01008×3 | UmsGfsUmCmUmGfCmAmCmAmGmGfGmU fA(moe)CfGmGmGmsUmsAm |
| RZ011360 | CmsAmsGmAmCmAmCfGfUfAfCmCmUmGmC mAmGmAmUm CR01008×3 | AmsUfsCmUmGmCfAmGmGmUmAmCfGmU fG(moe)UfCmUmGmsCmsAm |
| RZ011361 | AmsCmsAmCmGmUmAfCfCfUfGmCmAmGmA mUmCmUmAm CR01008×3 | UmsAfsGmAmUmCfUmGmCmAmGmGfUmA fC(moe)GfUmGmUmsCmsUm |
| RZ011362 | CmsUmsAmCmUmAmCfAfAfUfGmUmGmAmG mUmGmAmUm CR01008×3 | AmsUfsCmAmCmUfCmAmCmAmUmUfGmU fA(moe)GfUmAmGmsGmsGm |
| RZ011363 | CmsAmsAmUmGmUmGfAfGfUfGmAmUmGm AmGmAmUmCm CR01008×3 | GmsAfsUmCmUmCfAmUmCmAmCmUfCmA fC(moe)AfUmUmGmsUmsAm |
| RZ011364 | GmsUmsGmAmUmGmAfGfAfUfCmUmCmUm UmUmCmCmAm CR01008×3 | UmsGfsGmAmAmAfGmAmGmAmUmCfUmC fA(moe)UfCmAmCmsUmsCm |
| RZ011365 | AmsGmsAmUmCmUmCfUfUfUfCmCmAmCmU mGmCmUmAm CR01008×3 | UmsAfsGmCmAmGfUmGmGmAmAmAfGmA fG(moe)AfUmCmUmsCmsAm |
| RZ011366 | CmsAmsGmAmCmAmGfCfGfAfUmCmUmGmU mGmAmCmAm CR01008×3 | UmsGfsUmCmAmCfAmGmAmUmCmGfCmU fG(moe)UfCmUmGmsCmsCm |
| RZ011367 | GmsCmsCmGmAmAmGfCfUfUfUmCmCmUmG mUmCmUmUm CR01008×3 | AmsAfsGmAmCmAfGmGmAmAmAmGfCmU fU(moe)CfGmGmCmsCmsAm |
| RZ011368 | CmsCmsUmGmGmUmGfCfUfAfGmAmUmGmG mAmUmCmAm CR01008×3 | UmsGfsAmUmCmCfAmUmCmUmAmGfCmA fC(moe)CfAmGmGmsUmsAm |

(continued)

| No. | Modified Sense Strand (5'-3') | Modified Antisense Strand (5'-3') |
|---|---|---|
| RZ011369 | CmsUmsAmGmAmUmGfGfAfUfCmAmGmAmCmAmGmCmAm CR01008×3 | UmsGfsCmUmGmUfCmUmGmAmUmCfCmAfU(moe)CfUmAmGmsCmsAm |
| RZ011370 | CmsAmsAmCmUmUmCfAfCfAfGmGmAmGmCmCmAmAmAm CR01008×3 | UmsUfsUmGmGmCfUmCmCmUmGmUfGmAfA(moe)GfUmUmGmsCmsUm |
| RZ011371 | AmsAmsAmGmUmGmUfCfUfAfGmUmCmAmAmCmUmUmAm CR01008×3 | UmsAfsAmGmUmUfGmAmCmUmAmGfAmCfA(moe)CfUmUmUmsUmsUm |
| RZ011372 | AmsAmsGmUmGmUmCfUfAfGfUmCmAmAmCmUmUmAmAm CR01008×3 | UmsUfsAmAmGmUfUmGmAmCmUmAfGmAfC(moe)AfCmUmUmsUmsUm |
| RZ011373 | AmsGmsUmGmUmCmUfAfGfUfCmAmAmCmUmUmAmAmUm CR01008×3 | AmsUfsUmAmAmGfUmUmGmAmCmUfAmGfA(moe)CfAmCmUmsUmsUm |
| RZ011374 | CmsUmsAmGmUmCmAfAfCfUfUmAmAmUmUmGmAmGmAm CR01008×3 | UmsCfsUmCmAmAfUmUmAmAmGmUfUmGfA(moe)CfUmAmGmsAmsCm |
| RZ011375 | UmsUmsGmAmGmAmAfGfGfUfGmGmCmAmAmGmUmUmAm CR01008×3 | UmsAfsAmCmUmUfGmCmCmAmCmCfUmUfC(moe)UfCmAmAmsUmsUm |
| RZ011376 | GmsUmsGmGmCmAmAfGfUfUfAmUmGmGmUmGmUmGmAm CR01008×3 | UmsCfsAmCmAmCfCmAmUmAmAmCfUmUfG(moe)CfCmAmCmsCmsUm |
| RZ011377 | AmsAmsGmAmUmAmUfGfGfUfCmUmAmGmUmGmAmCmAm CR01008×3 | UmsGfsUmCmAmCfUmAmGmAmCmCfAmUfA(moe)UfCmUmUmsGmsGm |
| RZ011378 | GmsAmsUmAmUmGmGfUfCfUfAmGmUmGmAmCmAmUmAm CR01008×3 | UmsAfsUmGmUmCfAmCmUmAmGmAfCmCfA(moe)UfAmUmCmsUmsUm |
| RZ011379 | CmsCmsAmAmAmAmUfUfUfGfGmGmUmCmAmAmAmGmUm CR01008×3 | AmsCfsUmUmUmGfAmCmCmCmAmAfAmUfU(moe)UfUmGmGmsGmsGm |
| RZ011380 | GmsCmsAmGmCmUmCfAfAfUfGmAmAmAmUmCmAmAmUm CR01008×3 | AmsUfsUmGmAmUfUmUmCmAmUmUfGmAfG(moe)CfUmGmCmsUmsUm |
| RZ011381 | GmsCmsUmCmAmAmUfGfAfAfAmUmCmAmAmUmUmAmUm CR01008×3 | AmsUfsAmAmUmUfGmAmUmUmUmCfAmUfU(moe)GfAmGmCmsUmsGm |

(continued)

| No. | Modified Sense Strand (5'-3') | Modified Antisense Strand (5'-3') |
|---|---|---|
| RZ011382 | CmsAmsAmUmGmAmAfAfUfCfAmAmUmUmAmUmGmAmAm CR01008×3 | UmsUfsCmAmUmAfAmUmUmGmAmUfUmUfC(moe)AfUmUmGmsAmsGm |
| RZ011383 | GmsCmsCmAmUmGmUfCfAfUfCmAmUmCmCmUmCmAmUm CR01008×3 | AmsUfsGmAmGmGfAmUmGmAmUmGfAmCfA(moe)UfGmGmCmsGmsGm |
| RZ011384 | AmsGmsGmAmUmUmAfUfCfUfGmGmAmUmGmUmCmUmAm CR01008×3 | UmsAfsGmAmCmAfUmCmCmAmGmAfUmAfA(moe)UfCmCmUmsCmsCm |
| RZ011385 | GmsGmsAmUmUmAmUfCfUfGfGmAmUmGmUmCmUmAmUm CR01008×3 | AmsUfsAmGmAmCfAmUmCmCmAmGfAmUfA(moe)AfUmCmCmsUmsCm |
| RZ011386 | UmsCmsUmGmGmAmUfGfUfCfUmAmUmGmUmGmUmUmUm CR01008×3 | AmsAfsAmCmAmCfAmUmAmGmAmCfAmUfC(moe)CfAmGmAmsUmsAm |
| RZ011387 | AmsAmsGmUmGmAmAfCfAfUfCmAmAmUmGmCmUmUmUm CR01008×3 | AmsAfsAmGmCmAfUmUmGmAmUmGfUmUfC(moe)AfCmUmUmsGmsGm |
| RZ011388 | CmsAmsAmGmAmAmAfGfAfCfAmAmUmGmAmGmCmAmAm CR01008×3 | UmsUfsGmCmUmCfAmUmUmGmUmCfUmUfU(moe)CfUmUmGmsGmsAm |
| RZ011389 | CmsUmsCmUmGmAmGfUfCfUfCmUmGmUmGmGmCmAmUm CR01008×3 | AmsUfsGmCmCmAfCmAmGmAmGmAfCmUfC(moe)AfGmAmGmsAmsCm |
| RZ011390 | GmsUmsUmUmCmAmCfUfGfUfGmGmAmUmGmAmCmAmAm CR01008×3 | UmsUfsGmUmCmAfUmCmCmAmCmAfGmUfG(moe)AfAmAmCmsAmsAm |
| RZ011391 | GmsAmsAmGmCmAmGfGfAfAfUmUmCmCmUmGmAmAmUm CR01008×3 | AmsUfsUmCmAmGfGmAmAmUmUmCfCmUfG(moe)CfUmUmCmsUmsUm |
| RZ011392 | CmsAmsGmGmAmAmUfUfCfCfUmGmAmAmUmUmUmUmAm CR01008×3 | UmsAfsAmAmAmUfUmCmAmGmGmAfAmUfU(moe)CfCmUmGmsCmsUm |
| RZ011393 | CmsCmsCmUmGmAmUfCfAfAfGmCmUmCmAmAmGmAmAm CR01008×3 | UmsUfsCmUmUmGfAmGmCmUmUmGfAmUfC(moe)AfGmGmGmsCmsAm |
| RZ011394 | UmsGmsAmUmCmAmAfGfCfUfCmAmAmGmAmAmUmAmAm CR01008×3 | UmsUfsAmUmUmCfUmUmGmAmGmCfUmUfG(moe)AfUmCmAmsGmsGm |

(continued)

| No. | Modified Sense Strand (5'-3') | Modified Antisense Strand (5'-3') |
|---|---|---|
| RZ011395 | GmsCmsUmCmAmAmGfAfAfUfAmAmGmCmUmGmAmAmAm CR01008×3 | UmsUfsUmCmAmGfCmUmUmAmUmUfCmUfU(moe)GfAmGmCmsUmsUm |
| RZ011396 | CmsAmsGmGmCmUmAfUfGfAfCmAmAmAmGmUmCmAmAm CR01008×3 | UmsUfsGmAmCmUfUmUmGmUmCmAfUmAfG(moe)CfCmUmGmsGmsGm |
| RZ011397 | CmsUmsUmGmAmUmAfGfUfUfCmAmCmAmAmGmAmGmAm CR01008×3 | UmsCfsUmCmUmUfGmUmGmAmAmCfUmAfU(moe)CfAmAmGmsGmsGm |
| RZ011398 | CmsAmsAmGmAmGmAfAfGfUfCmGmUmUmUmCmAmUmUm CR01008×3 | AmsAfsUmGmAmAfAmCmGmAmCmUfUmCfU(moe)CfUmUmGmsUmsGm |
| RZ011399 | AmsGmsAmGmAmAmGfUfCfGfUmUmUmCmAmUmUmCmAm CR01008×3 | UmsGfsAmAmUmGfAmAmAmCmGmAfCmUfU(moe)CfUmCmUmsUmsGm |
| RZ011400 | GmsAmsGmAmAmGmUfCfGfUfUmUmCmAmUmUmCmAmAm CR01008×3 | UmsUfsGmAmAmUfGmAmAmAmCmGfAmCfU(moe)UfCmUmCmsUmsUm |
| RZ011401 | GmsAmsGmGmAmUmUfUfGfGfGmUmUmUmUmCmUmAmUm CR01008×3 | AmsUfsAmGmAmAfAmAmCmCmCmAfAmAfU(moe)CfCmUmCmsAmsUm |
| RZ011402 | CmsCmsUmUmUmAmUfCfUfUfGmGmGmCmCmUmCmUmUm CR01008×3 | AmsAfsGmAmGmGfCmCmCfAmAmGmAmUfAmAfAmGmGmsGmsCm |
| RZ011403 | AmsAmsGmGmCmGmGfCfUfCfCmUmUmCmCmGmAmCmUm CR01008×3 | AmsGfsUmCmGmGfAmAmGfGmAmGmCmCfGmCfCmUmUmsUmsGm |
| RZ011404 | GmsCmsGmGmCmUmCfCfUfUfCmCmGmAmCmUmUmCmUm CR01008×3 | AmsGfsAmAmGmUfCmGmGfAmAmGmGmAfGmCfCmGmCmsCmsUm |
| RZ011405 | GmsCmsUmCmCmUmUfCfCfGfAmCmUmUmCmUmCmCmAm CR01008×3 | UmsGfsGmAmGmAfAmGmUfCmGmGmAmAfGmGfAmGmCmsCmsGm |
| RZ011406 | CmsCmsCmGmUmAmCfCfCfUfGmUmGmCmAmGmAmCmAm CR01008×3 | UmsGfsUmCmUmGfCmAmCfAmGmGmGmUfAmCfGmGmGmsUmsAm |
| RZ011407 | CmsAmsGmAmCmAmCfGfUfAfCmCmUmGmCmAmGmAmUm CR01008×3 | AmsUfsCmUmGmCfAmGmGfUmAmCmGmUfGmUfCmUmGmsCmsAm |

(continued)

| No. | Modified Sense Strand (5'-3') | Modified Antisense Strand (5'-3') |
|---|---|---|
| RZ011408 | AmsCmsAmCmGmUmAfCfCfUfGmCmAmGmA mUmCmUmAm CR01008×3 | UmsAfsGmAmUmCfUmGmCfAmGmGmUmA fCmGfUmGmUmsCmsUm |
| RZ011409 | CmsUmsAmCmUmAmCfAfAfUfGmUmGmAmG mUmGmAmUm CR01008×3 | AmsUfsCmAmCmUfCmAmCfAmUmUmGmU fAmGfUmAmGmsGmsGm |
| RZ011410 | CmsAmsAmUmGmUmGfAfGfUfGmAmUmGm AmGmAmUmCm CR01008×3 | GmsAfsUmCmUmCfAmUmCfAmCmUmCmA fCmAfUmUmGmsUmsAm |
| RZ011411 | GmsUmsGmAmUmGmAfGfAfUfCmUmCmUm UmUmCmCmAm CR01008×3 | UmsGfsGmAmAmAfGmAmGfAmUmCmUmC fAmUfCmAmCmsUmsCm |
| RZ011412 | AmsGmsAmUmCmUmCfUfUfUfCmCmAmCmU mGmCmUmAm CR01008×3 | UmsAfsGmCmAmGfUmGmGfAmAmAmGmA fGmAfUmCmUmsCmsAm |
| RZ011413 | CmsAmsGmAmCmAmGfCfGfAfUmCmUmGmU mGmAmCmAm CR01008×3 | UmsGfsUmCmAmCfAmGmAfUmCmGmCmU fGmUfCmUmGmsCmsCm |
| RZ011414 | GmsCmsCmGmAmAmGfCfUfUfUmCmCmUmG mUmCmUmUm CR01008×3 | AmsAfsGmAmCmAfGmGmAfAmAmGmCmU fUmCfGmGmCmsCmsAm |
| RZ011415 | CmsCmsUmGmGmUmGfCfUfAfGmAmUmGmG mAmUmCmAm CR01008×3 | UmsGfsAmUmCmCfAmUmCfUmAmGmCmA fCmCfAmGmGmsUmsAm |
| RZ011416 | CmsUmsAmGmAmUmGfGfAfUfCmAmGmAmC mAmGmCmAm CR01008×3 | UmsGfsCmUmGmUfCmUmGfAmUmCmCmA fUmCfUmAmGmsCmsAm |
| RZ011417 | CmsAmsAmCmUmUmCfAfCfAfGmGmAmGmC mCmAmAmAm CR01008×3 | UmsUfsUmGmGmCfUmCmCfUmGmUmGmA fAmGfUmUmGmsCmsUm |
| RZ011418 | AmsAmsAmGmUmGmUfCfUfAfGmUmCmAm AmCmUmUmAm CR01008×3 | UmsAfsAmGmUmUfGmAmCfUmAmGmAmC fAmCfUmUmUmsUmsUm |
| RZ011419 | AmsAmsGmUmGmUmCfUfAfGfUmCmAmAmC mUmUmAmAm CR01008×3 | UmsUfsAmAmGmUfUmGmAfCmUmAmGmA fCmAfCmUmUmsUmsUm |
| RZ011420 | AmsGmsUmGmUmCmUfAfGfUfCmAmAmCmU mUmAmAmUm CR01008×3 | AmsUfsUmAmAmGfUmUmGfAmCmUmAmG fAmCfAmCmUmsUmsUm |

(continued)

| No. | Modified Sense Strand (5'-3') | Modified Antisense Strand (5'-3') |
|---|---|---|
| RZ011421 | CmsUmsAmGmUmCmAfAfCfUfUmAmAmUmUmGmAmGmAm CR01008×3 | UmsCfsUmCmAmAfUmUmAfAmGmUmUmGfAmCfUmAmGmsAmsCm |
| RZ011422 | UmsUmsGmAmGmAmAfGfGfUfGmGmCmAmAmGmUmUmAm CR01008×3 | UmsAfsAmCmUmUfGmCmCfAmCmCmUmUfCmUfCmAmAmsUmsUm |
| RZ011423 | GmsUmsGmGmCmAmAfGfUfUfAmUmGmGmUmGmUmGmAm CR01008×3 | UmsCfsAmCmAmCfCmAmUfAmAmCmUmUfGmCfCmAmCmsCmsUm |
| RZ011424 | AmsAmsGmAmUmAmUfGfGfUfCmUmAmGmUmGmAmCmAm CR01008×3 | UmsGfsUmCmAmCfUmAmGfAmCmCmAmUfAmUfCmUmUmsGmsGm |
| RZ011425 | GmsAmsUmAmUmGmGfUfCfUfAmGmUmGmAmCmAmUmAm CR01008×3 | UmsAfsUmGmUmCfAmCmUfAmGmAmCmCfAmUfAmUmCmsUmsUm |
| RZ011426 | CmsCmsAmAmAmAmUfUfUfGfGmGmUmCmAmAmAmGmUm CR01008×3 | AmsCfsUmUmUmGfAmCmCfCmAmAmAmUfUmUfUmGmGmsGmsGm |
| RZ011427 | GmsCmsAmGmCmUmCfAfAfUfGmAmAmAmUmCmAmAmUm CR01008×3 | AmsUfsUmGmAmUfUmUmCfAmUmUmGmAfGmCfUmGmCmsUmsUm |
| RZ011428 | GmsCmsUmCmAmAmUfGfAfAfAmUmCmAmAmUmUmAmUm CR01008×3 | AmsUfsAmAmUmUfGmAmUfUmUmCmAmUfUmGfAmGmCmsUmsGm |
| RZ011429 | CmsAmsAmUmGmAmAfAfUfCfAmAmUmUmAmUmGmAmAm CR01008×3 | UmsUfsCmAmUmAfAmUmUfGmAmUmUmUfCmAfUmUmGmsAmsGm |
| RZ011430 | GmsCmsCmAmUmGmUfCfAfUfCmAmUmCmCmUmCmAmUm CR01008×3 | AmsUfsGmAmGmGfAmUmGfAmUmGmAmCfAmUfGmGmCmsGmsGm |
| RZ011431 | AmsGmsGmAmUmUmAfUfCfUfGmGmAmUmGmUmCmUmAm CR01008×3 | UmsAfsGmAmCmAfUmCmCfAmGmAmUmAfAmUfCmCmUmsCmsCm |
| RZ011432 | GmsGmsAmUmUmAmUfCfUfGfGmAmUmGmUmCmUmAmUm CR01008×3 | AmsUfsAmGmAmCfAmUmCfCmAmGmAmUfAmAfUmCmCmsUmsCm |
| RZ011433 | UmsCmsUmGmGmAmUfGfUfCfUmAmUmGmUmGmUmUmUm CR01008×3 | AmsAfsAmCmAmCfAmUmAfGmAmCmAmUfCmCfAmGmAmsUmsAm |

(continued)

| No. | Modified Sense Strand (5'-3') | Modified Antisense Strand (5'-3') |
|---|---|---|
| RZ011434 | AmsAmsGmUmGmAmAfCfAfUfCmAmAmUmGmCmUmUmUm CR01008×3 | AmsAfsAmGmCmAfUmUmGfAmUmGmUmUfCmAfCmUmUmsGmsGm |
| RZ011435 | CmsAmsAmGmAmAmAfGfAfCfAmAmUmGmAmGmCmAmAm CR01008×3 | UmsUfsGmCmUmCfAmUmUfGmUmCmUmUfUmCfUmUmGmsGmsAm |
| RZ011436 | CmsUmsCmUmGmAmGfUfCfUfCmUmGmUmGmGmCmAmUm CR01008×3 | AmsUfsGmCmCmAfCmAmGfAmGmAmCmUfCmAfGmAmGmsAmsCm |
| RZ011437 | GmsUmsUmUmCmAmCfUfGfUfGmGmAmUmGmAmCmAmAm CR01008×3 | UmsUfsGmUmCmAfUmCmCfAmCmAmGmUfGmAfAmAmCmsAmsAm |
| RZ011438 | GmsAmsAmGmCmAmGfGfAfAfUmUmCmCmUmGmAmAmUm CR01008×3 | AmsUfsUmCmAmGfGmAmAfUmUmCmCmUfGmCfUmUmCmsUmsUm |
| RZ011439 | CmsAmsGmGmAmAmUfUfCfCfUmGmAmAmUmUmUmUmAm CR01008×3 | UmsAfsAmAmAmUfUmCmAfGmGmAmAmUfUmCfCmUmGmsCmsUm |
| RZ011440 | CmsCmsCmUmGmAmUfCfAfAfGmCmUmCmAmAmGmAmAm CR01008×3 | UmsUfsCmUmUmGfAmGmCfUmUmGmAmUfCmAfGmGmGmsCmsAm |
| RZ011441 | UmsGmsAmUmCmAmAfGfCfUfCmAmAmGmAmAmUmAmAm CR01008×3 | UmsUfsAmUmUmCfUmUmGfAmGmCmUmUfGmAfUmCmAmsGmsGm |
| RZ011442 | GmsCmsUmCmAmAmGfAfAfUfAmAmGmCmUmGmAmAmAm CR01008×3 | UmsUfsUmCmAmGfCmUmUfAmUmUmCmUfUmGfAmGmCmsUmsUm |
| RZ011443 | CmsAmsGmGmCmUmAfUfGfAfCmAmAmAmGmUmCmAmAm CR01008×3 | UmsUfsGmAmCmUfUmUmGfUmCmAmUmAfGmCfCmUmGmsGmsGm |
| RZ011444 | CmsUmsUmGmAmUmAfGfUfUfCmAmCmAmAmGmAmGmAm CR01008×3 | UmsCfsUmCmUmUfGmUmGfAmAmCmUmAfUmCfAmAmGmsGmsGm |
| RZ011445 | CmsAmsAmGmAmGmAfAfGfUfCmGmUmUmUmCmAmUmUm CR01008×3 | AmsAfsUmGmAmAfAmCmGfAmCmUmUmCfUmCfUmUmGmsUmsGm |
| RZ011446 | AmsGmsAmGmAmAmGfUfCfGfUmUmUmCmAmUmUmCmAm CR01008×3 | UmsGfsAmAmUmGfAmAmAfCmGmAmCmUfUmCfUmCmUmsUmsGm |

(continued)

| No. | Modified Sense Strand (5'-3') | Modified Antisense Strand (5'-3') |
|---|---|---|
| RZ011447 | GmsAmsGmAmAmGmUfCfGfUfUmUmCmAm UmUmCmAmAm CR01008×3 | UmsUfsGmAmAmUfGmAmAfAmCmGmAmC fUmUfCmUmCmsUmsUm |
| RZ011448 | GmsAmsGmGmAmUmUfUfGfGfGmUmUmUm UmCmUmAmUm CR01008×3 | AmsUfsAmGmAmAfAmAmCfCmCmAmAmA fUmCfCmUmCmsAmsUm |

**Biological Detection Experiments**

Unless otherwise stated, all of the reagents and consumable materials (Table 4) and instrument and equipment (Table 5) used in the present disclosure are products commercially available from the following manufacturers.

Table 4 Main Reagents and Consumable Materials

| Name | Manufacturer |
|---|---|
| 1×PBS | M&C GENE TECHNOLOGY (BEIJING) LTD. |
| DMEM medium | M&C GENE TECHNOLOGY (BEIJING) LTD. |
| Opti-MEM™ medium | Gibco |
| Serum | Sigema |
| Pancreatin | M&C GENE TECHNOLOGY (BEIJING) LTD. |
| Double antibody | BBI |
| Lipofectamine RNAiMax | Invitrogen |
| Nucleic acid extraction or purification kit | Zhejiang Hanwei Science and Technology Co. Ltd. |
| RevertAid First Strand cDNA Synthesis Kit | Thermo Fisher Scientific |
| TaqMan Fast Advanced Master Mix | Thermo Fisher Scientific |
| Hanwei RNA extraction kit | Zhejiang Hanwei Science and Technology Co. Ltd. |
| HBSS | M&C GENE TECHNOLOGY (BEIJING) LTD. |
| EDTA | Solarbio |
| Type IV Collagenase | Sigma |
| RNALater | Thermo Fisher Scientific |

Table 5 Main Instrument and Equipment

| Name | Manufacturer |
|---|---|
| Full-automatic nucleic acid extractor | Zhejiang Hanwei Science and Technology Co. Ltd. |
| High-speed refrigerated centrifuge | Eppendorf |
| Carbon dioxide incubator | Thermo Fisher Scientific |
| Biological safety cabinet | Shanghai Lishen |
| Thermostatic water bath | Shanghai Boxun |
| Automated cell counter | Shanghai Countstar Inc. |
| Inverted Microscope | Olympus |
| NANODROP OneC | Thermo Fisher Scientific |
| Gradient PCR amplifier | Eppendorf |
| CFX Opus 384 | Bio-Rad |
| Tissuelyser II full-automatic tissue homogenizer | Shanghai Jingxin Industrial Development Co., Ltd. |

**Experiment 1 Evaluation of *In vitro* Activity of Double-Stranded RNAi Agents**

The present example evaluated the inhibitory activity of RZ011001 to RZ011255 with the identical target CFB, against the target gene CFB in cells by using the evaluation method for inhibitory activity against the target gene in human hepatocarcinoma cell line HepG2, with RZ000001 as the negative control.

Formulation of Tested Articles:

Each double-stranded RNAi agent tested article in the above was centrifuged, and then dissolved by adding an appropriate amount of PBS according to specification of each vial, to formulate 20 μM stock solution. The stock solution was then further subjected to gradient dilution with PBS into 1 μM working solution. Dose experiments were performed at final duplex concentration of 10 nM.

96-well Transfection and Detection:

HepG2 cells grown to near confluency were digested with trypsin, and the cells were washed to prepare a cell

suspension. 100 μL of the cell suspension was added to each well of the 96-well plate, with 12,000 cells per well, followed by culture at 37 °C in an incubator containing 5% $CO_2$. When the cells were adhered to walls for 24 h, DMEM medium in the 96-well plate was aspirated and discarded, 80 μL of Opti-MEM™ medium was added to each well, and then the 96-well plate was further cultured in the incubator. 1 μL of 1 μM working solution was dispersed in 9 μL of Opti-MEM to form siRNA mixture, 0.3 μL of RNAiMAX was dispersed in 9.7 μL of Opti-MEM, and well mixed with each siRNA mixture to form transfection complex. The transfection complex was incubated at room temperature for 10 minutes, and then the transfection complex was added to the 96-well plate, at 20 μL per well. After culture for 4 h, each well was supplemented with 100 μL of DMEM medium containing 20% FBS, and the 96-well plate was further cultured in the incubator for 24 h.

The 96-well plate was taken out, and total RNA was extracted using the full-automatic nucleic acid extractor (purchased from Zhejiang Hanwei Science and Technology Co. Ltd.) and the nucleic acid extraction kit (purchased from Zhejiang Hanwei Science and Technology Co. Ltd., GO-MNTR-100) according to standard procedures for total RNA extraction.

The reverse transcription kit (Thermo Fisher Scientific company, RevertAid First Strand cDNA Synthesis Kit, K1622) was used and Oligo $(dT)_{18}$ reverse transcription primer was selected, to formulate 20 μL of reverse transcription system according to the method described in the reverse transcription kit instruction and complete the reverse transcription reaction. Next, the expression level of the target gene mRNA in the HepG2 cells was detected on the fluorescent quantitative PCR instrument (Bio-Rad, CFX Opus 384) using the real-time fluorescent quantitative PCR kit (Thermo Fisher Scientific company, TaqMan Fast Advanced Master Mix, 4444557). In the real-time fluorescent quantitative PCR method, the glyceraldehyde-3-phosphate dehydrogenase (GAPDH) gene was used as the internal reference gene, and the target gene and the GAPDH internal reference gene were detected using primers for the target gene and primers for the GAPDH internal reference gene, respectively. Sequences of primers for detection were listed in Table 6.

Table 6 Sequences of Primers for Detection

| Gene | | Primer Type | Sequence of Primer | Fluorescent Group |
|---|---|---|---|---|
| Target Gene | CFB | upstream primer | 5'-GCTGTGAGAGAGATGCTCAA-3' (SEQ ID NO:511) | / |
| | | downstream primer | 5'-GACTCACTCCTCCAGTACAAAG-3' (SEQ ID NO:512) | / |
| | | probe primer | 5'-CCCAGGCTATGACAAAGTCAAGGACAT-3' (SEQ ID NO:513) | 5'FAM; 3'MGB |
| Internal Reference Gene | GAPDH | upstream primer | 5'-AAGAAGGTGGTGAAGCAGG-3' (SEQ ID NO:514) | / |
| | | downstream primer | 5'-CAAAGTGGTCGTTGAGGG-3' (SEQ ID NO:515) | / |
| | | probe primer | 5'-CAACAGCGACACCCACTC-3' (SEQ ID NO:516) | 5'VIC; 3'MGB |

In the real-time fluorescent quantitative PCR method, the relative quantitative calculation of the expression level and inhibitory rate of the target gene mRNA in various test groups was performed by the ΔΔCt method according to the technical method in the embodiments.

Table 7 Inhibitory Activity against Target Gene in HepG2 Cells after Administration of Double-Stranded RNAi Agents

| Group | 10 nM | |
|---|---|---|
| | % Residual Activity | STDEV |
| Mock | 100.00 | 4.64 |
| RZ000001 | 99.94 | 0.59 |
| RZ011001 | 17.94 | 2.11 |
| RZ011002 | 36.71 | 7.70 |
| RZ011003 | 35.89 | 0.53 |
| RZ011004 | 29.80 | 1.22 |

(continued)

| | % Residual Activity | STDEV |
|---|---|---|
| RZ011005 | 19.58 | 4.32 |
| RZ011006 | 18.39 | 0.52 |
| RZ011007 | 18.52 | 1.84 |
| RZ011008 | 24.16 | 2.94 |
| RZ011009 | 31.82 | 5.34 |
| RZ011010 | 33.48 | 3.44 |
| RZ011011 | 88.29 | 13.82 |
| RZ011012 | 24.67 | 0.03 |
| RZ011013 | 31.54 | 3.20 |
| RZ011014 | 25.93 | 1.96 |
| RZ011015 | 24.78 | 2.82 |
| RZ011016 | 48.35 | 5.84 |
| RZ011017 | 32.51 | 1.45 |
| RZ011018 | 16.76 | 1.46 |
| RZ011019 | 33.46 | 0.98 |
| RZ011020 | 62.58 | 5.44 |
| RZ011021 | 23.28 | 0.71 |
| RZ011022 | 18.94 | 0.56 |
| RZ011023 | 82.27 | 6.45 |
| RZ011024 | 48.02 | 0.52 |
| RZ011025 | 23.64 | 4.17 |
| RZ011026 | 30.25 | 7.19 |
| RZ011027 | 28.08 | 1.72 |
| RZ011028 | 26.55 | 1.15 |
| RZ011029 | 37.13 | 3.00 |
| RZ011030 | 84.36 | 10.64 |
| RZ011031 | 64.61 | 1.18 |
| RZ011032 | 23.35 | 1.51 |
| RZ011033 | 17.67 | 3.21 |
| RZ011034 | 19.20 | 1.90 |
| RZ011035 | 21.98 | 0.92 |
| RZ011036 | 24.77 | 2.42 |
| RZ011037 | 37.13 | 7.05 |
| RZ011038 | 22.39 | 3.10 |
| RZ011039 | 36.65 | 0.49 |
| RZ011040 | 48.59 | 4.31 |
| RZ011041 | 107.88 | 14.75 |
| RZ011042 | 76.95 | 7.16 |
| RZ011043 | 68.44 | 11.94 |

(continued)

| | % Residual Activity | STDEV |
|---|---|---|
| RZ011044 | 80.98 | 5.85 |
| RZ011045 | 106.68 | 5.99 |
| RZ011046 | 112.07 | 0.24 |
| RZ011047 | 107.98 | 11.31 |
| RZ011048 | 60.65 | 7.75 |
| RZ011049 | 25.89 | 1.62 |
| RZ011050 | 25.67 | 5.34 |
| RZ011051 | 78.83 | 4.33 |
| RZ011052 | 18.02 | 1.04 |
| RZ011053 | 70.00 | 11.81 |
| RZ011054 | 45.00 | 2.71 |
| RZ011055 | 38.61 | 1.05 |
| RZ011056 | 92.86 | 7.26 |
| RZ011057 | 27.41 | 2.83 |
| RZ011058 | 74.24 | 9.16 |
| RZ011059 | 106.49 | 3.54 |
| RZ011060 | 86.59 | 16.34 |
| RZ011061 | 103.07 | 1.72 |
| RZ011062 | 25.97 | 0.21 |
| RZ011063 | 89.24 | 3.31 |
| RZ011064 | 80.18 | 4.39 |
| RZ011065 | 111.61 | 3.04 |
| RZ011066 | 54.24 | 3.28 |
| RZ011067 | 38.63 | 1.36 |
| RZ011068 | 68.26 | 8.36 |
| RZ011069 | 36.31 | 0.68 |
| RZ011070 | 62.17 | 1.39 |
| RZ011071 | 76.45 | 2.59 |
| RZ011072 | 63.69 | 8.58 |
| RZ011073 | 29.01 | 0.54 |
| RZ011074 | 77.71 | 12.39 |
| RZ011075 | 42.18 | 4.46 |
| RZ011076 | 19.94 | 5.67 |
| RZ011077 | 62.00 | 6.54 |
| RZ011078 | 73.50 | 6.32 |
| RZ011079 | 38.51 | 8.07 |
| RZ011080 | 29.61 | 2.52 |
| RZ011081 | 28.21 | 0.51 |
| RZ011082 | 33.52 | 1.96 |

(continued)

| | % Residual Activity | STDEV |
|---|---|---|
| RZ011083 | 46.03 | 2.47 |
| RZ011084 | 19.57 | 0.53 |
| RZ011085 | 112.01 | 7.71 |
| RZ011086 | 55.73 | 6.12 |
| RZ011087 | 36.16 | 0.87 |
| RZ011088 | 39.07 | 1.32 |
| RZ011089 | 93.75 | 0.25 |
| RZ011090 | 40.17 | 3.50 |
| RZ011091 | 17.44 | 1.77 |
| RZ011092 | 61.56 | 4.45 |
| RZ011093 | 41.87 | 1.17 |
| RZ011094 | 88.56 | 12.70 |
| RZ011095 | 97.63 | 7.29 |
| RZ011096 | 51.49 | 0.27 |
| RZ011097 | 30.08 | 0.72 |
| RZ011098 | 19.57 | 0.90 |
| RZ011099 | 78.09 | 8.05 |
| RZ011100 | 80.57 | 3.43 |
| RZ011101 | 21.90 | 1.00 |
| RZ011102 | 19.13 | 0.20 |
| RZ011103 | 19.52 | 0.11 |
| RZ011104 | 21.82 | 0.93 |
| RZ011105 | 23.16 | 0.87 |
| RZ011106 | 20.16 | 0.71 |
| RZ011107 | 18.62 | 0.80 |
| RZ011108 | 25.17 | 2.48 |
| RZ011109 | 17.68 | 1.27 |
| RZ011110 | 57.10 | 0.87 |
| RZ011111 | 40.46 | 0.76 |
| RZ011112 | 28.21 | 3.69 |
| RZ011113 | 11.39 | 0.84 |
| RZ011114 | 16.87 | 2.89 |
| RZ011115 | 23.00 | 1.02 |
| RZ011116 | 41.69 | 0.50 |
| RZ011117 | 20.63 | 0.30 |
| RZ011118 | 74.46 | 3.46 |
| RZ011119 | 33.67 | 1.11 |
| RZ011120 | 41.88 | 2.98 |
| RZ011121 | 20.26 | 2.82 |

(continued)

| | % Residual Activity | STDEV |
|---|---|---|
| RZ011122 | 24.93 | 0.84 |
| RZ011123 | 11.12 | 0.26 |
| RZ011124 | 14.68 | 0.35 |
| RZ011125 | 16.29 | 3.90 |
| RZ011126 | 68.94 | 9.35 |
| RZ011127 | 22.42 | 2.29 |
| RZ011128 | 26.00 | 1.47 |
| RZ011129 | 81.32 | 6.74 |
| RZ011130 | 18.20 | 1.11 |
| RZ011131 | 43.06 | 2.04 |
| RZ011132 | 33.16 | 4.06 |
| RZ011133 | 20.09 | 0.73 |
| RZ011134 | 29.32 | 3.62 |
| RZ011135 | 51.36 | 0.06 |
| RZ011136 | 20.29 | 1.92 |
| RZ011137 | 86.51 | 10.42 |
| RZ011138 | 67.04 | 4.34 |
| RZ011139 | 27.83 | 2.68 |
| RZ011140 | 19.39 | 5.06 |
| RZ011141 | 61.55 | 0.71 |
| RZ011142 | 22.27 | 0.16 |
| RZ011143 | 64.35 | 1.43 |
| RZ011144 | 55.50 | 1.69 |
| RZ011145 | 19.65 | 0.23 |
| RZ011146 | 22.63 | 2.30 |
| RZ011147 | 28.12 | 1.10 |
| RZ011148 | 24.97 | 0.78 |
| RZ011149 | 48.71 | 7.25 |
| RZ011150 | 13.52 | 1.75 |
| RZ011151 | 16.07 | 0.12 |
| RZ011152 | 13.89 | 3.85 |
| RZ011153 | 23.06 | 2.68 |
| RZ011154 | 18.13 | 1.74 |
| RZ011155 | 14.04 | 2.25 |
| RZ011156 | 15.17 | 1.38 |
| RZ011157 | 19.80 | 4.05 |
| RZ011158 | 17.66 | 1.86 |
| RZ011159 | 24.50 | 1.05 |
| RZ011160 | 15.00 | 2.01 |

(continued)

| | % Residual Activity | STDEV |
|---|---|---|
| RZ011161 | 18.02 | 1.84 |
| RZ011162 | 12.59 | 0.66 |
| RZ011163 | 16.83 | 0.41 |
| RZ011164 | 22.02 | 0.28 |
| RZ011165 | 38.98 | 3.85 |
| RZ011166 | 23.18 | 1.68 |
| RZ011167 | 31.50 | 8.95 |
| RZ011168 | 26.86 | 2.74 |
| RZ011169 | 27.50 | 4.45 |
| RZ011170 | 18.30 | 1.23 |
| RZ011171 | 18.76 | 2.70 |
| RZ011172 | 17.26 | 0.19 |
| RZ011173 | 27.55 | 1.24 |
| RZ011174 | 66.41 | 1.99 |
| RZ011175 | 59.11 | 4.72 |
| RZ011176 | 70.50 | 8.37 |
| RZ011177 | 49.10 | 3.68 |
| RZ011178 | 74.43 | 6.32 |
| RZ011179 | 53.70 | 3.00 |
| RZ011180 | 43.18 | 2.93 |
| RZ011181 | 93.00 | 5.83 |
| RZ011182 | 54.32 | 4.05 |
| RZ011183 | 16.37 | 0.50 |
| RZ011184 | 22.86 | 0.08 |
| RZ011185 | 35.22 | 0.89 |
| RZ011186 | 26.53 | 2.01 |
| RZ011187 | 22.28 | 0.15 |
| RZ011188 | 64.51 | 2.76 |
| RZ011189 | 57.10 | 2.90 |
| RZ011190 | 61.60 | 6.92 |
| RZ011191 | 17.49 | 2.52 |
| RZ011192 | 19.20 | 1.10 |
| RZ011193 | 9.28 | 0.06 |
| RZ011194 | 12.39 | 0.17 |
| RZ011195 | 21.13 | 1.10 |
| RZ011196 | 19.38 | 2.34 |
| RZ011197 | 42.29 | 5.04 |
| RZ011198 | 61.73 | 8.87 |
| RZ011199 | 13.43 | 1.13 |

(continued)

| | % Residual Activity | STDEV |
|---|---|---|
| RZ011200 | 15.79 | 2.67 |
| RZ011201 | 11.16 | 0.24 |
| RZ011202 | 13.02 | 0.18 |
| RZ011203 | 29.51 | 2.72 |
| RZ011204 | 69.35 | 7.10 |
| RZ011205 | 41.80 | 1.14 |
| RZ011206 | 83.32 | 1.85 |
| RZ011207 | 23.56 | 2.14 |
| RZ011208 | 59.79 | 6.71 |
| RZ011209 | 30.71 | 4.78 |
| RZ011210 | 36.18 | 4.23 |
| RZ011211 | 40.19 | 4.41 |
| RZ011212 | 23.08 | 1.51 |
| RZ011213 | 43.93 | 1.63 |
| RZ011214 | 69.44 | 10.07 |
| RZ011215 | 23.93 | 3.20 |
| RZ011216 | 40.20 | 7.15 |
| RZ011217 | 22.56 | 7.68 |
| RZ011218 | 24.68 | 6.55 |
| RZ011219 | 74.81 | 9.79 |
| RZ011220 | 53.11 | 13.97 |
| RZ011221 | 35.62 | 6.53 |
| RZ011222 | 56.02 | 11.57 |
| RZ011223 | 92.12 | 3.14 |
| RZ011224 | 18.84 | 2.88 |
| RZ011225 | 18.05 | 2.02 |
| RZ011226 | 18.41 | 1.61 |
| RZ011227 | 20.57 | 5.89 |
| RZ011228 | 49.11 | 1.20 |
| RZ011229 | 21.35 | 2.07 |
| RZ011230 | 36.93 | 2.28 |
| RZ011231 | 23.09 | 2.32 |
| RZ011232 | 24.17 | 1.33 |
| RZ011233 | 16.70 | 0.40 |
| RZ011234 | 90.21 | 4.15 |
| RZ011235 | 20.18 | 1.22 |
| RZ011236 | 45.51 | 1.74 |
| RZ011237 | 30.25 | 3.00 |
| RZ011238 | 18.00 | 1.24 |

(continued)

| | % Residual Activity | STDEV |
|---|---|---|
| RZ011239 | 43.21 | 0.58 |
| RZ011240 | 30.08 | 3.27 |
| RZ011241 | 13.58 | 0.79 |
| RZ011242 | 12.78 | 1.00 |
| RZ011243 | 13.22 | 1.24 |
| RZ011244 | 18.59 | 0.02 |
| RZ011245 | 15.20 | 0.28 |
| RZ011246 | 15.84 | 1.61 |
| RZ011247 | 35.53 | 5.29 |
| RZ011248 | 24.55 | 1.70 |
| RZ011249 | 39.70 | 2.11 |
| RZ011250 | 22.76 | 6.59 |
| RZ011251 | 17.54 | 5.66 |
| RZ011252 | 17.48 | 3.65 |
| RZ011253 | 28.59 | 7.83 |
| RZ011254 | 24.71 | 5.43 |
| RZ011255 | 19.27 | 0.35 |

**Experiment 2 Evaluation of *in vivo* Activity of RNAi Agents in HDI Mice**

The present example evaluated the inhibitory activity of the double-stranded RNAi agents with the identical target CFB, conjugated to L96 ligand at the 3'-end of the sense strand, against the target gene CFB by using the Balb/c mouse hydrodynamic injection models, with RZ000001 as the negative control.

Plasmid construction: pcDNA-CMV-RG011 plasmid (ID: NM 001710.6), constructed by Sangon Biotech (Shanghai) Co., Ltd.

Construction of Mouse Models:

The Balb/c mouse hydrodynamic injection models were models established by rapidly injecting pcDNA-CMV-RG011 plasmid solution into mice through mouse tail vein under high pressure. On the $4^{th}$ day of experiment, the mice received hydrodynamic tail vein injection of 10 μg of pcDNA-CMV-RG011 within 5 seconds, at the injection volume of 8% of the mouse body weight. The plasmid DNA for injection was diluted in normal saline, and the solution was prepared before injection and stored at 4 °C.

Animal Grouping, Administration, and Tissue Sample Collection:

Balb/c mice aged 6-8 weeks were randomly divided into groups based on body weight (all females), 5 mice in each group. Each test group received a pre-determined dose of the drug conjugate, and the PBS control group was added. All mice received a single subcutaneous abdominal injection at a dose calculated according to the body weight. Each drug conjugate was administered as a PBS solution at 0.1 mg/mL (based on the double-stranded RNAi agent), at an administration volume of 10 mL/kg mouse body weight, that is, each drug conjugate was administered at a dose of 1 mg/kg mouse body weight (based on the double-stranded RNAi agent). The PBS control group was administered the same volume of PBS solution (without the drug conjugate). The day of administration was recorded as the $0^{th}$ Day (recorded as D0), plasmid injection was performed on the $3^{rd}$ day (recorded as D3) post-administration, and 5 mice per group were sacrificed on the $4^{th}$ day (recorded as D4). The sacrificed mice were subjected to gross anatomy, and liver tissue of each sacrificed mouse was collected, cut into a plurality of 2 $mm^3$ pieces, and stored in RNAlater.

For each mouse, an appropriate amount of liver tissue sample was taken from the RNAlater. The liver tissue sample was fragmented in the Tissuelyser II full-automatic tissue homogenizer for 60 seconds, and then total RNA was

extracted using the full-automatic nucleic acid extractor (purchased from Zhejiang Hanwei Science and Technology Co. Ltd.) and the nucleic acid extraction kit (purchased from Zhejiang Hanwei Science and Technology Co. Ltd., GO-MNTR-100) according to the standard procedures for total RNA extraction.

For each mouse, 1 μg of the total RNA was taken, the reverse transcription kit (Thermo Fisher Scientific company, RevertAid First Strand cDNA Synthesis Kit, K1622) was used and Oligo $(dT)_{18}$ reverse transcription primer was selected, to formulate 20 μL of reverse transcription system according to the method described in the reverse transcription kit instruction and complete the reverse transcription reaction. After the reaction was quenched, 60 μL of RNase-Free water was added into the reverse transcription system, to provide the cDNA solution. Next, the expression level of the target gene mRNA in the animals was detected on the fluorescent quantitative PCR instrument (Bio-Rad, CFX Opus 384) using the real-time fluorescent quantitative PCR kit (Thermo Fisher Scientific, TaqMan Fast Advanced Master Mix, 4444557). In the real-time fluorescent quantitative PCR method, Nero gene on plasmid backbone was used as the internal reference gene, and the target gene and the Nero internal reference gene were detected using the primers for the target gene and primers for the Nero internal reference gene, respectively. Sequences of the primers for detection were listed in Table 8.

Table 8 Sequences of Primers for Detection

| Gene | | Primer Type | Sequence of Primer | Fluorescent Group |
|---|---|---|---|---|
| Target Gene | CFB | upstream primer | 5'-GCTGTGAGAGAGATGCTCAA-3' (SEQ ID NO:511) | / |
| | | downstream primer | 5'-GACTCACTCCTCCAGTACAAAG-3' (SEQ ID NO:512) | / |
| | | Probe primer | 5'-CCCAGGCTATGACAAAGTCAAGGACAT-3' (SEQ ID NO:513) | 5'FAM; 3'MGB |
| Internal Reference Gene | Nero | upstream primer | 5'-CGTTGGCTACCCGTGATATT-3' (SEQ ID NO:517) | / |
| | | downstream primer | 5'-CTCGTCAAGAAGGCGATAGAAG-3' (SEQ ID NO:518) | / |
| | | Probe primer | 5'-CCGCTTCCTCGTGCTTTACGGTAT-3' (SEQ ID NO:519) | 5'VIC; 3'MGB |

10 μL of Real-time PCR reaction system was formulated in each PCR detection well according to the method described in the instruction of the real-time fluorescent quantitative PCR kit. Each reaction system contained 4 μL of the cDNA solution obtained by the above reverse transcription reaction, 5 μL of TaqMan™ Fast Advanced Master Mix (2×), 0.15 μL of 10 μM upstream primer, 0.15 μL of 10 μM downstream primer (see Table X for primer information), 0.15 μL of 10 μM probe primer, and 0.55 μL of RNase-Free $H_2O$. The formulated reaction system was subjected to Real-time PCR amplification on the real-time fluorescent quantitative PCR instrument (Bio-Rad, CFX Opus 384) by the two-step method, wherein the amplification procedure was 50 °C for 2 min, then pre-denaturation at 95 °C for 20 s, denaturation at 95 °C for 3 s, annealing at 60 °C, and extension for 30 s. Processes of denaturation, annealing, and extension were repeated for 40 cycles. In the real-time fluorescent quantitative PCR method, the relative quantitative calculation of the expression level and inhibitory rate of the target gene mRNA in various test groups was performed by the ΔΔCt method according to the technical method in the embodiments.

Table 9 Inhibitory Activity against Target Gene in Balb/c-HDI Mice after Administration of Double-Stranded RNAi Agents

| Group | 1 mg/kg | |
|---|---|---|
| | % Residual Activity | STDEV |
| PBS | 100.00 | 6.92 |
| RZ000001 | 130.62 | 17.75 |
| RZ011256 | 45.91 | 4.53 |
| RZ011257 | 119.76 | 14.46 |
| RZ011258 | 118.92 | 46.86 |

(continued)

| Group | 1 mg/kg | |
|---|---|---|
| | % Residual Activity | STDEV |
| RZ011259 | 131.11 | 43.57 |
| RZ011260 | 92.83 | 16.73 |
| RZ011261 | 99.52 | 31.42 |
| RZ011262 | 90.55 | 15.84 |
| RZ011263 | 25.93 | 7.61 |
| RZ011264 | 100.95 | 33.83 |
| RZ011265 | 41.11 | 9.94 |
| RZ011266 | 70.32 | 20.76 |
| RZ011267 | 97.73 | 19.23 |
| RZ011268 | 65.23 | 14.71 |
| RZ011269 | 122.25 | 61.59 |
| RZ011270 | 90.29 | 14.64 |
| RZ011271 | 135.14 | 24.42 |
| RZ011272 | 58.93 | 15.16 |
| RZ011273 | 63.11 | 13.64 |
| RZ011274 | 22.22 | 11.33 |
| RZ011275 | 44.95 | 10.57 |
| RZ011276 | 80.04 | 28.80 |
| RZ011277 | 74.99 | 15.75 |
| RZ011278 | 42.10 | 11.62 |
| RZ011279 | 43.77 | 11.12 |
| RZ011280 | 44.45 | 8.80 |
| RZ011281 | 9.92 | 2.24 |
| RZ011282 | 24.83 | 9.00 |
| RZ011283 | 24.34 | 10.69 |
| RZ011284 | 59.20 | 15.72 |
| RZ011285 | 20.78 | 8.44 |
| RZ011286 | 68.50 | 25.45 |
| RZ011287 | 42.15 | 11.22 |
| RZ011288 | 23.98 | 7.28 |
| RZ011289 | 72.90 | 21.91 |
| RZ011290 | 32.80 | 4.71 |
| RZ011291 | 41.16 | 10.48 |
| RZ011292 | 19.48 | 5.36 |
| RZ011293 | 20.27 | 2.93 |
| RZ011294 | 63.05 | 3.78 |
| RZ011295 | 34.81 | 18.25 |
| RZ011296 | 25.56 | 10.78 |

(continued)

| Group | 1 mg/kg | |
|---|---|---|
| | % Residual Activity | STDEV |
| RZ011297 | 77.92 | 25.89 |
| RZ011298 | 58.06 | 11.37 |
| RZ011299 | 19.04 | 7.07 |
| RZ011300 | 98.26 | 35.23 |
| RZ011301 | 12.32 | 5.07 |
| RZ011302 | 69.49 | 10.78 |
| RZ011303 | 19.34 | 5.68 |

The results in Table 9 above demonstrate that at the dose of 1 mg/kg, RZ011281, RZ011292, RZ011293, RZ011299, RZ011301, and RZ011303 can significantly inhibit the CFB mRNA expression, and the inhibitory effect was ≥80%.

**Experiment 3 Evaluation of Activity of Double-Stranded RNAi Agents in Primary Mouse Hepatocytes**

The present example evaluated the inhibitory activity of the double-stranded RNAi agents with the identical target CFB, conjugated to L96 ligand at the 3'-end of the sense strand, against the target gene CFB by using the primary C57BL/6J mouse hepatocytes, with RZ000001 as the negative control.

Isolation of primary mouse hepatocytes: Mice aged 6-8 weeks were anesthetized, and the mouse abdominal cavity was exposed to locate the hepatic portal veins and inferior vena cavas, which were perfused with pre-warmed (37 °C) HBSS-EDTA, followed by digestion with 0.08% type IV collagenase in HBSS containing $Ca^{2+}$ and $Mg^{2+}$. Digestion was terminated when the liver transitioned from an elastic, distended state to a slow-rebound state and exhibited visible texture. The perfused liver was taken out from the animal body, the cell suspension was filtered with a cell sieve to remove undigested tissue and connective tissue, and the cells were collected into the centrifuge tube. After the cell suspension was centrifuged, the culture medium was added for resuspension, followed by staining with trypan blue at final concentration of 0.04% for 2 min, thereby determining the cell viability.

Cell plating: After the cells were diluted to an appropriate density, the cells were plated in the 96-well culture plate at $2\times10^4$ cells/well, 100 μL/well.

Cell transfection: 1 μL of the diluted compound was added to the working concentration of 10 nM, and the culture plate was gently shaken back and forth to mix uniformly. The cell plate was further cultured at 37 °C in the incubator containing 5% $CO_2$ for 24 h.

The 96-well plate was taken out, and total RNA was extracted using the full-automatic nucleic acid extractor (purchased from Zhejiang Hanwei Science and Technology Co. Ltd.) and the nucleic acid extraction kit (purchased from Zhejiang Hanwei Science and Technology Co. Ltd., GO-MNTR-100) according to the standard procedures for total RNA extraction.

The reverse transcription kit (Thermo Fisher Scientific company, RevertAid First Strand cDNA Synthesis Kit, K1622) was used and Oligo (dT)18 reverse transcription primer was selected, to formulate 20 μL of reverse transcription system according to the method described in the reverse transcription kit instruction and complete the reverse transcription reaction. Next, the expression level of the target gene mRNA in the primary mouse hepatocytes was detected on the fluorescent quantitative PCR instrument (Bio-Rad, CFX Opus 384) using the real-time fluorescent quantitative PCR kit (Thermo Fisher Scientific company, TaqMan Fast Advanced Master Mix, 4444557). In the real-time fluorescent quantitative PCR method, the glyceraldehyde-3-phosphate dehydrogenase (GAPDH) gene was used as the internal reference gene, and the target gene and the GAPDH internal reference gene were detected using primers for the target gene and primers for the GAPDH internal reference gene, respectively. Sequences of primers for detection were listed in Table 10.

Table 10 Sequences of Primers for Detection

| Gene | | Primer Type | Sequences of Primer | Fluorescent Group |
|---|---|---|---|---|
| Target Gene | CFB | upstream primer | 5'-GCCACGATATGGTCTCCTGA-3' (SEQ ID NO:520) | / |
| | | downstream primer | 5'-GAGCTTCTCTGTGACCCAGT-3' (SEQ ID NO:521) | / |
| | | probe primer | 5'-CGGCATCGCTACTCCTCTCATCAGA-3' (SEQ ID NO:522) | 5'FAM; 3'MGB |
| Internal Reference Gene | GAPDH | upstream primer | 5'-TATGACTCCACTCACGGCAA-3' (SEQ ID NO:523) | / |
| | | downstream primer | 5'-TGGAAGATGGTGATGGGCTT-3' (SEQ ID NO:524) | / |
| | | probe primer | 5'-TCTCGGCCTTGACTGTGCCGT-3' (SEQ ID NO:525) | 5'VIC; 3'MGB |

10 μL of Real-time PCR reaction system was formulated in each PCR detection well according to the method described in the instruction of the real-time fluorescent quantitative PCR kit. Each reaction system contained 4 μL of the cDNA solution obtained by the above reverse transcription reaction, 5 μL of TaqMan™ Fast Advanced Master Mix (2×), 0.15 μL of 10 μM upstream primer, 0.15 μL of 10 μM downstream primer (see Table X for primer information), 0.15 μL of 10 μM probe primer, and 0.55 μL of RNase-Free $H_2O$. The formulated reaction system was subjected to Real-time PCR amplification on the real-time fluorescent quantitative PCR instrument (Bio-Rad, CFX Opus 384) by the two-step method, wherein the amplification procedure was 50 °C for 2 min, then pre-denaturation at 95 °C for 20 s, denaturation at 95 °C for 3 s, annealing at 60 °C, and extension for 30 s. Processes of denaturation, annealing, and extension were repeated for 40 cycles. In the real-time fluorescent quantitative PCR method, the relative quantitative calculation of the expression level and inhibitory rate of the target gene mRNA in various test groups was performed by the ΔΔCt method according to the technical method in the embodiments.

Table 11 Inhibitory Activity against Target Gene in Primary Mouse Hepatocytes after Administration of Double-Stranded RNAi Agents

| Group | 10 nM | |
|---|---|---|
| | % Residual Activity | STDEV |
| Mock | 100.00 | 6.31 |
| RZ000001 | 96.85 | 1.30 |
| RZM11001 | 5.41 | 0.40 |
| RZM11002 | 5.71 | 0.86 |
| RZM11003 | 24.04 | 1.32 |
| RZM11004 | 89.20 | 4.50 |
| RZM11005 | 35.50 | 0.91 |
| RZM11006 | 34.55 | 3.83 |
| RZM11007 | 76.43 | 1.57 |
| RZM11008 | 11.04 | 2.21 |
| RZM11009 | 45.97 | 1.87 |
| RZM11010 | 88.43 | 10.14 |
| RZM11011 | 17.93 | 3.32 |
| RZM11012 | 70.25 | 2.08 |
| RZM11013 | 36.06 | 1.64 |

(continued)

| Group | 10 nM | |
|---|---|---|
| | % Residual Activity | STDEV |
| RZM11014 | 2.65 | 0.02 |
| RZM11015 | 4.25 | 0.05 |
| RZM11016 | 2.75 | 0.24 |
| RZM11017 | 17.62 | 0.03 |
| RZM11018 | 6.28 | 0.05 |
| RZM11019 | 36.47 | 0.06 |
| RZM11020 | 9.73 | 0.51 |
| RZM11021 | 15.19 | 1.52 |
| RZM11022 | 35.78 | 4.95 |
| RZM11023 | 8.19 | 0.08 |
| RZM11024 | 54.93 | 0.60 |
| RZM11025 | 6.78 | 0.03 |
| RZM11026 | 12.21 | 1.41 |
| RZM11027 | 10.55 | 0.18 |
| RZM11028 | 33.50 | 0.42 |
| RZM11029 | 31.04 | 3.12 |
| RZM11030 | 32.48 | 0.41 |
| RZM11031 | 44.19 | 0.26 |
| RZM11032 | 14.50 | 0.50 |
| RZM11033 | 16.17 | 0.65 |
| RZM11034 | 91.86 | 14.03 |
| RZM11035 | 20.22 | 0.26 |
| RZM11036 | 13.01 | 0.89 |
| RZM11037 | 23.98 | 2.37 |
| RZM11038 | 5.46 | 0.23 |
| RZM11039 | 3.37 | 0.50 |
| RZ011003 | 66.72 | 1.59 |
| RZ011095 | 85.58 | 2.00 |
| RZ011096 | 81.92 | 3.10 |
| RZ011097 | 25.44 | 2.96 |
| RZ011098 | 24.01 | 1.64 |
| RZ011171 | 21.65 | 2.63 |
| RZ011172 | 46.40 | 0.46 |
| RZ011254 | 15.77 | 2.44 |
| RZ011255 | 3.96 | 1.86 |

The results in Table 11 and FIG. 1 demonstrate that at the dose of 10 nM, multiple L96 vector conjugates can significantly inhibit the CFB mRNA expression in the primary mouse hepatocytes.

**Experiment 4 Evaluation of RNAi Agents *in vivo* Activity in C57BL/6J Mice**

The present example evaluated the mRNA inhibitory activity of the RNAi agents with L96 ligand in the liver tissue of C57BL/6J mice, with RZ000001 as the negative control.

Animal Grouping, Administration, and Tissue Sample Collection:

C57BL/6J male mice aged 6-8 weeks (Zhejiang Vital River Laboratory Animal Technology Co., Ltd.) were randomly grouped according to the body weight, 5 mice in each group. Each test group received a pre-determined dose of the drug conjugate, and the PBS control group was added. All mice were administered at a dose calculated according to the body weight. The administration volume was 10 mL/kg mouse body weight. The mice received a single subcutaneous abdominal injection. Each drug conjugate was administered as a PBS solution at 0.3 mg/mL (based on siRNA), that is, each drug conjugate was administered at a dose of 3 mg/kg mouse body weight (based on siRNA). The PBS control group was administered the same volume of PBS solution (without the drug conjugate).

The day of administration was recorded as the $0^{th}$ Day (recorded as D0), and the mice in all groups were sacrificed on the $7^{th}$ day (recorded as D7) after the administration. The sacrificed mice were subjected to gross anatomy, and liver tissue of each sacrificed mouse was collected, cut into a plurality of 2 $mm^3$ pieces, and stored in RNAlater for measurement of the CFB mRNA expression level. In the real-time fluorescent quantitative PCR method, the GAPDH gene was used as the internal reference gene, and the target gene and the GAPDH internal reference gene were detected using the primers for the target gene and primers for the GAPDH internal reference gene, respectively. Sequences of the primers for detection were listed in Table 10.

In the real-time fluorescent quantitative PCR method, the relative quantitative calculation of the expression level and inhibitory rate of the target gene mRNA in various test groups was performed by the $\Delta\Delta$Ct method according to the technical method in the embodiments.

Table 12 Inhibitory Activity against Target Gene in C57BL/6J Mice after Administration of Double-Stranded RNAi Agents

| Group | 3 mg/kg | |
|---|---|---|
| | % Residual Activity | STDEV |
| PBS | 100.00 | 3.43 |
| RZ000001 | 99.73 | 8.92 |
| RZM11014 | 17.08 | 2.88 |
| RZM11015 | 23.46 | 4.46 |
| RZM11016 | 15.53 | 3.51 |
| RZM11025 | 26.64 | 7.56 |
| RZM11039 | 8.85 | 1.24 |
| RZ011097 | 58.92 | 20.26 |
| RZ011098 | 37.94 | 6.02 |
| RZ011171 | 15.84 | 2.98 |
| RZ011254 | 69.64 | 7.77 |
| RZ011255 | 10.54 | 1.21 |

The results in Table 12 and FIG. 2 demonstrate that at the single dose of 3 mg/kg, RZM11014, RZM11016, RZM11039, RZ011171, and RZ011255 could significantly reduce the mRNA expression level in the liver tissue of C57BL/6J mice, and the inhibitory effect was greater than 80%.

**Experiment 5 Evaluation of *in vivo* Activity of Double-Stranded RNAi Agents with Different Ligands in C57BL/6J Mice**

The present example evaluated the mRNA inhibitory activity of the double-stranded RNAi agents with different ligands-RZ011304, RZ011305, RZ011306, RZ011307, and RZ011255-in liver tissue of C57BL/6J mice.

Animal Grouping, Administration, and Tissue Sample Collection:

C57BL/6J male mice aged 6-8 weeks (Zhejiang Vital River Laboratory Animal Technology Co., Ltd.) were randomly grouped according to the body weight, 5 mice in each group. Each test group received a pre-determined dose of the drug conjugate, and the PBS control group was added. All mice were administered at a dose calculated according to the body weight. The administration volume was 10 mL/kg mouse body weight. The mice received a single subcutaneous abdominal injection. Each drug conjugate was administered as a PBS solution at 0.3 mg/mL (based on siRNA), that is, each drug conjugate was administered at a dose of 3 mg/kg mouse body weight (based on siRNA). The PBS control group was administered the same volume of PBS solution (without the drug conjugate).

The day of administration was recorded as the $0^{th}$ Day (recorded as D0), and the mice in all groups were sacrificed on the $7^{th}$ day (recorded as D7) after the administration. The sacrificed mice were subjected to gross anatomy, and liver tissue of each sacrificed mouse was collected, cut into a plurality of 2 $mm^3$ pieces, and stored in RNAlater for measurement of the expression level of CFB mRNA. In the real-time fluorescent quantitative PCR method, the GAPDH gene was used as the internal reference gene, and the target gene and the GAPDH internal reference gene were detected using the primers for the target gene and primers for the GAPDH internal reference gene, respectively. Sequences of the primers for detection were listed in Table 10.

In the real-time fluorescent quantitative PCR method, the relative quantitative calculation of the expression level and inhibitory rate of the target gene mRNA in various test groups was performed by the ΔΔCt method according to the technical method in the embodiments.

Table 13 Inhibitory Activity against Target Gene in C57BL/6J Mice after Administration of Double-Stranded RNAi Agents with Different Ligands

| Group | Day 7 | |
|---|---|---|
| | % Residual Activity | STDEV |
| PBS | 100.00 | 8.87 |
| RZ011255 | 13.44 | 1.48 |
| RZ011304 | 7.88 | 1.69 |
| RZ011305 | 6.76 | 1.95 |
| RZ011306 | 11.85 | 3.44 |
| RZ011307 | 9.21 | 1.42 |

The results in Table 13 and FIG. 3 demonstrate that at the single dose of 3 mg/kg, RZ011304, RZ011305, RZ011306, and RZ011307 could significantly reduce the mRNA expression level in the liver tissue of C57BL/6J mice, indicating that the activity of RZ01 1304, RZ011305, RZ011306, and RZ011307 is superior to that of RZ011255.

Finally, it should be noted that various embodiments in the above are merely used for illustrating the technical solutions of the present disclosure, rather than limiting the present disclosure. Although the detailed description has been made to the present disclosure with various preceding embodiments, those ordinarily skilled in the art should understand that they still could modify the technical solutions described in various preceding embodiments, or make equivalent substitutions to some or all of the technical features therein. These modifications or substitutions do not make corresponding technical solutions essentially depart from the scope of technical solutions of various embodiments of the present disclosure.

## INDUSTRIAL APPLICABILITY

The present disclosure provides a complement factor B (CFB) inhibitor, wherein the inhibitor comprises a double-stranded oligonucleotide, a double-stranded RNAi agent and a pharmaceutical composition comprising the same. The present disclosure provides a method for treating, preventing, or alleviating a disease associated with dysregulation of a complement alternative pathway in a subject by administering the complement factor B (CFB) inhibitor. The present disclosure further provides a method for inhibiting CFB expression by administering a CFB specific inhibitor (double-stranded RNAi agent) to a subject.

## Claims

1. A double-stranded oligonucleotide (dsRNA) for inhibiting complement factor B (CFB) gene expression, wherein the double-stranded oligonucleotide comprises a sense strand and an antisense strand, wherein the sense strand comprises at least 15 contiguous nucleotides in any one of sequences as set forth in SEQ ID NOs: 1-255 in Table 1 or a nucleotide sequence which differs from the at least 15 contiguous nucleotides by no more than 3 nucleotides; and/or, the antisense strand comprises at least 15 contiguous nucleotides in any one of sequences as set forth in SEQ ID NOs: 256-510 in Table 1 or a nucleotide sequence which differs from the at least 15 contiguous nucleotides by no more than 3 nucleotides;

   preferably, the sense strand comprises a nucleotide sequence which differs from any one of sequences as set forth in SEQ ID NOs: 1-255 in Table 1 by no more than 3 nucleotides, and/or the antisense strand comprises a nucleotide sequence which differs from any one of sequences as set forth in SEQ ID NOs: 256-510 in Table 1 by no more than 3 nucleotides,
   wherein the antisense strand and the sense strand are complementary or substantially complementary, wherein the substantially complementary means that there are no more than three-nucleotide mismatches between the sense strand and the antisense strand within the double-stranded region.

2. The double-stranded oligonucleotide according to claim 1, wherein the sense strand comprises a nucleotide sequence which differs from any one of sequences as set forth in SEQ ID NOs: 1-255 in Table 1 by no more than 1 or 2 nucleotides, and/or the antisense strand comprises a nucleotide sequence which differs from any one of sequences as set forth in SEQ ID NOs: 256-510 in Table 1 by no more than 1 or 2 nucleotides;

   preferably, the sense strand comprises a nucleotide sequence which differs from any one of sequences as set forth in SEQ ID NOs: 1-255 in Table 1 by no more than 1 nucleotide, and/or the antisense strand comprises a nucleotide sequence which differs from any one of sequences as set forth in SEQ ID NOs: 256-510 in Table 1 by no more than 1 nucleotide; and
   further preferably, the sense strand comprises any one of nucleotide sequences as set forth in SEQ ID NOs: 1-255 in Table 1, and/or the antisense strand comprises any one of nucleotide sequences as set forth in SEQ ID NOs: 256-510 in Table 1.

3. The double-stranded oligonucleotide according to any one of claims 1-2, wherein the sense strand or the antisense strand comprises a nucleotide sequence of any sense strand or antisense strand selected from the group consisting of duplexes represented by the following: RN011001, RN011005, RN011006, RN011007, RN011018, RN011021, RN011022, RN011033, RN011034, RN011035, RN011038, RN011052, RN011076, RN011097, RN011098, RN011101, RN011102, RN011103, RN011104, RN011106, RN011107, RN011109, RN011113, RN011114, RN011117, RN011123, RN011124, RN011125, RN011140, RN01115, RN011151, RN011152, RN011156, RN011160, RN011171, RN011183, RN011193, RN011194, RN011199, RN011201, RN011202, RN011233, RN011238, RN011241, RN011242, RN011243, RN011254, and RN011255; and
   preferably, the sense strand or the antisense strand comprises a nucleotide sequence of the sense strand or antisense strand of the duplex represented by RN011255.

4. The double-stranded oligonucleotide according to any one of claims 1-2, wherein the double-stranded oligonucleotide comprises one or more selected from the group consisting of duplexes represented by the following: RN011001, RN011005, RN011006, RN011007, RN011018, RN011021, RN011022, RN011033, RN011034, RN011035, RN011038, RN011052, RN011076, RN011097, RN011098, RN011101, RN011102, RN011103, RN011104, RN011106, RN011107, RN011109, RN011113, RN011114, RN011117, RN011123, RN011124, RN011125, RN011140, RN01115, RN011151, RN011152, RN011156, RN011160, RN011171, RN011183, RN011193, RN011194, RN011199, RN011201, RN011202, RN011233, RN011238, RN011241, RN011242, RN011243, RN011254, and RN011255; and
   preferably, the double-stranded oligonucleotide comprises nucleotide sequences of the duplex represented by RN011255.

5. The double-stranded oligonucleotide according to any one of claims 1-4, wherein all nucleotides of the sense strand and all nucleotides of the antisense strand are modified nucleotides;

   optionally, the antisense strand and the sense strand are 17-25 nucleotides in length; and
   optionally, at least one of the antisense strand and the sense strand comprises a 3'-overhang of 1-2 nucleotides.

**6.** The double-stranded oligonucleotide according to claim 5, wherein the modified nucleotides are selected from one or more of 3'-terminal deoxy-thymine (dT) nucleotides, 2'-O-methyl-modified nucleotides, 2'-fluoro-modified nucleotides, 2'-deoxy-modified nucleotides, 2'-O-methoxyethyl-modified nucleotides, and 2'-O$(CH_2)_n$OR substituted nucleotides, wherein n is 1 or 2, and R is selected from substituted or unsubstituted $C_1$-$C_6$ alkyl or substituted or unsubstituted $C_1$-$C_6$ alkoxy, wherein the substituent is selected from the group consisting of halogen, $C_1$-$C_6$ alkyl, $C_1$-$C_6$ alkoxy, hydroxyl, and amino;

preferably, the modified nucleotides are selected from one or more of 2'-O-methyl-modified nucleotides, 2'-fluoro-modified nucleotides, 2'-O-methoxyethyl-modified nucleotides, and 2'-O$(CH_2)_n$OR substituted nucleotides; and further preferably, the sense strand of the double-stranded oligonucleotide comprises any modified nucleotide sequence in the sense strand as listed in Table 2 or Table 3, and/or the antisense strand comprises any modified nucleotide sequence in the antisense strand as listed in Table 2 or Table 3.

**7.** A double-stranded RNAi agent for inhibiting complement factor B (CFB) gene expression, wherein the double-stranded RNAi agent comprises a sense strand and an antisense strand, wherein the sense strand and the antisense strand are complementary or substantially complementary to form a double-stranded region, wherein the substantially complementary means that there are no more than three-nucleotide mismatches between the sense strand and the antisense strand within the double-stranded region, and the double-stranded region is represented by Formula (I) as follows:

SS: 5'- $(N)_{a'}$ - $(X)_{p'}$ - $(N)_{b'}$ - $(X)_{q'}$ - $(N)_{c'}$ - $(X)_{r'}$ - $(N)_{d'}$ -3' AS: 3' - $(N)_a$ - $(X)_p$ - $(N)_b$ - $(X)_q$ - $(N)_c$ -5' (I),

wherein SS represents the sense strand, and AS represents the antisense strand;
all nucleotides of the sense strand and the antisense strand are modified nucleotides;
each N independently represents a modified nucleotide selected from the group consisting of a 2'-O-methyl-modified nucleotide, a 2'-fluoro-modified nucleotide, and a 2'-deoxy-modified nucleotide;
each X independently represents a 2'-O-methoxyethyl-modified nucleotide, a 2'-O-methyl-modified nucleotide, or a 2'-O$(CH_2)_n$OR substituent-modified nucleotide, wherein n is 1 or 2, and R is selected from substituted or unsubstituted $C_1$-$C_6$ alkyl or substituted or unsubstituted $C_1$-$C_6$ alkoxy, wherein the substituent is selected from the group consisting of halogen, $C_1$-$C_6$ alkyl, $C_1$-$C_6$ alkoxy, hydroxyl, and amino;
the a, a', p, p', b, b', q, q', c, c', r', and d' each independently represent the number of nucleotides, wherein a' is an integer selected from 3 to 8; p' is an integer selected from 0 to 3; b' is an integer selected from 4 to 13; q' is an integer selected from 0 to 4; c' is an integer selected from 3 to 9; r' is an integer selected from 0 to 3; d' is an integer selected from 0 to 9; a is an integer selected from 4 to 7; p is 0 or 1; b is an integer selected from 4 to 8; q is an integer selected from 0 to 4; and c is an integer selected from 6 to 10; and
p', q', r', p, and q are not simultaneously 0, and $0 \leq q' + r' \leq 4$.

**8.** The double-stranded RNAi agent according to claim 7, wherein a' is an integer selected from 3 to 8; p' is 0 or 1; b' is an integer selected from 4 to 13; q' is 0 or 1; c' is an integer selected from 3 to 9; r' is 0 or 1; d' is an integer selected from 1 to 8; a is an integer selected from 4 to 7; p is 1; b is an integer selected from 4 to 8; q is 0 or 1; and c is an integer selected from 6 to 10;

N is selected from a 2'-O-methyl-modified nucleotide or a 2'-fluoro-modified nucleotide, and the nucleotide X is a 2'-O-methoxyethyl-modified nucleotide;
preferably, the duplex comprises at least one 2'-O-methoxyethyl-modified nucleotide or 2'-O$(CH_2)_n$OR-modified nucleotide;
further preferably, the double-stranded RNAi agent comprises a duplex consisting of following modified sense strands and antisense strands, in a direction from 5'-end to 3'-end:

the sense strand is: GmsGmsAmUmUmUmGfGfGfUfUmUmUmCmUmAmUmAmAm, or
GmsGmsAmUmUmUmGfGfGfUfUmUmUmCmUmAmUmAmsAm;
the antisense strand is: UmsUfsAmUmAmGfAmAmAfAmCmCmCmAfAmAfUmCmCmsUmsCm, or
UmsUfsAmUmAmGfAmAmAmAmCmCfCmAfAmAfUmCmCmsUmsCm, or
UmsUfsAmUmAmGfAmAmAfAmCmCmCmAfA(moe)AfUmCmCmsUmsCm, or
UmsUfsAmUmAmGfAmAmAmAmCmCfCmAfA(moe)AfUmCmCmsUmsCm, or

UmsUfsAmUmAmGfAmAmAfAmsCmCmCmAfA(moe)AfUmCmCmsUmsCm.

**9.** The double-stranded RNAi agent according to any one of claims 7-8, wherein all the nucleotides of the sense strand and/or the antisense strand of the double-stranded RNAi are modified nucleotides,

wherein in a direction from 5'-end to 3'-end, at least three of nucleotides at positions 7-10 of a nucleotide sequence in the sense strand are fluoro-modified nucleotides, and nucleotides at remaining positions are 2'-O-methyl-modified nucleotides or 2'-O-methoxyethyl-modified nucleotides;
optionally, in the direction from the 5'-end to the 3'-end, at least four of nucleotides at positions 2, 6, 9-12, 14, and 16 of a nucleotide sequence in the antisense strand are 2'-fluoro-modified nucleotides, and nucleotides at remaining positions are 2'-O-methyl-modified or 2'-O-methoxyethyl-modified nucleotides; and
optionally, the sense strand and/or the antisense strand comprises at least one 2'-O-methoxyethyl-modified nucleotide.

**10.** The double-stranded RNAi agent according to claim 9, wherein

in the direction from the 5'-end to the 3'-end, the nucleotides at positions 7-10 of the nucleotide sequence in the sense strand are fluoro-modified nucleotides, and the nucleotides at the remaining positions are 2'-O-methyl-modified nucleotides or 2'-O-methoxyethyl-modified nucleotides; and
positions 2, 6, 14, and 16 of the nucleotide sequence in the antisense strand are 2'-fluoro-modified nucleotides, a nucleotide at any position selected from position 9, 10, 11 or 12 is a 2'-fluoro-modified nucleotide, and the nucleotides at the remaining positions are 2'-O-methyl-modified nucleotides or 2'-O-methoxyethyl-modified nucleotides.

**11.** The double-stranded RNAi agent according to claim 9 or 10, wherein

in the direction from the 5'-end to the 3'-end, the nucleotides at positions 7-10 of the nucleotide sequence in the sense strand are fluoro-modified nucleotides, and the nucleotides at the remaining positions are 2'-O-methyl-modified nucleotides; and
positions 2, 6, 14, and 16 of the nucleotide sequence in the antisense strand are 2'-fluoro-modified nucleotides, a nucleotide at any position selected from position 9, 10, 11 or 12 is a 2'-fluoro-modified nucleotide, a nucleotide at position 15 is a 2'-O-methoxyethyl-modified nucleotide, and the remaining positions are 2'-O-methyl-modified nucleotides.

**12.** The double-stranded RNAi agent according to any one of claims 7-8, wherein all the nucleotides of the sense strand and/or the antisense strand are modified nucleotides; and modification of the sense strand and the antisense strand is selected from any one of following (1)-(4):

(1) in a direction from 5'-end to 3'-end, in the sense strand, nucleotides at positions 7-10 of a nucleotide sequence are fluoro-modified nucleotides, and nucleotides at remaining positions are 2'-O-methyl-modified nucleotides; and in the antisense strand, positions 2, 6, 9, 14, and 16 of a nucleotide sequence are 2'-fluoro-modified nucleotides, and remaining positions are 2'-O-methyl-modified nucleotides;
(2) in the direction from the 5'-end to the 3'-end, in the sense strand, the nucleotides at positions 7-10 of the nucleotide sequence are fluoro-modified nucleotides, and the nucleotides at the remaining positions are 2'-O-methyl-modified nucleotides; and in the antisense strand, positions 2, 6, 12, 14, and 16 of the nucleotide sequence are 2'-fluoro-modified nucleotides, and the remaining positions are 2'-O-methyl-modified nucleotides;
(3) in the direction from the 5'-end to the 3'-end, in the sense strand, the nucleotides at positions 7-10 of the nucleotide sequence are fluoro-modified nucleotides, and the nucleotides at the remaining positions are 2'-O-methyl-modified nucleotides; and in the antisense strand, positions 2, 6, 9, 14, and 16 of the nucleotide sequence are 2'-fluoro-modified nucleotides, the nucleotide at position 15 is a 2'-O-methoxyethyl-modified nucleotide, and the remaining positions are 2'-O-methyl-modified nucleotides;
(4) in the direction from the 5'-end to the 3'-end, in the sense strand, the nucleotides at positions 7-10 of the nucleotide sequence are fluoro-modified nucleotides, and the nucleotides at the remaining positions are 2'-O-methyl-modified nucleotides; and in the antisense strand, positions 2, 6, 12, 14, and 16 of the nucleotide sequence are 2'-fluoro-modified nucleotides, the nucleotide at position 15 is a 2'-O-methoxyethyl-modified nucleotide, and the remaining positions are 2'-O-methyl-modified nucleotides.

**13.** The double-stranded RNAi agent according to any one of claims 7-12, wherein the double-stranded RNAi agent

further comprises a ligand;

optionally, the ligand is conjugated to a 3'-end of the sense strand of the double-stranded RNAi agent; optionally, the ligand is one or more GalNAc (N-acetylgalactosamine) derivatives attached through a linker.

**14.** The double-stranded RNAi agent according to claim 13, wherein the ligand is as follows:

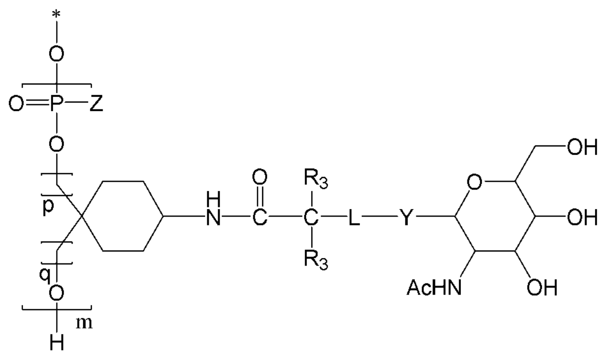

,

wherein * represents a ligation site for attachment to oligonucleotide molecules; m is 1, 2, 3, or 4; Z, p, q, $R_3$, L, and Y are defined as follows: each Z is independently selected from hydroxyl or thiol; each p is independently 1, 2, or 3; each q is independently selected from 1, 2, or 3; each $R_3$ is independently selected from H, $C_1$-$C_6$ alkyl, $C_1$-$C_6$ haloalkyl, or $C_1$-$C_6$ alkoxy; L is a $C_1$-$C_{20}$ alkylene chain, or is a $C_1$-$C_{20}$ alkylene chain with one or more C atoms in the chain replaced by any substituent selected from the group consisting of O, S, NH, and -NH-C(O)-; and each Y is independently selected from NH, O, or S.

**15.** The double-stranded RNAi agent according to claim 13 or 14, wherein a structure of the ligand is as follows:

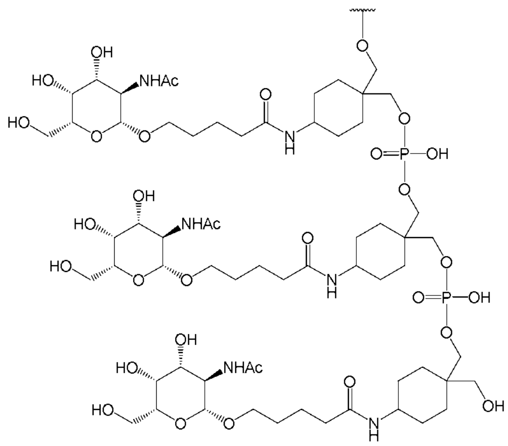

or

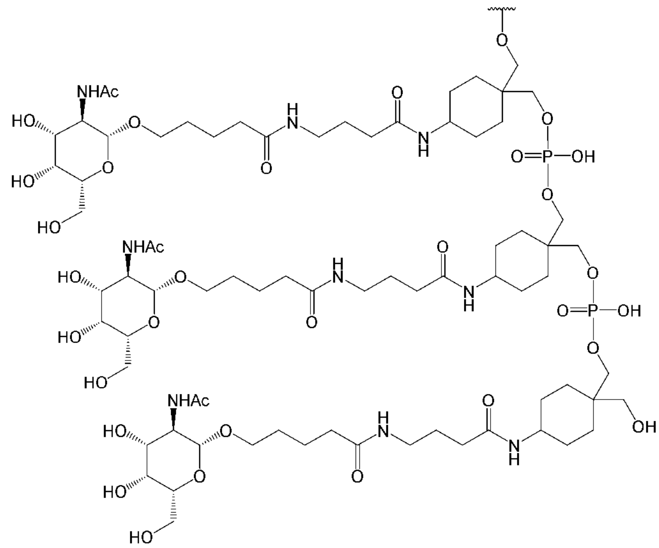

.

**16.** The double-stranded RNAi agent according to any one of claims 7-15, represented by a formula as follows:

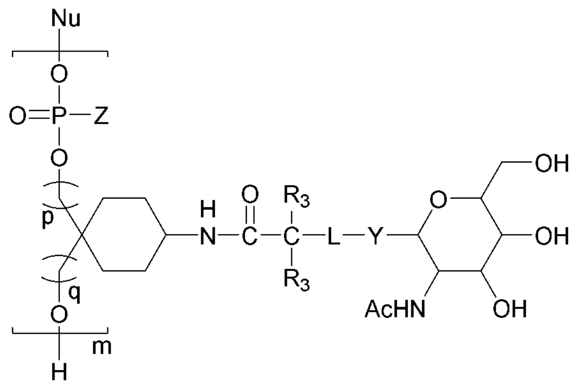

,

wherein Nu represents a double-stranded oligonucleotide molecule;
preferably, the sense strand of the double-stranded oligonucleotide comprises any modified nucleotide sequence in the sense strand as listed in Table 3; and/or the antisense strand comprises any modified nucleotide sequence in the antisense strand as listed in Table 3; and
optionally, the ligand is attached to 3'-end of the sense strand.

**17.** The double-stranded RNAi agent according to claim 12, wherein the 3'-end of the sense strand in the double-stranded RNAi agent is covalently coupled to a ligand with a structure as follows:

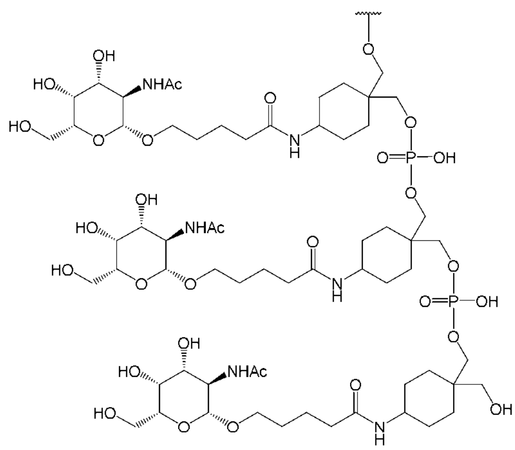

or

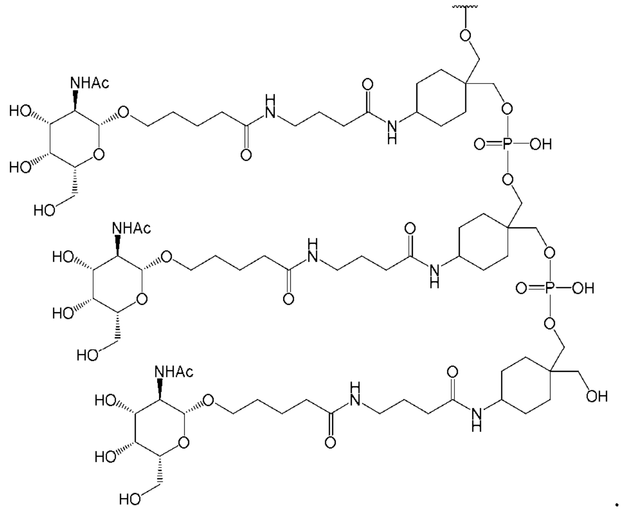

18. The double-stranded RNAi agent according to claim 17, wherein the double-stranded RNAi agent comprises a duplex selected from the group consisting of duplexes represented by RZ011304, RZ011305, RZ011306, and RZ011307;

preferably, the sense strand in the double-stranded RNAi agent is:

5'-GmsGmsAmUmUmUmGfGfGfUfUmUmUmCmUmAmUmAmAm _ (CR01008×3)-3', or
5'-GmsGmsAmUmUmUmGfGfGfUfUmUmUmCmUmAmUmAmsAm _ (CR01008×3)-3'; and

the antisense strand is:

5'- UmsUfsAmUmAmGfAmAmAfAmCmCmCmAfAmAfUmCmCmsUmsCm -3', or
5'- UmsUfsAmUmAmGfAmAmAmAmCmCfCmAfAmAfUmCmCmsUmsCm -3', or
5'- UmsUfsAmUmAmGfAmAmAfAmCmCmCmAfA(moe)AfUmCmCmsUmsCm -3', or
5'- UmsUfsAmUmAmGfAmAmAmAmCmCfCmAfA(moe)AfUmCmCmsUmsCm -3' or
5'- UmsUfsAmUmAmGfAmAmAfAmsCmCmCmAfA(moe)AfUmCmCmsUmsCm -3', wherein Am, Cm, Gm, and Um are 2'-O-methyladenosine-3'-phosphate, 2'-O-methylcytidine-3'-phosphate, 2'-O-methylguanosine phosphate, and 2'-O-methyluridine phosphate, respectively; Af, Cf, Gf, and Uf are 2'-fluoroadenosine-3'-phosphate, 2'-fluorocytidine-3'-phosphate, 2'-fluoroguanosine-3'-phosphate, and 2'-fluorouridine-3'-phosphate, respectively; A(moe) is 2'-O-methoxyethyladenosine-3'-phosphate; and s is phosphorothioate; and CR01008×3 is a ligand with a structure as follows:

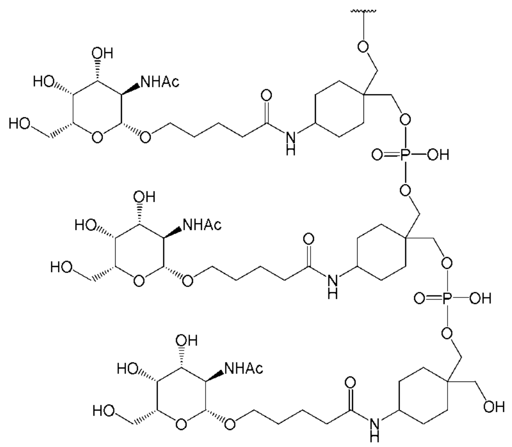

or

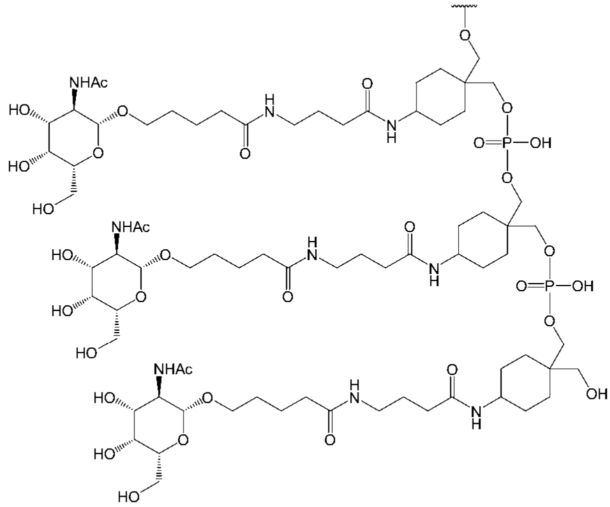

.

**19.** The double-stranded RNAi agent according to any one of claims 7-18, wherein the sense strand and/or the antisense strand further comprises a 3' and/or 5' extension or overhang of 1-3 nucleotides in length;

optionally, the sense strand is 17-21 nucleotides in length, and the antisense strand is 19-23 nucleotides in length;
optionally, the sense strand and/or the antisense strand of the RNAi agent independently comprises one or more phosphorothioate internucleotide linkages;
optionally, the sense strand comprises two contiguous phosphorothioate linkages between terminal nucleotides located at 3'-end and 5'-end, or the antisense strand comprises two contiguous phosphorothioate linkages between terminal nucleotides located at 3'-end and 5'-end;
optionally, the double-stranded region is 19 to 23 nucleotide pairs in length; or the double-stranded region is 19 to 21 nucleotide pairs in length; and
optionally, at least one strand within the double-stranded region of the RNAi agent comprises a 3' overhang of 1-2 nucleotides.

**20.** The double-stranded RNAi agent according to claim 7, wherein the double-stranded RNAi agent comprises the sense strand and the antisense strand forming the double-stranded region, wherein each strand is 14 to 25 nucleotides in length, and the antisense strand comprises a region partially complementary to an mRNA encoding CFB, wherein the double-stranded region is represented by Formula II as follows:

$$\text{SS: 5'-}(N)_{a'}-(X)_{p'}-(N)_{b'}-(X)_{q'}-(N)_{c'}-(X)_{r'}-(N)_{d'}\text{-3' AS: 3'-}(N)_a-(X)_p-(N)_b-(X)_q-(N)_c\text{-5'}$$

(II)

wherein SS represents the sense strand, AS represents the antisense strand; each N independently represents a modified nucleotide selected from the group consisting of a 2'-O-methyl-modified nucleotide, a 2'-fluoro-modified nucleotide, and a 2'-deoxy-modified nucleotide; and each X independently represents a 2'-O-methoxyethyl-modified nucleotide;
a, a', p, p', b, b', q, q', c, c', r', and d' each independently represent the number of nucleotides, wherein a' is an integer selected from 3 to 8; p' is 0 or 1; b' is an integer selected from 4 to 13; q' is 0 or 1; c' is an integer selected from 3 to 9; r' is 0 or 1; d' is an integer selected from 1 to 8; a is an integer selected from 4 to 7; p is 1; b is an integer selected from 4 to 8; q is 0 or 1; c is an integer selected from 6 to 10; and p', q', r', p, and q are not simultaneously 0, and $0 \leq q' + r' \leq 2$;
moreover, at least one fluoro-modified nucleotide is present in $(N)_a$, and position 16 of the antisense strand counting from the 5'-end is a fluoro-modified nucleotide; at least one fluoro-modified nucleotide is present in $(N)_b$, and position 14 of the antisense strand counting from the 5'-end is a fluoro-modified nucleotide; at least two fluoro-modified nucleotides are present in $(N)_c$, and positions 2 and 6 of the antisense strand counting from the 5'-end are both fluoro-modified nucleotides; and first 4 nucleotides of $(N)_{b'}$ comprise at least two fluoro-modified nucleotides;
preferably, in a direction from the 5'-end to the 3'-end, nucleotides at positions 7-10 of a nucleotide sequence in the

sense strand are fluoro-modified nucleotides, and nucleotides at remaining positions are 2'-O-methyl-modified nucleotides; and positions 2, 6, 14, and 16 of a nucleotide sequence in the antisense strand are 2'-fluoro-modified nucleotides, a nucleotide at any position selected from position 9, 10, 11 or 12 is a 2'-fluoro-modified nucleotide, a nucleotide at position 15 is a 2'-O-methoxyethyl-modified nucleotide, and nucleotides at remaining positions are 2'-O-methyl-modified nucleotides,
wherein the sense strand is conjugated to a ligand.

**21.** The double-stranded RNAi agent according to claim 20, wherein the ligand has a structure as follow:

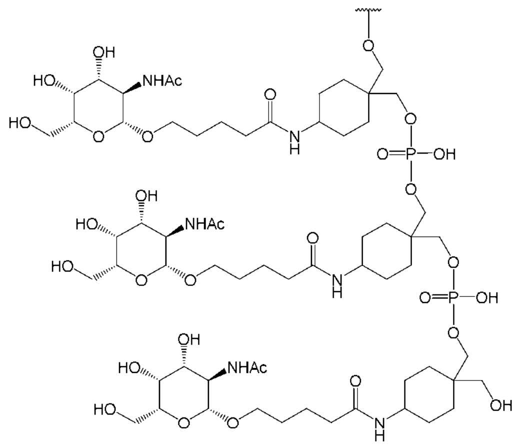

or

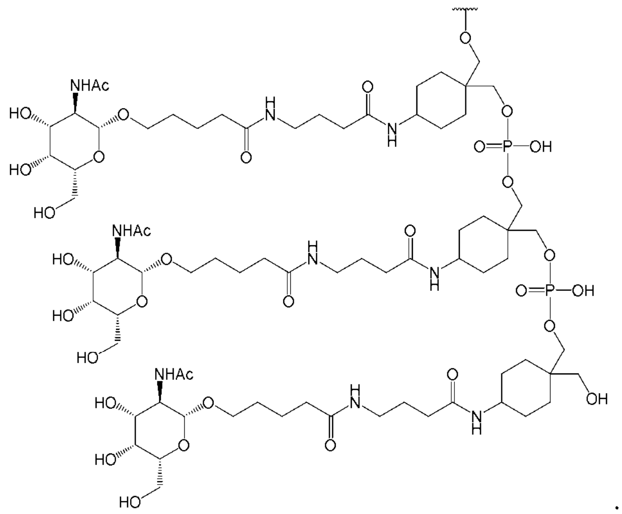

**22.** Use of the double-stranded oligonucleotide according to any one of claims 1-6 and the double-stranded RNAi agent according to any one of claims 7-21 in the manufacture of a medicament for relieving, preventing and/or treating a disease or disorder mediated by a complement factor B gene,

optionally, the disease or disorder comprises kidney disease, systemic lupus erythematosus and related diseases, macular degeneration, atypical hemolytic uremic syndrome, thrombotic microangiopathy, myasthenia gravis, ischemia-reperfusion injury, paroxysmal nocturnal hemoglobinuria, and rheumatoid arthritis; and preferably, the kidney disease comprises C3 glomerulopathy, lupus nephritis, IgA nephropathy, diabetic nephropathy, membranous nephropathy, and polycystic kidney disease.

**23.** A pharmaceutical composition, comprising the double-stranded oligonucleotide according to any one of claims 1-6 and the double-stranded RNAi agent according to any one of claims 7-21, and further comprising a pharmaceutically optional auxiliary material.

**24.** A kit, comprising the double-stranded oligonucleotide according to any one of claims 1-6 and the double-stranded RNAi agent according to any one of claims 7-21 or a pharmaceutical composition thereof.

**25.** A method for inhibiting complement factor B gene expression, wherein the method includes administering the double-stranded oligonucleotide according to any one of claims 1-6, or the double-stranded RNAi agent according to any one of claims 7-21, or the pharmaceutical composition according to claim 23 to a subject;

optionally, complement factor B expression is inhibited by at least 50%, 60%, 70%, 80%, 90%, or 95%; and
optionally, inhibition of the complement factor B expression reduces a complement factor B protein level in serum of the subject by at least 50%, 60%, 70%, 80%, 90%, or 95%.

**26.** A method for relieving, treating and/or preventing a disease or disorder mediated by complement factor B, comprising administering the double-stranded oligonucleotide according to any one of claims 1-6, or the double-stranded RNAi agent according to any one of claims 7-21, or the pharmaceutical composition according to claim 23 to a subject, wherein

optionally, a disease or disorder mediated by the complement factor B includes a disease associated with mRNA level of complement factor B gene expression;
optionally, the disease or disorder comprises kidney disease, systemic lupus erythematosus and related diseases, macular degeneration, atypical hemolytic uremic syndrome, thrombotic microangiopathy, myasthenia gravis, ischemia-reperfusion injury, paroxysmal nocturnal hemoglobinuria, and rheumatoid arthritis; and
preferably, the kidney disease comprises C3 glomerulopathy, lupus nephritis, IgA nephropathy, diabetic nephropathy, membranous nephropathy, and polycystic kidney disease.

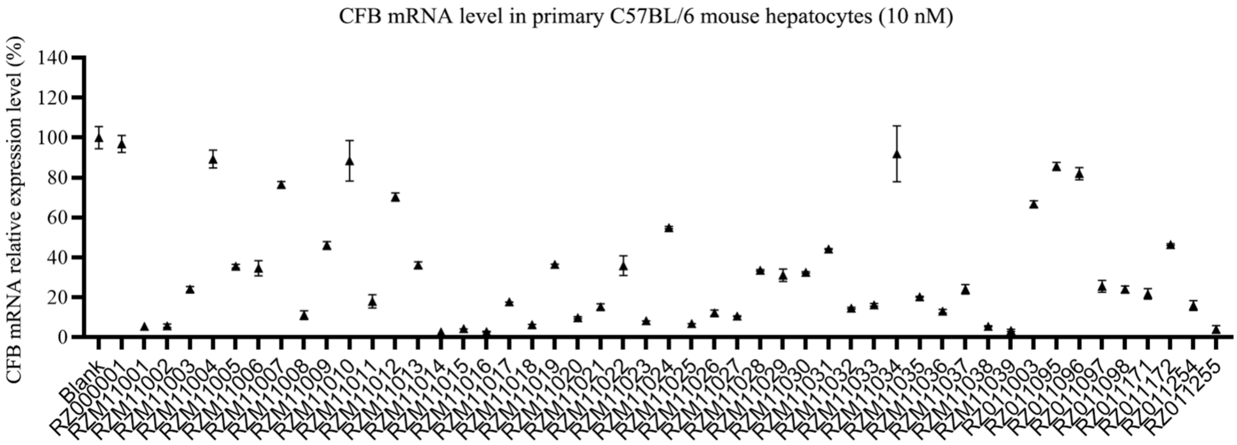
CFB mRNA level in primary C57BL/6 mouse hepatocytes (10 nM)
CFB mRNA relative expression level (%)
140
120
100
80
60
40
20
0

**FIG. 1**

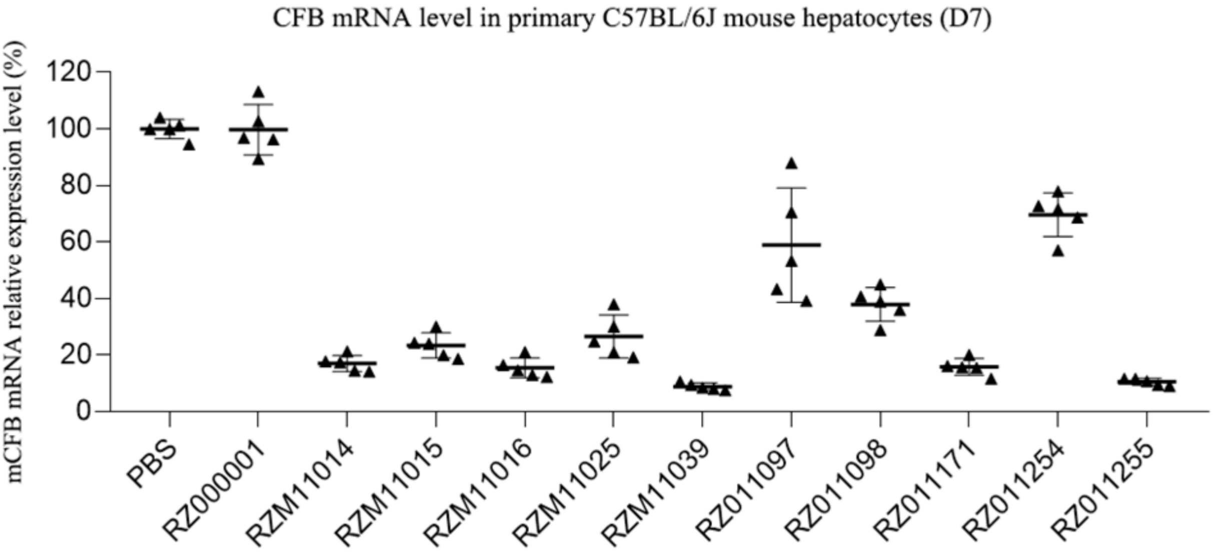
CFB mRNA level in primary C57BL/6J mouse hepatocytes (D7)
mCFB mRNA relative expression level (%)
120
100
80
60
40
20
0
PBS
RZ000001
RZM11014
RZM11015
RZM11016
RZM11025
RZM11039
RZ011097
RZ011098
RZ011171
RZ011254
RZ011255

**FIG. 2**

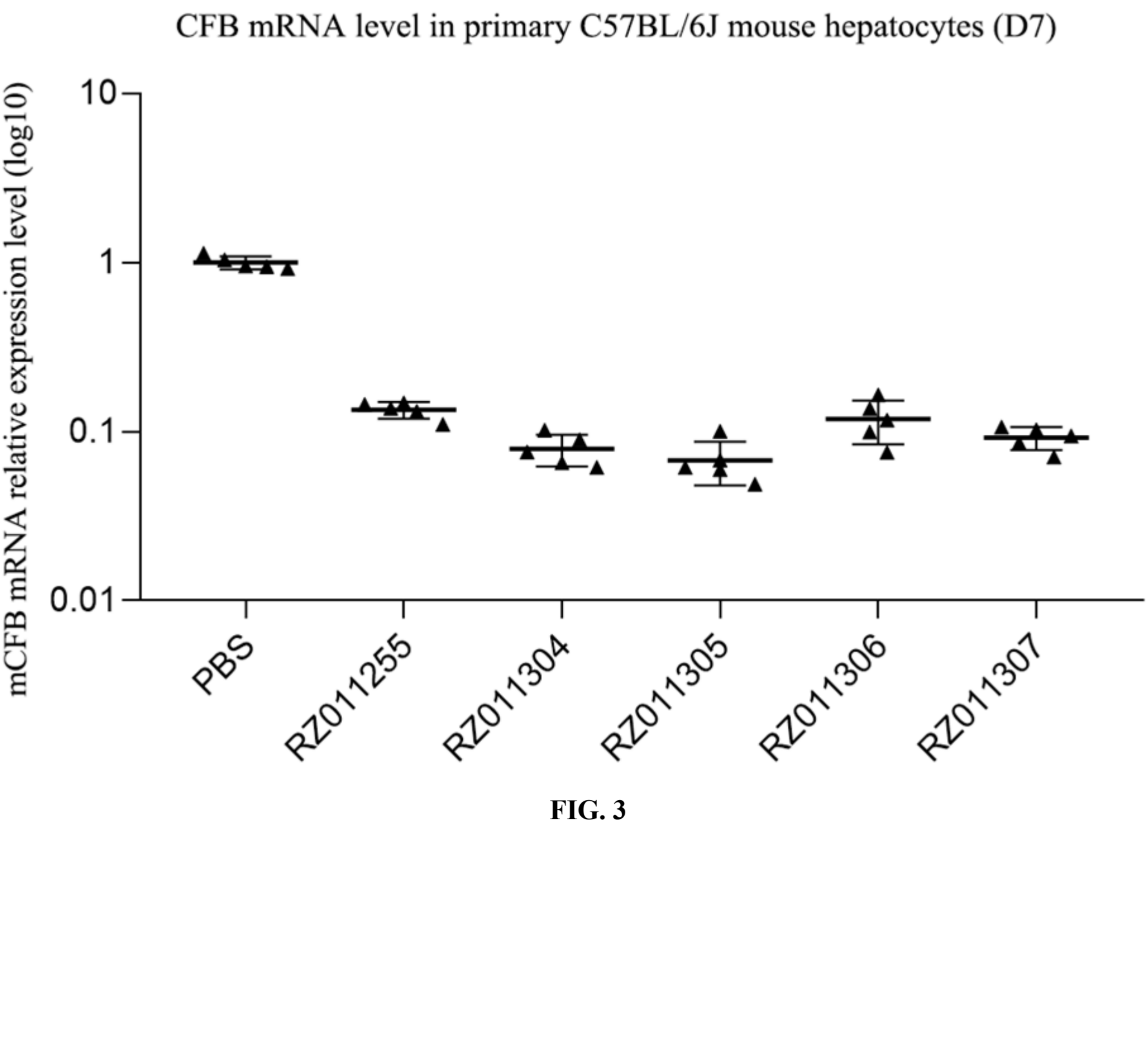
CFB mRNA level in primary C57BL/6J mouse hepatocytes (D7)
mCFB mRNA relative expression level (log10)
10
1
0.1
0.01
PBS
RZ011255
RZ011304
RZ011305
RZ011306
RZ011307

FIG. 3

**INTERNATIONAL SEARCH REPORT**

International application No.
**PCT/CN2024/107887**

**A. CLASSIFICATION OF SUBJECT MATTER**

C12N15/113(2010.01)i；A61K31/713(2006.01)i；A61K31/712(2006.01)i；A61K31/7088(2006.01)i

According to International Patent Classification (IPC) or to both national classification and IPC

**B. FIELDS SEARCHED**

Minimum documentation searched (classification system followed by classification symbols)

IPC:C12N,A61K

Documentation searched other than minimum documentation to the extent that such documents are included in the fields searched

Electronic data base consulted during the international search (name of data base and, where practicable, search terms used)

ATTXT, BETXT, CATXT, CHTXT, CNTXT, DETXT, DWPI, EATXT, ENTXTC, EPTXT, ESTXT, FRTXT, GBTXT, JPTXT, KRTXT, LEXTXT, RUTXT, SGTXT, TWTXT, USTXT, VEN, WOTXT, WPABSC, Genbank, 中国专利生物序列检索系统, China Patent Biological Sequence Search System, CNKI, 万方, WANFANG, ISI web of Science: 补体因子B, 因子B, CFB, facter B, SIRNA, dsRNA, 沉默, silence, SEQ ID NOs: 1 and 256

**C. DOCUMENTS CONSIDERED TO BE RELEVANT**

| Category* | Citation of document, with indication, where appropriate, of the relevant passages | Relevant to claim No. |
|---|---|---|
| A | CN 106232804 A (IONIS PHARMACEUTICALS, INC.) 14 December 2016 (2016-12-14) claims 1, 24 and 41, and description, paragraph 454, and embodiment 115 | 1-6, 22-26 (all in part) |
| A | US 2010184021 A1 (COMPUGEN LTD.) 22 July 2010 (2010-07-22) description, paragraphs 999-1000 | 1-6, 22-26 (all in part) |
| A | CN 105814205 A (ALNYLAM PHARMACEUTICALS INC.) 27 July 2016 (2016-07-27) claims 1-62, and description, table 3 | 1-6, 22-26 (all in part) |
| A | WO 2017135397 A1 (KYOWA HAKKO KIRIN CO., LTD.) 10 August 2017 (2017-08-10) claims 1-13, and description, table 2 | 1-6, 22-26 (all in part) |
| A | WO 2023031359 A1 (SILENCE THERAPEUTICS GMBH) 09 March 2023 (2023-03-09) claims 1-15 | 1-6, 22-26 (all in part) |
| A | WO 2023097291 A1 (ADARX PHARMACEUTICALS, INC.) 01 June 2023 (2023-06-01) description, table 2 | 1-6, 22-26 (all in part) |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **21 October 2024** | **31 October 2024** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/107887**

**C. DOCUMENTS CONSIDERED TO BE RELEVANT**

| Category* | Citation of document, with indication, where appropriate, of the relevant passages | Relevant to claim No. |
|---|---|---|
| A | WO 2023129496 A2 (APELLIS PHARMACEUTICALS, INC.) 06 July 2023 (2023-07-06) claims 1-55 | 1-6, 22-26 (all in part) |
| A | WO 2023141247 A2 (ALEXION PHARMACEUTICALS, INC.) 27 July 2023 (2023-07-27) claims 1-98 | 1-6, 22-26 (all in part) |

Form PCT/ISA/210 (second sheet) (July 2022)

**INTERNATIONAL SEARCH REPORT**

International application No.
**PCT/CN2024/107887**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   - a. ☑ forming part of the international application as filed.
   - b. ☐ furnished subsequent to the international filing date for the purposes of international search (Rule 13*ter*.1(a)),
     - ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

International application No.
**PCT/CN2024/107887**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **25-26 (in part)**
because they relate to subject matter not required to be searched by this Authority, namely:

Claims 25-26 (in part) set forth a method for inhibiting the gene expression of complement factor B and a method for alleviating, treating and/or preventing complement factor B-mediated diseases or conditions, which methods comprise administering to a subject the double-stranded oligonucleotide of any one of claims 1-6 or the pharmaceutical composition of claim 23. The methods belong to a method for treatment of diseases as defined in PCT Rule 39.1(iv). However, a search is still performed on the basis of the pharmaceutical use of the double-stranded oligonucleotide.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

Invention 1: claims 1-6 and 22-26 (all in part) relate to a dsRNA of RN011001, a pharmaceutical composition, a kit, and a use thereof.

Inventions 2-208: claims 1-2, 5-6 and 22-26 (all in part) relate to dsRNAs of RN011002-RN011004, RN011008-RN011017, RN011019-RN011020, RN011023-RN011032, RN011036-RN011037, RN011039-RN011051, RN011053-RN011075, RN011077-RN011096, RN011099-RN011100, RN011105, RN011108, RN011110-RN011112, RN011115-RN011116, RN011118-RN011122, RN011126-RN011139, RN011141-RN011149, RN011153-RN011155, RN011157-RN011159, RN011161-RN011170, RN011172-RN011182, RN011184-RN011192, RN011195-RN011198, RN011200, RN011203-RN011232, RN011234-RN011237, RN011239-RN011240 and RN011244-RN011253, pharmaceutical compositions, kits, and uses thereof.

Inventions 209-254: claims 1-6 and 22-26 (all in part) relate to dsRNAs of RN011005, RN011006, RN011007, RN011018, RN011021, RN011022, RN011033, RN011034, RN011035, RN011038, RN011052, RN011076, RN011097, RN011098, RN011101, RN011102, RN011103, RN011104, RN011106, RN011107, RN011109, RN011113, RN011114, RN011117, RN011123, RN011124, RN011125, RN011140, RN01115, RN011151, RN011152, RN011156, RN011160, RN011171, RN011183, RN011193, RN011194, RN011199, RN011201, RN011202, RN011233, RN011238, RN011241, RN011242, RN011243, RN011254, pharmaceutical compositions, kits, and uses thereof.

Invention 255: claims 1-6 and 8-26 (all in part) relate to a dsRNA of RN011255, a double-stranded RNAi agent, a pharmaceutical composition, a kit, and a use thereof.

Invention 256: claims 7-21 and 22-26 (in part) relate to a double-stranded RNAi agent having modified nucleotides, a pharmaceutical composition, a kit, and a use thereof.

The common feature comprised in inventions 1 to 256 is an RNAi molecule targeting CFB; however, the feature is known in the prior art. Therefore, inventions 1 to 256 do not have a same or corresponding special technical feature, do not comply with PCT Rule 13.2, and lack unity of invention (PCT Rule 13.1).

Form PCT/ISA/210 (continuation of first sheet) (July 2022)

**INTERNATIONAL SEARCH REPORT**

International application No.
**PCT/CN2024/107887**

**Box No. III Observations where unity of invention is lacking (Continuation of item 3 of first sheet)**

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **claims 1-6 and 22-26 (all in part), which relate to a dsRNA of RN011001, a pharmaceutical composition, a kit, and a use thereof**

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (July 2022)

**INTERNATIONAL SEARCH REPORT**
**Information on patent family members**

International application No.
**PCT/CN2024/107887**

| Patent document cited in search report | Publication date (day/month/year) | Patent family member(s) | Publication date (day/month/year) |
|---|---|---|---|
| CN 106232804 A | 14 December 2016 | EP 3137596 A2 | 08 March 2017 |
| | | EP 3137596 A4 | 20 September 2017 |
| | | EP 3137596 B1 | 10 July 2019 |
| | | SG 10201809953 QA | 28 December 2018 |
| | | MX 2016014294 A | 02 February 2017 |
| | | US 2017159055 A1 | 08 June 2017 |
| | | US 10280423 B2 | 07 May 2019 |
| | | SI 3137596 T1 | 30 August 2019 |
| | | US 2020048638 A1 | 13 February 2020 |
| | | PL 3608406 T3 | 22 May 2023 |
| | | NZ 763982 A | 24 November 2023 |
| | | DK 3137596 T3 | 02 September 2019 |
| | | PL 3137596 T3 | 29 November 2019 |
| | | CR 20160554 A | 07 March 2017 |
| | | HUE 046272 T2 | 28 February 2020 |
| | | IL 271659 A | 27 February 2020 |
| | | US 2023357776 A1 | 09 November 2023 |
| | | EP 3608406 A1 | 12 February 2020 |
| | | EP 3608406 B1 | 15 February 2023 |
| | | HRP 20191664 T1 | 13 December 2019 |
| | | JP 2020058368 A | 16 April 2020 |
| | | JP 7001663 B2 | 04 February 2022 |
| | | UA 121656 C2 | 10 July 2020 |
| | | CL 2016002764 A1 | 29 December 2017 |
| | | ES 2941742 T3 | 25 May 2023 |
| | | PT 3137596 T | 27 September 2019 |
| | | DOP 2016000291 A | 15 February 2017 |
| | | RS 59182 B1 | 31 October 2019 |
| | | WO 2015168635 A2 | 05 November 2015 |
| | | WO 2015168635 A3 | 07 January 2016 |
| | | US 2023057718 A1 | 23 February 2023 |
| | | US 11732265 B2 | 22 August 2023 |
| | | RU 2016147047 A | 01 June 2018 |
| | | RU 2016147047 A3 | 26 October 2018 |
| | | RU 2701645 C2 | 30 September 2019 |
| | | CR 20200228 A | 09 July 2020 |
| | | BR 112016022593 A2 | 10 October 2017 |
| | | BR 112016022593 B1 | 26 April 2022 |
| | | CA 2943894 A1 | 05 November 2015 |
| | | NZ 724366 A | 27 October 2023 |
| | | PE 20170010 A1 | 04 March 2017 |
| | | SG 11201608502 TA | 29 November 2016 |
| | | IL 247883 A0 | 30 November 2016 |
| | | IL 247883 B | 25 March 2021 |
| | | PH 12016502062 A1 | 19 December 2016 |
| | | LT 3137596 T | 10 September 2019 |
| | | JP 2017514489 A | 08 June 2017 |
| | | JP 6637442 B2 | 29 January 2020 |
| | | PE 20190626 A1 | 26 April 2019 |
| | | KR 20160147892 A | 23 December 2016 |

Form PCT/ISA/210 (patent family annex) (July 2022)

**INTERNATIONAL SEARCH REPORT**
**Information on patent family members**

International application No.
**PCT/CN2024/107887**

| Patent document cited in search report | Publication date (day/month/year) | Patent family member(s) | Publication date (day/month/year) |
|---|---|---|---|
| | | KR 102369736 B1 | 02 March 2022 |
| | | EP 4219718 A2 | 02 August 2023 |
| | | EP 4219718 A3 | 10 January 2024 |
| | | CL 2018001996 A1 | 08 March 2019 |
| | | EA 201692204 A1 | 28 April 2017 |
| | | EA 036496 B1 | 17 November 2020 |
| | | AU 2015252858 A1 | 06 October 2016 |
| | | AU 2015252858 B2 | 10 December 2020 |
| | | AU 2015252858 C1 | 16 September 2021 |
| | | ES 2745825 T3 | 03 March 2020 |
| US 2010184021 A1 | 22 July 2010 | EP 1973945 A2 | 01 October 2008 |
| | | EP 1973945 A4 | 18 November 2009 |
| | | EP 2216339 A1 | 11 August 2010 |
| | | WO 2007080597 A2 | 19 July 2007 |
| | | WO 2007080597 A3 | 26 March 2009 |
| | | CA 2637267 A1 | 19 July 2007 |
| CN 105814205 A | 27 July 2016 | AU 2014362262 A1 | 30 June 2016 |
| | | AU 2014362262 B2 | 13 May 2021 |
| | | JP 2020188768 A | 26 November 2020 |
| | | JP 7057390 B2 | 19 April 2022 |
| | | US 2020263183 A1 | 20 August 2020 |
| | | US 11186842 B2 | 30 November 2021 |
| | | US 2022213486 A1 | 07 July 2022 |
| | | US 2020339998 A1 | 29 October 2020 |
| | | SG 11201604692 UA | 28 July 2016 |
| | | JP 2017500031 A | 05 January 2017 |
| | | JP 6710638 B2 | 17 June 2020 |
| | | SG 10201804960 RA | 30 July 2018 |
| | | KR 20210158880 A | 31 December 2021 |
| | | IL 282401 A | 30 June 2021 |
| | | IL 282401 B | 01 August 2022 |
| | | KR 20160097307 A | 17 August 2016 |
| | | KR 102344559 B1 | 31 December 2021 |
| | | US 2016298124 A1 | 13 October 2016 |
| | | US 10465194 B2 | 05 November 2019 |
| | | IL 245678 A0 | 30 June 2016 |
| | | IL 245678 B | 29 July 2021 |
| | | CA 2931090 A1 | 18 June 2015 |
| | | CA 3107872 A1 | 18 June 2015 |
| | | MX 2016007409 A | 14 December 2016 |
| | | IL 314045 A | 01 September 2024 |
| | | JP 2022106742 A | 20 July 2022 |
| | | EP 3080270 A2 | 19 October 2016 |
| | | EP 3080270 B1 | 27 October 2021 |
| | | EP 3798306 A1 | 31 March 2021 |
| | | EA 201691215 A1 | 30 November 2016 |
| | | WO 2015089368 A2 | 18 June 2015 |
| | | WO 2015089368 A3 | 20 August 2015 |
| | | AU 2021204655 A1 | 29 July 2021 |
| | | AU 2021204655 B2 | 21 December 2023 |

Form PCT/ISA/210 (patent family annex) (July 2022)

**INTERNATIONAL SEARCH REPORT**
**Information on patent family members**

International application No.
**PCT/CN2024/107887**

| Patent document cited in search report | Publication date (day/month/year) | Patent family member(s) | Publication date (day/month/year) |
|---|---|---|---|
| | | AU 2024201770 A1 | 02 May 2024 |
| | | IL 294470 A | 01 September 2022 |
| | | IL 294470 B1 | 01 August 2024 |
| | | BR 112016013148 A2 | 26 September 2017 |
| | | BR 112016013148 B1 | 27 February 2024 |
| | | MX 2021006052 A | 06 July 2021 |
| WO 2017135397 A1 | 10 August 2017 | TW 201730341 A | 01 September 2017 |
| WO 2023031359 A1 | 09 March 2023 | CN 118234861 A | 21 June 2024 |
| | | TW 202319058 A | 16 May 2023 |
| | | EP 4396346 A1 | 10 July 2024 |
| | | CA 3230589 A1 | 09 March 2023 |
| | | AU 2022336157 A1 | 17 March 2024 |
| | | JP 2024532506 A | 05 September 2024 |
| | | KR 20240051221 A | 19 April 2024 |
| | | IL 311146 A | 01 April 2024 |
| WO 2023097291 A1 | 01 June 2023 | US 2024043836 A1 | 08 February 2024 |
| | | EP 4437104 A1 | 02 October 2024 |
| | | CA 3240273 A1 | 01 June 2023 |
| | | AU 2022396536 A1 | 06 June 2024 |
| | | KR 20240107360 A | 09 July 2024 |
| | | MX 2024006368 A | 11 June 2024 |
| | | CO 2024006751 A2 | 27 June 2024 |
| | | IL 312928 A | 01 July 2024 |
| | | PE 20241340 A1 | 03 July 2024 |
| WO 2023129496 A2 | 06 July 2023 | WO 2023129496 A3 | 02 November 2023 |
| | | WO 2023129496 A9 | 15 August 2024 |
| WO 2023141247 A2 | 27 July 2023 | CN 118647384 A | 13 September 2024 |
| | | TW 202345866 A | 01 December 2023 |
| | | WO 2023141247 A3 | 31 August 2023 |
| | | AU 2023208539 A1 | 22 August 2024 |
| | | KR 20240135650 A | 11 September 2024 |
| | | IL 314240 A | 01 September 2024 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202310938654